(19)

**Europäisches Patentamt**
**European Patent Office**
**Office européen des brevets**

(11) **EP 4 148 071 A1**

(12) **EUROPEAN PATENT APPLICATION**

(43) Date of publication:
**15.03.2023  Bulletin 2023/11**

(51) International Patent Classification (IPC):
***C08B 15/00*** (2006.01)

(21) Application number: **22194012.5**

(22) Date of filing: **06.09.2022**

(52) Cooperative Patent Classification (CPC):
**C08B 15/00;** A61K 2035/128

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.09.2021  FI 20215963**

(71) Applicant: **UPM-Kymmene Corporation**
**00100 Helsinki (FI)**

(72) Inventors:
 • **Nuopponen, Markus**
  **00220 Helsinki (FI)**
 • **Sheard, Jonathan**
  **Bromsgrove, B61 8SG (GB)**

 • **Eilers, Wouter**
  **Reading Berkshire, RG6 6AH (GB)**
 • **Widera, Darius**
  **Reading Berkshire, RG6 6AH (GB)**
 • **Cottrell, Graeme S**
  **Reading Berkshire, RG6 6AH (GB)**
 • **Coopman, Karen**
  **Loughborough, LE11 3TU (GB)**
 • **Barrett, Lisa**
  **Loughborough, LE11 3TU (GB)**
 • **Dragosavac, Marijana**
  **Loughborough, LE11 3TU (GB)**

(74) Representative: **Berggren Oy**
  **P.O. Box 16**
  **Eteläinen Rautatiekatu 10A**
  **00101 Helsinki (FI)**

(54) **A METHOD FOR PREPARING MICROBEADS, MICROBEADS, A CELL CULTURE, A METHOD FOR PROVIDING CELL-DERIVED PRODUCTS, THE MICROBEADS FOR USE FOR PROVIDING BIOACTIVE SUBSTANCES TO A TARGET, AND USE OF CHEMICALLY ANIONICALLY MODIFIED NANOFIBRILLAR CELLULOSE**

(57)    The present disclosure provides a method for preparing microbeads comprising nanofibrillar cellulose, the method comprising providing a dispersion of chemically anionically modified nanofibrillar cellulose having a number-average diameter of 200 nm or less, forming the nanofibrillar cellulose into microbeads, to obtain microbeads comprising chemically anionically modified nanofibrillar cellulose in the range of 0.2-2% by weight. The present disclosure also provides the microbeads, a cell culture, a method for providing cell-derived products, the microbeads for use for providing bioactive substances to a target, and use of chemically anionically modified nanofibrillar cellulose for preparing the microbeads.

EP 4 148 071 A1

## Description

### Field of the application

The present application relates to methods for preparing microbeads, and to microbeads obtained with the methods. The present application also relates to cell cultures comprising the microbeads. The present application also relates to a method for providing cell-derived products, to use of the microbeads for providing bioactive substances to a target, and to use of chemically anionically modified nanofibrillar cellulose for preparing the microbeads.

### Background

It is often desired to handle and culture eukaryotic cells, especially mammalian stem cells, for specific purposes. Such operations may require specific environment and materials, which enable handling, maintaining and culturing the cells, and sometimes producing desired cell-derived products. Hydrogels, both of synthetic and natural origin, have emerged as suitable scaffolds for 3D cell culture. The network of interconnected pores in hydrogels allows retention of a large amount of biological fluid facilitating transport of oxygen, nutrients and waste. Furthermore, most hydrogels can be formed under mild cytocompatible conditions and their biological properties can be modulated by surface chemistry.
Nanofibrillar cellulose (NFC) hydrogels can be used as a matrix for culturing cells. NFC is nonanimal derived material with fibre diameter in nanometre range and fibre length in micrometre range. NFC can be isolated for example from the cell walls of wood, other plants, and from certain bacteria. However there are still challenges in using also NFC hydrogels for culturing challenging cell types, such as stem cells, and using thereof in specific methods, such as in transfection methods, cell-derived product productions and for maintaining the cells in challenging conditions. For example the migration of agents in the hydrogel is not optimal for all purposes. Further, there are still challenges in maintaining cells in active or undifferentiated form, or carrying out recombinant techniques on cells in the hydrogel.
There is a need for improved material for culturing and storing cells, especially in methods involving maintaining the cells or producing cell-derived products.

### Summary

It was found out how to prepare microbeads from nanofibrillar cellulose for use in cell culture and cell storage. It was possible to control the process to obtain microbeads with desired properties.
The present disclosure provides a method for preparing microbeads comprising nanofibrillar cellulose, the method comprising

- providing an aqueous dispersion of chemically anionically modified nanofibrillar cellulose having a number-average diameter of 200 nm or less, such as 100 nm or less,
- forming the nanofibrillar cellulose into microbeads having an average diameter in the range of 100-1200 $\mu$m,
  to obtain microbeads comprising chemically anionically modified nanofibrillar cellulose having a concentration in the range of 0.2-2% by weight in the microbeads, such as in the range of 0.2-1% by weight, such as in the range of 0.2-0.8% by weight,
- the method further comprising treating the chemically anionically modified nanofibrillar cellulose or the microbeads with multivalent cations to ionically crosslink the nanofibrillar cellulose.

The present disclosure also provides microbeads comprising chemically anionically modified nanofibrillar cellulose having a number-average diameter of 200 nm or less, such as 100 nm or less, and having a concentration in the range of 0.2-2% by weight, such as in the range of 0.2-0.8% by weight.
The present disclosure also provides a cell culture comprising cells in the microbeads.
The present disclosure also provides a method for producing cell-derived products, the method comprising

- providing the microbeads,
- providing cells,
- combining the cells with the microbeads, and
- allowing the cells to produce cell-derived products in the microbeads.

The present disclosure also provides the microbeads for use for providing bioactive substances to a target.
The present disclosure also provides use of chemically anionically modified nanofibrillar cellulose having a number-average diameter of 200 nm or less for preparing the microbeads.
The main embodiments are characterized in the independent claims. Various embodiments are disclosed in the dependent claims. The embodiments and examples disclosed herein are mutually freely combinable unless otherwise explicitly stated.

The chemically anionically modified nanofibrillar cellulose (ANFC) hydrogels described herein are useful in medical and scientific applications, wherein the materials comprising ANFC are in contact with living matter. The products containing the ANFC are highly biocompatible with the biological matter and provide several advantageous effects. Without binding to any specific theory, it is believed that the ANFC microbeads comprising very hydrophilic nanofibrillar cellulose having a very high specific surface area, and thus high water retention ability, when applied against cells, provides favourable moist environment between the cells and the hydrogel comprising nanofibrillar cellulose. The high amount of free hydroxyl groups in the nanofibrillar cellulose forms hydrogen bonds between the nanofibrillar cellulose and water molecules and enables gel formation and the high water retention ability of the nanofibrillar cellulose. The nanofibrillar cellulose hydrogel contains a high amount of water, and it also enables migration of fluids and/or substances, especially bioactive substances in active form. It was surprisingly found out that despite the high amount of free hydroxyl groups in the ANFC the material did not interact harmfully with the biologics produced by the cells thus allowing the biologics to be released from the ANFC for extraction.

The microbeads can be used as a matrix for cells in the production of cell-derived products and for storing cells thus providing an environment, which protects the cells and helps them to maintain their viability. The formed matrix, which may be called interstitial matrix, physically resembles ECM and provides a meshwork-like matrix which can be provided with desired pore sizes. The dimensions of the network of nanofibrillar cellulose, especially cellulose nanofibrils, is very close to natural ECM network of collagen nanofibrils. It provides structural support for cells and a network of interconnected pores for efficient cell migration and transfer of nutrients to the cells and cell-derived products from the cells. The nanofibrillar cellulose in the microbead form can for example stand the flow used in a continuous bioreactor systems and the cells are maintained in the nanofibrillar cellulose matrix, such as maintained at their locations and maintained at a desired state, for example at a desired state of undifferentiation in case of stem cells. This enables obtaining the same cell-derived product(s) during the whole production time.

Furthermore, nanofibrillar cellulose is non-animal-based material and therefore xeno-free, so there is no risk for disease transfer or rejection. Especially when human cells are concerned, the formed system can comprise, in addition to the cellulose and probably minor amount of additives, only human-derived components, so it does not contain any material from foreign animal or microbial species.

Cellulose nanofibrils have negligible fluorescence background. With the present materials it is possible to obtain a transparent and porous matrix for the cells, and the handling of the material is easy compared to the alternatives.

Cellulose is biocompatible due to moderate, if any, foreign responses and is safe especially for stem-cell applications, with no known toxicity. It is also biodurable; cellulose resorption is slow, as cells cannot synthesize cellulases required to degrade cellulose.

The present solutions are especially suitable for stimulating *in vivo* like biologics secretion, such as EV secretion and production of viruses, and the exploration of the sustained production of the biologics. For example, many of the cells cultured for exosomes are anchorage-dependent, thus NFC microbeads, which can incorporate the cells, are especially suitable for culturing exosome-secreting cells. This also applies to other types of cell-derived products as well, such as secreted or excreted biologics. In one solution cells in the microbeads are maintained while cell-derived product enriched media is harvested at a rate equal to addition of new media. A permeabilized or semi-permeable structure, such as a membrane or a filter, can be used to separate microbeads and cells from the cell-derived product enriched medium. The cell-derived products, such as extracellular vesicles, may be harvested by using any suitable methods, such as based on their sizes, for example by using size exclusion chromatography, one or more filters, and/or functional molecules, such as biomolecules binding an EV surface protein.

The present materials and arrangements enable culturing cells in a form suitable for facilitating efficient growth and cell culture production. The cells may be for example grown as aggregates, such as spheroids, which may be desired for cell culture production purposes. Spheroid cultures can be maintained for weeks, even months, in nanofibrillar cellulose hydrogel allowing efficient utilization of the cells in the bioreactor. The use of nanofibrillar cellulose microbeads as interstitial matrix may prevent or decrease undesired agglomeration of the cells or cell spheroids and help to maintain the desired phenotype of the cells and the spectrum of produced cell-derived products. It was also found out that the cells could adhere well to the present microbeads, especially to crosslinked microbeads.

Also, the nanofibrillar cellulose microbeads shelter the cells as well as the cell-derived products, which is especially important in case the cell-derived products are vesicles or the like fragile structures, during the process reducing issues associated with hydrodynamic shear forces in bioreactors. The nanofibrillar cellulose microbeads may be provided in such form that facilitates flow of liquids and diffusions of substances, such as the cell culture products but also nutrients, metabolites, or other substances having an impact to the process. The nanofibrillar cellulose mi-microbeads may also be used for capturing specific proteins or nucleic acids from cell or tissue extract.

In the present solutions it was found how to obtain good quality NFC microbeads with small enough diameter, which provides more surface area and enable new and more efficient uses of the microbeads. For example, it was found out that the present microbeads could provide enhanced flow of substances to and from the microbeads, which was found advantageous for example in the production of cell-derived products and providing bioactive substances.

**Brief description of the figures**

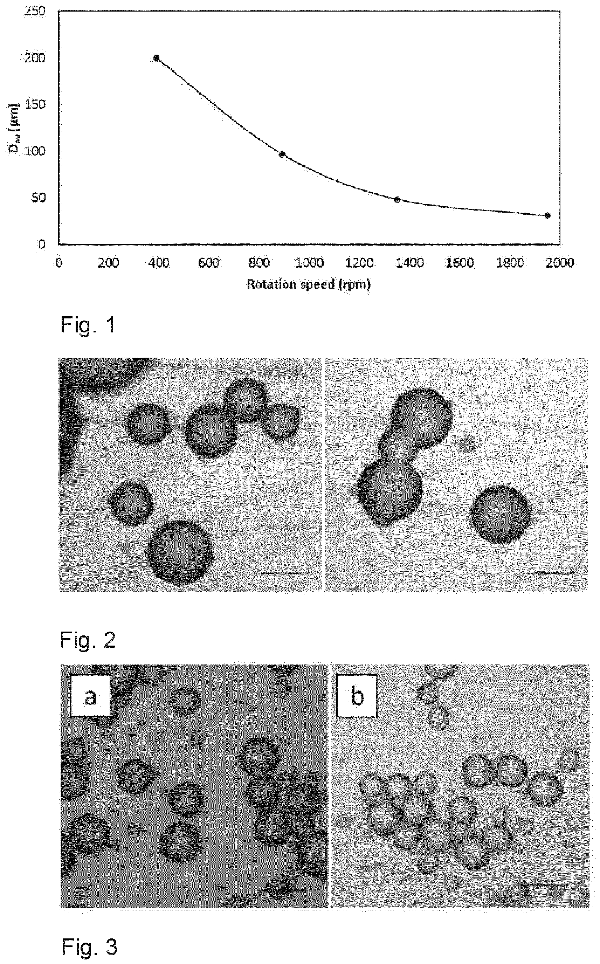

Figure 1 shows influence of rotation speed upon average diameter of alginate particles. Dispersed phase: 1% sodium alginate, continuous phase: 5 wt.% PGPR in Miglyol 840, membrane: full membrane of 15 $\mu$m pore diameter and 200 $\mu$m pore spacing, injection rate: 1 ml/min.

Figure 2 shows representative microphotographs of an emulsion created with the following conditions - Dispersed phase: 1% NFC, continuous phase: 5 wt.% PGPR in Miglyol 840, membrane: full membrane of 15 $\mu$m pore diameter and 200 $\mu$m pore spacing, rotation speed: 1350 rpm, injection rate 1 ml/min. Scale bar = 100 $\mu$m.

Figure 3 shows representative microphotographs of an emulsion A) immediately after emulsification and B) after 30 minutes of standing. Emulsion was produced using dispersed phase: 0.5% NFC, continuous phase: 5 wt.% PGPR in Miglyol 840, membrane: full membrane of 15 $\mu$m pore diameter and 200 $\mu$m pore spacing, rotation speed: 1350 rpm, injection rate 1 ml/min. Scale bar = 100 $\mu$m.

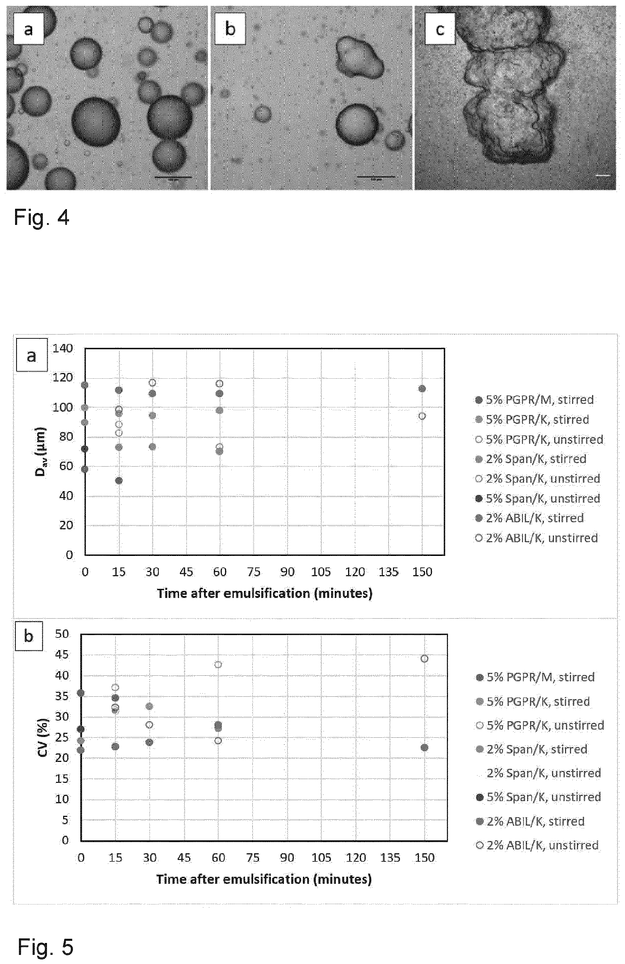

Figure 4 shows representative microphotographs of an emulsion A) immediately after emulsification, B) after 15 minutes of stirring and C) after 15 minutes of standing. Emulsion was produced using dispersed phase: 0.5% NFC, continuous phase: 5 wt.% PGPR in Miglyol 840, membrane: ring membrane of 15 $\mu$m pore diameter and 200 $\mu$m pore spacing, rotation speed: 1350 rpm, injection rate 1 ml/min. Scale bar = 100 $\mu$m.

Figure 5 shows 0.5% NFC particle (A) average size ($D_{av}$) and (B) coefficient of variation (CV) in different continuous phase formulations over time. 5% PGPR/M, 5 wt.% PGPR in Miglyol 840; 5% PGPR/K, 5 wt.% PGPR in kerosene; 2% Span/K, 2 wt.% Span 80 in kerosene; 5% Span/K, 5 wt.% Span 80 in kerosene; 2% ABIL/K, 2 vol.% ABIL EM 90 in kerosene. Operating conditions of production: 0.5% NFC in water dispersed phase, 15 $\mu$m pore diameter ring membrane, 1350 rpm rotation speed, 1 ml/min injection rate.

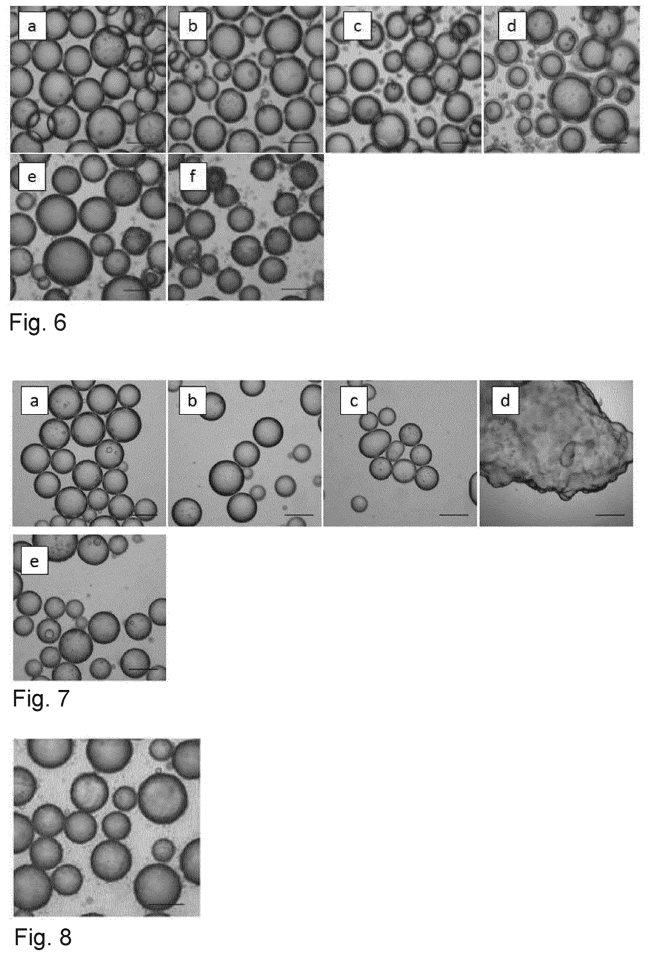

Figure 6 shows representative microphotographs of an emulsion A) immediately after emulsification or continuously stirred for B) 15 minutes, C) 30 minutes and D) 60 minutes or left to stand for E) 15 minutes and F) 60 minutes. Emulsion was produced using dispersed phase: 0.5% NFC in water, continuous phase: 5 wt.% PGPR in kerosene, membrane: ring membrane of 15 $\mu$m pore diameter and 200 $\mu$m pore spacing, rotation speed: 1350 rpm, injection rate 1 ml/min. Scale bar = 100 $\mu$m.

Figure 7 shows representative microphotographs of an emulsion A) immediately after emulsification or continuously stirred for B) 15 minutes, C) 30 minutes and D) 60 minutes or E) left to stand for 15 minutes. Emulsion was produced using dispersed phase: 0.5% NFC in water, continuous phase: 2 wt.% Span 80 in kerosene, membrane: ring membrane of 15 $\mu$m pore diameter and 200 $\mu$m pore spacing, rotation speed: 1350 rpm, injection rate 1 ml/min. Scale bar = 100 $\mu$m.

Figure 8 shows representative microphotograph of an emulsion imaged immediately after emulsification, created with the following conditions - Dispersed phase: 0.5% NFC in water, continuous phase: 5 wt.% Span 80 in kerosene, membrane: ring membrane of 15 $\mu$m pore diameter and 200 $\mu$m pore spacing, rotation speed: 1350 rpm, injection rate 1 ml/min. Scale bar = 100 $\mu$m.

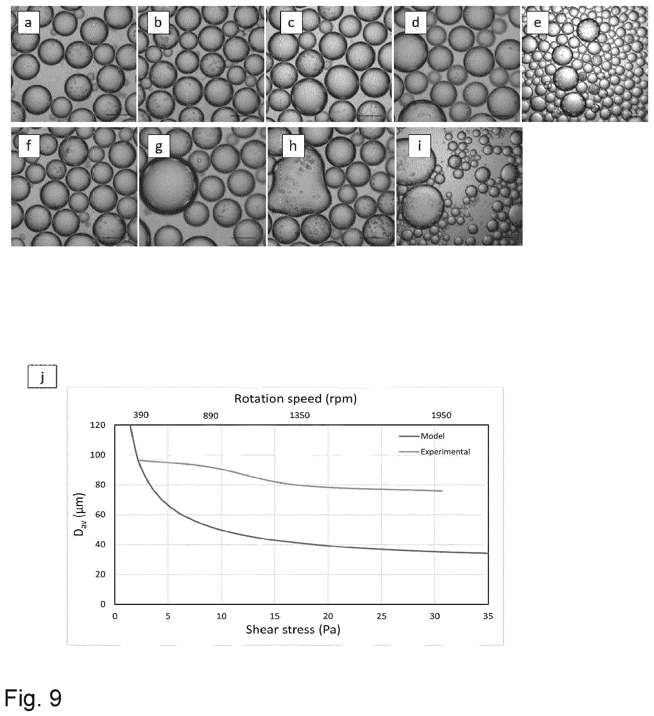

Figure 9 shows representative microphotographs of an emulsion A) immediately after emulsification or continuously stirred for B) 15 minutes, C) 30 minutes, D) 60 minutes and E) 150 minutes or left to stand for F) 15 minutes, G) 30 minutes, H) 60 minutes and I) 150 minutes. J) Average particle diameter versus shear stress determined experimentally ("Experimental") by varying the rotation speed from 390 to 1950 rpm, and by mathematical modelling ("Model", lower curve). Emulsions were produced using dispersed phase: 0.5% NFC in water, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bar = 100 $\mu$m.

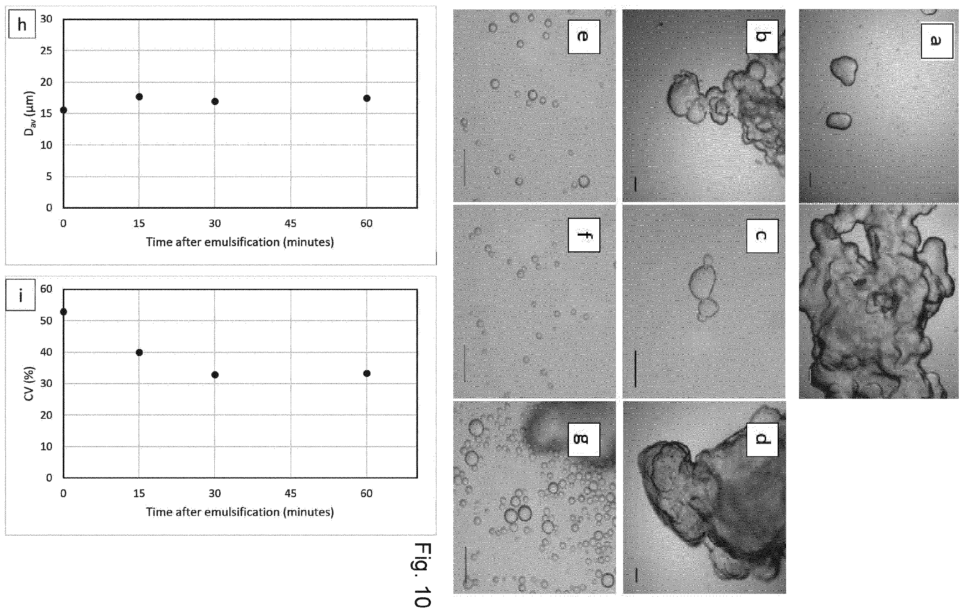

Figure 10 shows representative microphotographs of an emulsion A) immediately after emulsification or continuously stirred for B) 15 minutes, C) 30 minutes and D) 60 minutes or left to stand for E) 15 minutes, F) 30 minutes, G) 60 minutes. H) Average particle diameter ($D_{av}$) and I) coefficient of variation (CV) of the unstirred emulsion over time. Emulsion was produced using dispersed phase: 1.5% NFC in water, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bar = 100 $\mu$m.

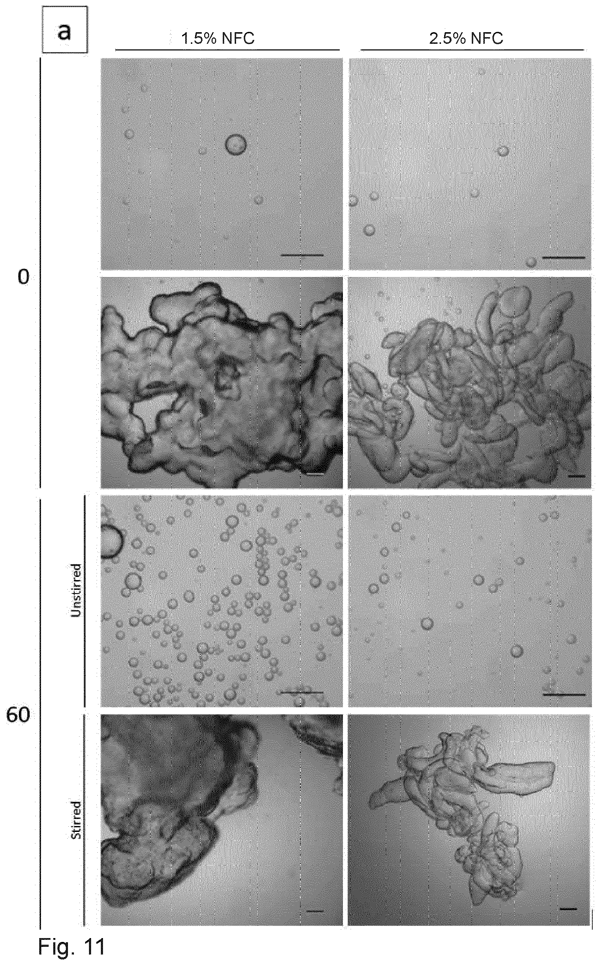

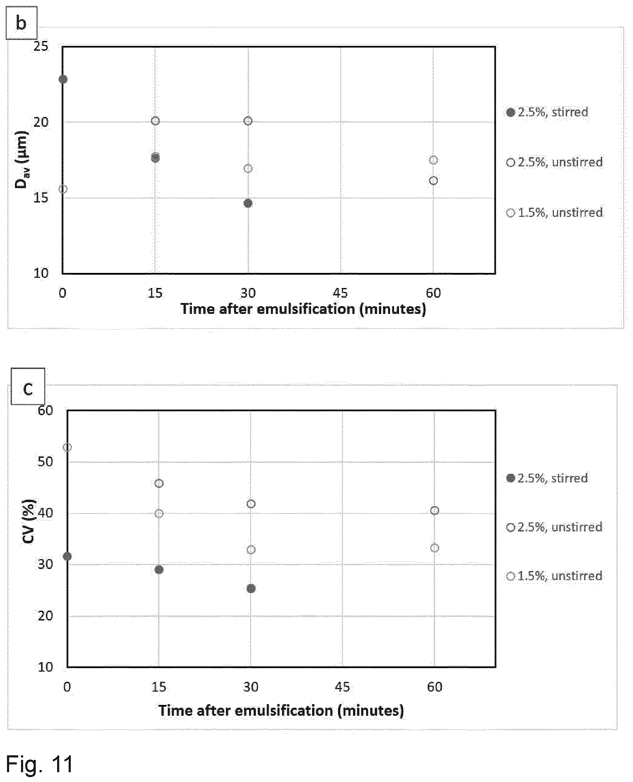

Figure 11 shows emulsions of undiluted NFC at concentrations of 1.5 and 2.5% in 2% ABIL EM 90 in kerosene continuous phase. A) Representative photographs of 1.5 and 2.5% NFC particles at 0 and 60 minutes after emulsification, both stirred and unstirred. Scale bars, 100 $\mu$m. B) Average particle diameter ($D_{av}$) over 60 minutes. C) Average coefficient of variation (CV) over 60 minutes. $D_{av}$ and CV for stirred 1.5% NFC emulsion could not be obtained due to insufficient number of particles. Emulsion was produced using dispersed phase: 1.5% or 2.5% NFC, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min.

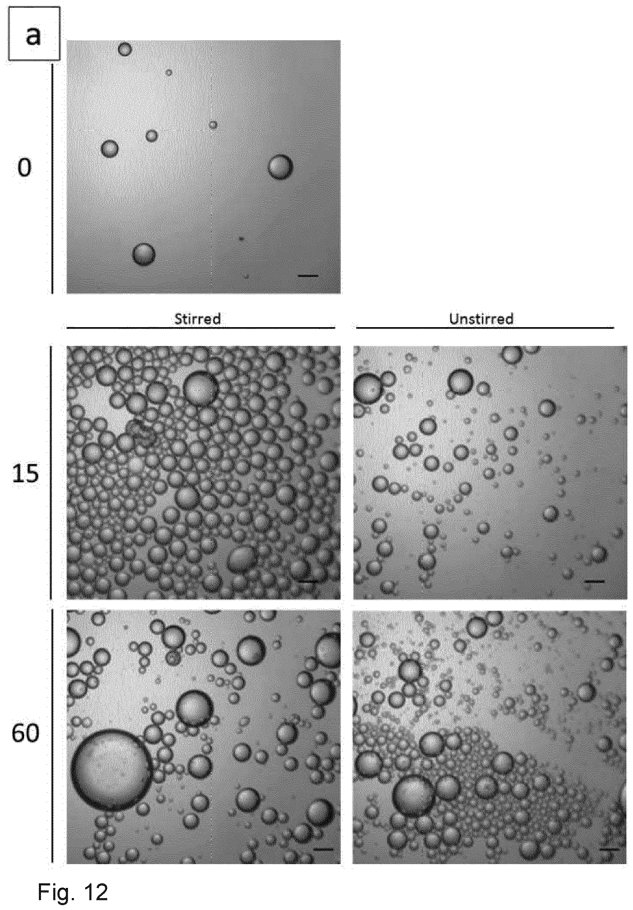

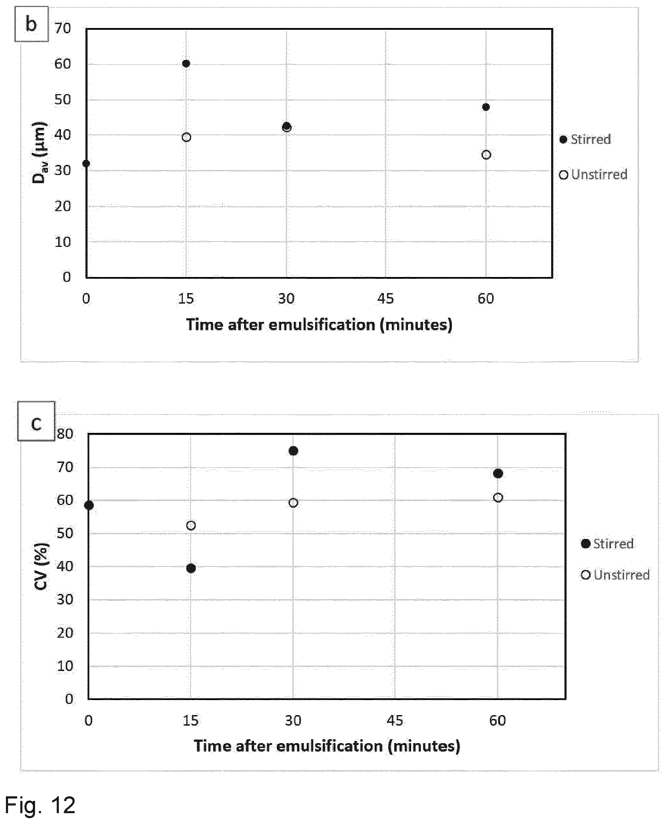

Figure 12 shows emulsions of 2% NFC diluted with basal medium (DMEM). A) Representative photographs of 2% NFC particles at 0, 15 and 60 minutes after emulsification, both stirred and unstirred. B) Average particle diameter ($D_{av}$) over 60 minutes. C) Average coefficient of variation (CV) over 60 minutes. Emulsion was produced using dispersed phase: 2% NFC in DMEM, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bars, 100 $\mu$m.

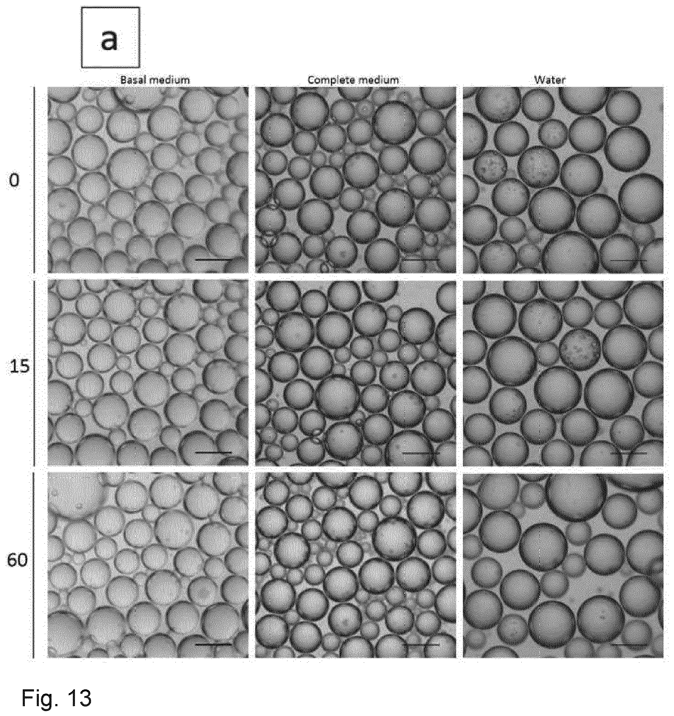

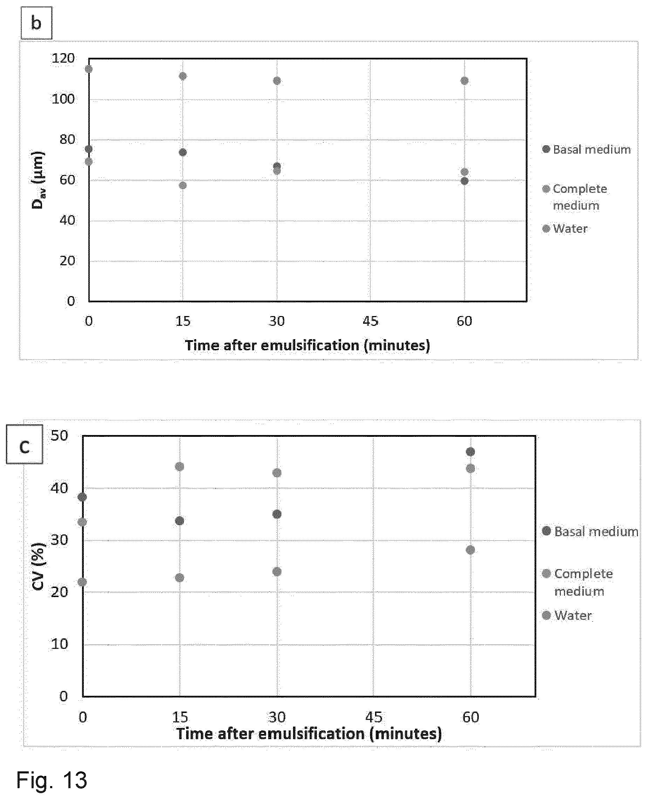

Figure 13 shows emulsions of 0.5% NFC diluted with basal medium (DMEM), complete medium or water. A) Representative photographs of 0.5% NFC particles diluted with basal medium, complete medium or water at 0, 15 and 60 minutes after emulsification. All emulsions were stirred. B) Average particle diameter ($D_{av}$) over 60 minutes, compared to 0.5% NFC particles diluted with distilled water. C) Average coefficient of variation (CV) over 60 minutes, compared to 0.5% NFC particles diluted with distilled water. Emulsion was produced using dispersed phase: 0.5% NFC in DMEM, or complete medium or distilled water, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bars, 100 $\mu$m.

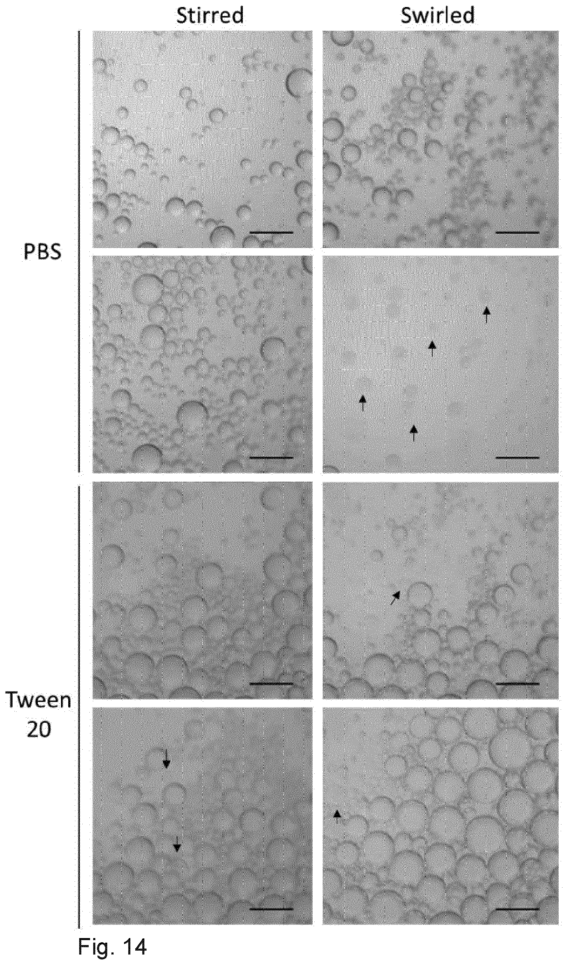

Figure 14 shows representative photographs of an emulsion of 0.5% NFC diluted with basal medium (DMEM). The emulsion was washed either in PBS or 2% Tween 20 in water and agitated by swirling or stirring gently. Black arrows indicate flattened, pink-tinged particles. Emulsion was produced using dispersed phase: 0.5% NFC in DMEM, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bars, 100 $\mu$m.

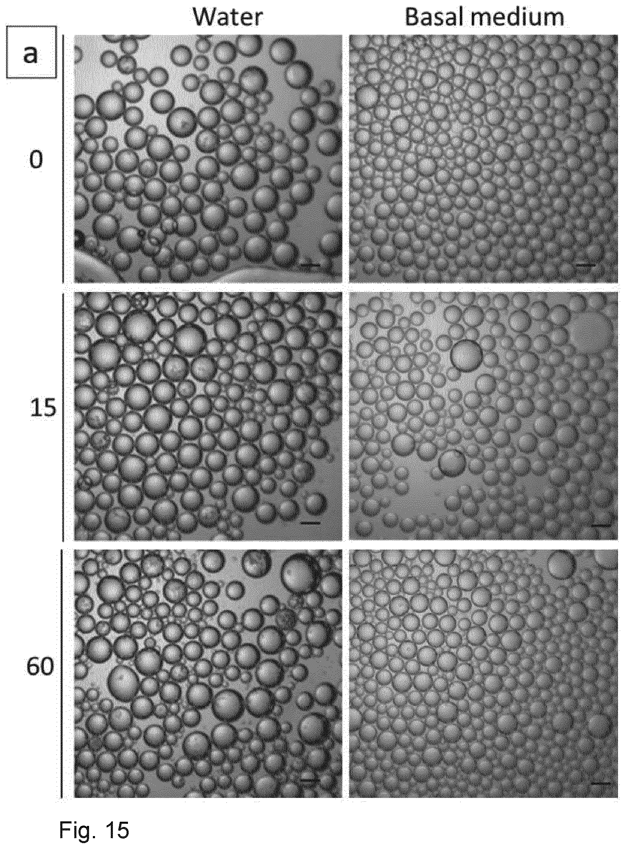

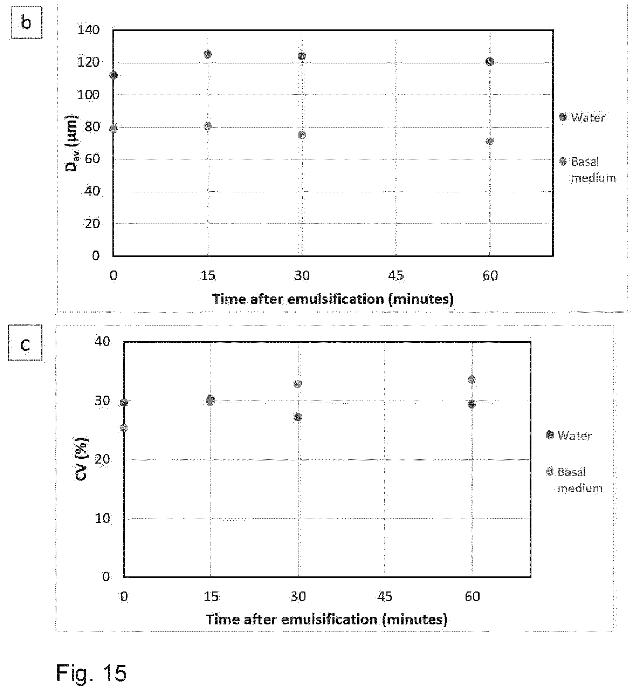

Figure 15 shows emulsions of 0.5% ANFC diluted with basal medium or water. A) Representative photographs of 0.5% ANFC particles diluted with basal medium or water at 0, 15 and 60 minutes after emulsification. All emulsions were stirred. B) Average particle diameter ($D_{av}$) over 60 minutes. C) Average coefficient of variation (CV) over 60 minutes. Emulsion was produced using dispersed phase: 0.5% ANFC in DMEM or water, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bars, 100 $\mu$m.

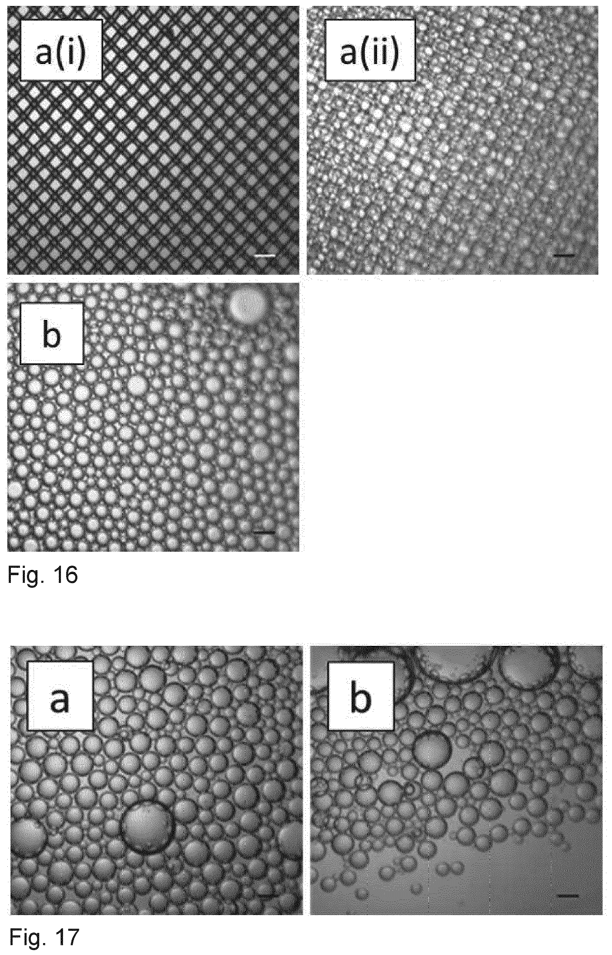

Figure 16 shows emulsion of 0.5% ANFC diluted with basal medium (DMEM) and washed. A) Cell strainer mesh without (i) or with (ii) 0.5% ANFC particles. B) Representative photograph of 0.5% ANFC particles recovered from the cell strainer after immersion in PBS. Emulsion was produced using dispersed phase: 0.5% ANFC in DMEM, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bars, 100 $\mu$m.

Figure 17 shows emulsions of 0.7% ANFC diluted with basal medium (DMEM). A) Representative photographs of 0.7% ANFC particles immediately after emulsification. B) Representative photographs of 0.7% ANFC particles after washing and immersion in PBS for 24 hours. Emulsion was produced using dispersed phase: 0.7% ANFC in DMEM, continuous phase: 2 vol.% ABIL EM 90 in kerosene, membrane: ring membrane of 15 $\mu$m pore diameter and 200 $\mu$m pore spacing, rotation speed: 1350 rpm, injection rate 1 ml/min. Scale bars, 100 $\mu$m.

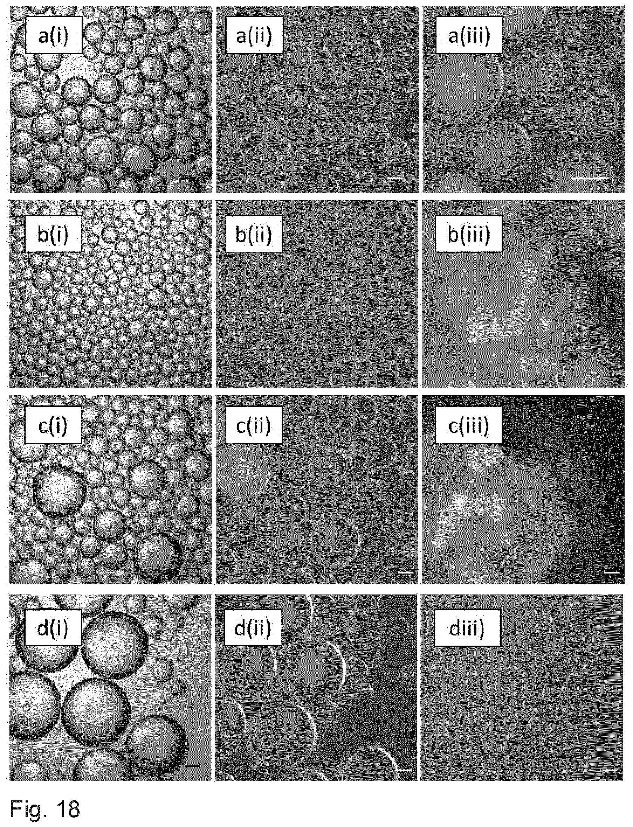

Figure 18 shows emulsions of NFC and ANFC stained with calcofluor white. A) Representative microphotographs of 0.5% NFC particles diluted with water under (i) visible and (ii), (iii) UV light. B) Representative microphotographs of 0.5% ANFC particles diluted with DMEM (i) under visible light, (ii) under UV light and (iii) residual, un-emulsified ANFC under UV light. C) Representative microphotographs of 0.5% ANFC particles diluted with DMEM and emulsified using an increased pore diameter of 40 $\mu$m and injection rate of 5 ml/min, (i) under visible light, (ii) under UV light and (iii) residual, un-emulsified ANFC under UV light. D) Representative microphotographs of 0.5% ANFC diluted with water using an increased pore diameter of 40 $\mu$m and injection rate of 5 ml/min, (i) under visible light, (ii) under UV light and (iii) residual, un-emulsified ANFC under UV light. Emulsions were produced using dispersed phase: 0.5% NFC or ANFC in water or DMEM, continuous phase: 2 vol.% ABIL EM 90 in kerosene, membrane: ring membrane of 15 $\mu$m pore diameter or full membrane of 40 $\mu$m diameter, rotation speed: 1350 rpm, injection rate 1 ml/min or 5 ml/min. Scale bars, 100 $\mu$m.

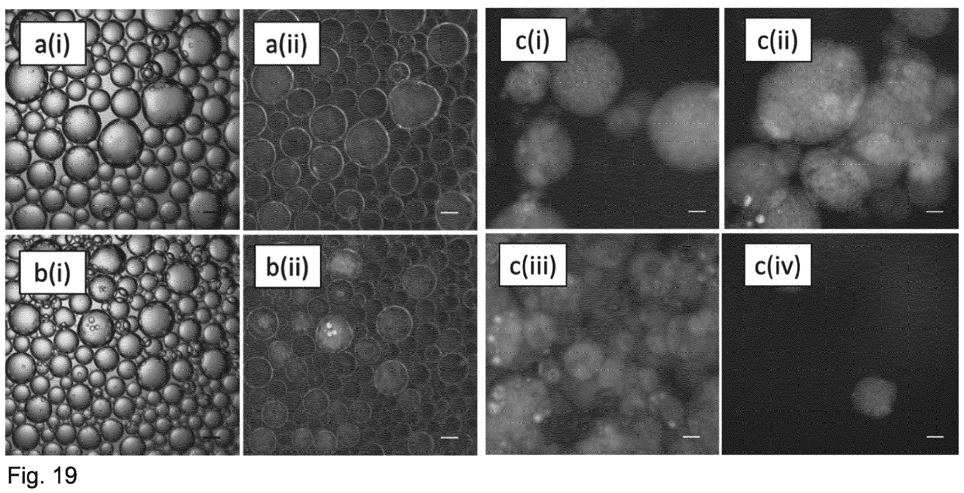

Figure 19 shows emulsions of 0.5% ANFC stained with calcofluor white and diluted with (A) PBS or (B) DMEM and homogenised prior to emulsification. Emulsions visualised under (i) visible light or (ii) UV light. Figure C shows ANFC particles that have been crosslinked in different ways. C(i) shows particles of 0.5% ANFC diluted with PBS that were filtered with a cell strainer and resuspended in 1M CaCl$_2$. C(ii) shows particles of 0.5% ANFC diluted with DMEM that were filtered with a cell strainer and resuspended in 1M CaCl2. C(iii) shows particles of 0.5% ANFC diluted with PBS mixed with a homogenate of 5% 1M CaCl2 in 2% ABIL EM 90 in kerosene. C(iv) shows particles of 0.5% ANFC diluted with PBS that had been stirred with 10% 2M CaCl2 for 15 minutes before filtration and resuspension in PBS. Emulsions were produced using dispersed phase: 0.5% ANFC in PBS or DMEM homogenised for 10 minutes at 6500 rpm with 1% calcfluor white, continuous phase: 2 vol.% ABIL EM 90 in kerosene, membrane: full membrane of 40 $\mu$m pore diameter, rotation speed: 1350 rpm, injection rate 5 ml/min. Scale bars, 100 $\mu$m

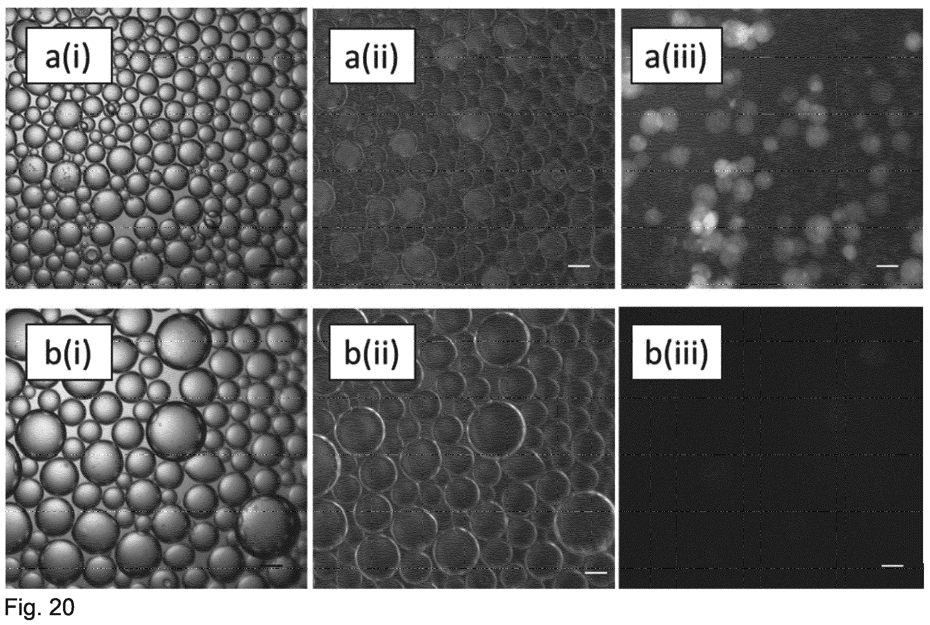

Figure 20 shows A) Particles of 0.5% ANFC diluted with PBS and stained with calcfluor white, made with a 15 $\mu$m ring membrane, in i) emulsion under visible light, ii) emulsion under UV light, iii) in PBS after crosslinking and washing. Emulsion produced using dispersed phase: 0.5% ANFC in PBS homogenised for 10 minutes at 6500 rpm with 1% calcfluor white, continuous phase: 2 vol.% ABIL EM 90 in kerosene, membrane: ring membrane of 15 $\mu$m pore diameter, rotation speed: 1350 rpm, injection rate 5 ml/min. B) Particles of 0.5% NFC diluted with DMEM and stained with calcfluor white, made with a 40 $\mu$m full membrane, in i) emulsion under visible light, ii) emulsion under UV light, iii) in DMEM after crosslinking and washing. Emulsion produced using dispersed phase: 0.5% ANFC in DMEM homogenised for 10 minutes at 6500 rpm with 1% calcfluor white, continuous phase: 2 vol.% ABIL EM 90 in kerosene, membrane: full membrane of 40 $\mu$m pore diameter, rotation speed: 1350 rpm, injection rate 10 ml/min. Scale bars, 100 $\mu$m

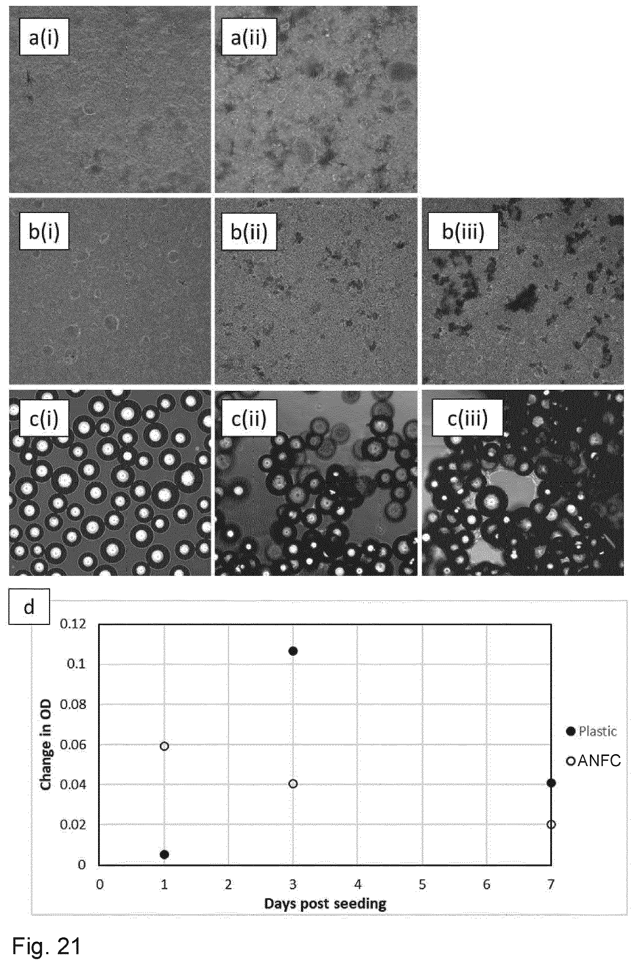

Figure 21 shows ANFC and plastic microcarriers. A) ANFC microcarriers i) before and ii) after filtration. B) ANFC microcarriers i) without cells, ii) at day 3 after cell seeding and iii) at day 7 after cell seeding. C) Plastic microcarriers i) without cells, ii) at day 3 after cell seeding and iii) at day 7 after cell seeding. D) Cell viability measured in change of optical density (OD) compared to no cell control for ANFC and plastic microcarriers over seven days.

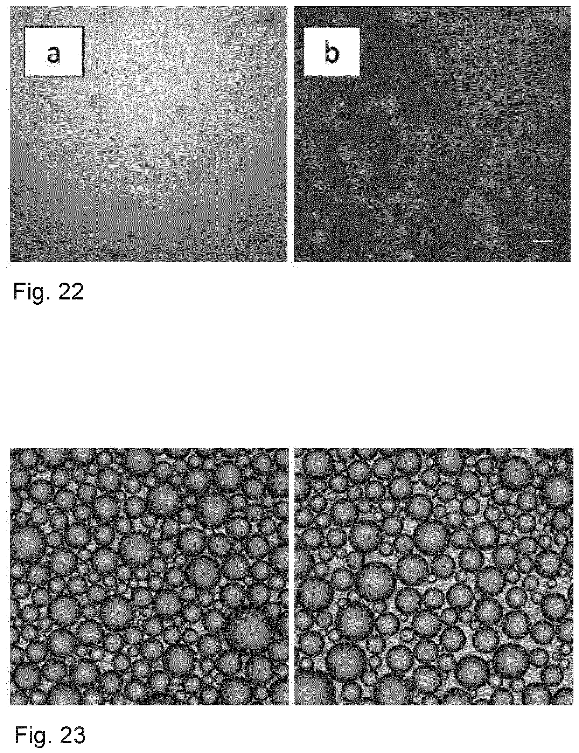

Figure 22 shows ANFC microcarriers stained with 1% calcfluor white, washed using 2% Tween 20 in PBS and resuspended in PBS, visualised under A) visible light or B) UV light. Scale bars, 100 $\mu$m

Figure 23 shows hMSCs encapsulated within ANFC particles. Emulsion produced using dispersed phase: 0.5% ANFC in DMEM homogenised for 10 minutes at 6500 rpm, with 300,000 cells ml-2, continuous phase: 2 vol.% ABIL EM 90 in kerosene, membrane: full membrane of 40 $\mu$m pore diameter, rotation speed: 1350 rpm, injection rate 10 ml/min.

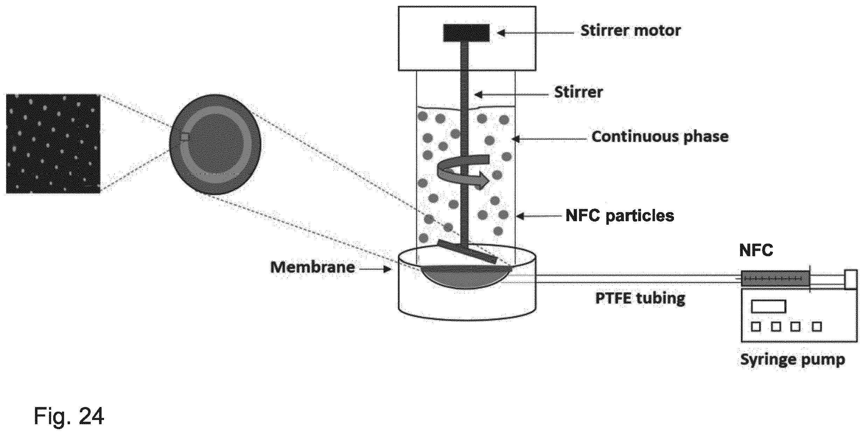

Figure 24 shows a schematic diagram of the dispersion cell used for membrane emulsification.

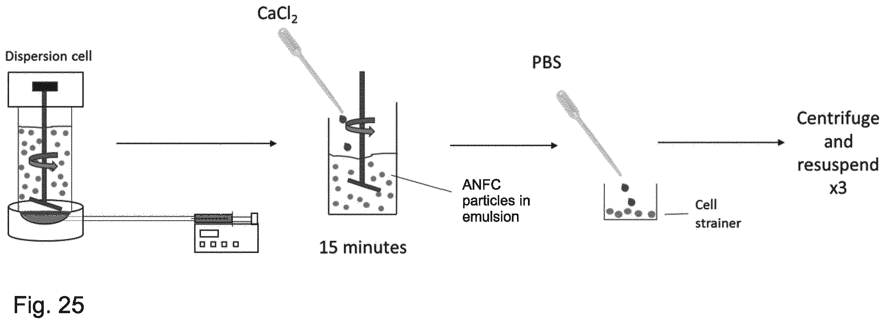

Figure 25 shows a successful ANFC particle crosslinking and washing procedure.

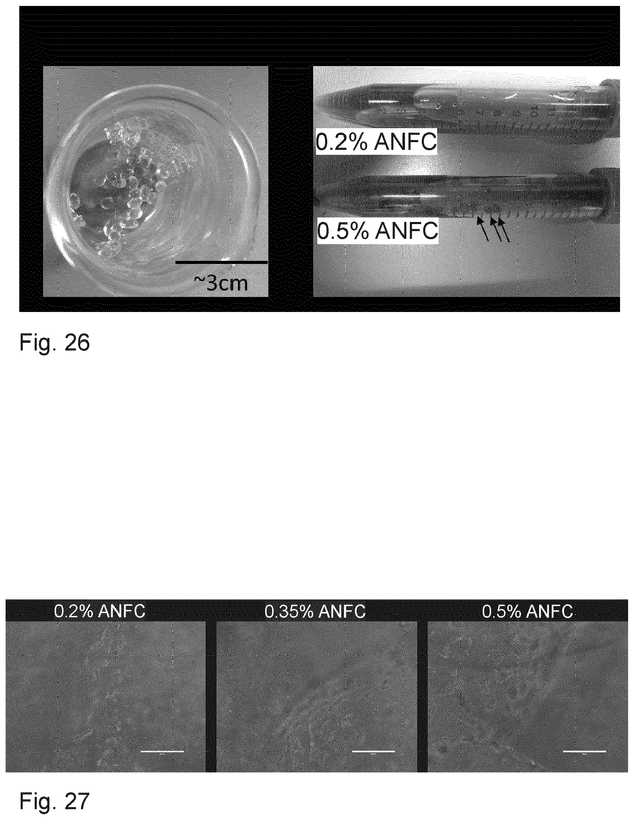

Figure 26 shows A: ANFC particles produced by ionic cross-linking of 0.5% NFC droplets. B: ANFC particles stained with calcfluor white after centrifugation at 1000G for 2 minutes. Black arrows point to three of the particles. Particles made from 0.2% ANFC have been completely disintegrated.

Figure 27 shows HEK293T cells growing on cross-linked ANFC particles, 2 days post-seeding. White scale bar = 400 $\mu$m.

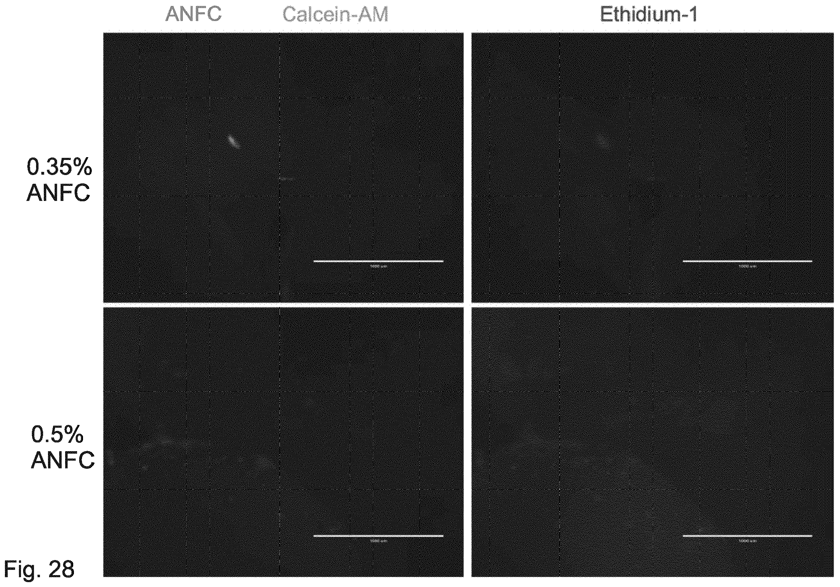

Figure 28 shows HEK293T cells on ANFC (blue) particles, 5 days post-seeding. Calcein-AM (green) indicates live cells, punctate Ethidium-1 staining (red) (largely absent in the images) indicates cell death. White scale bar = 1mm.

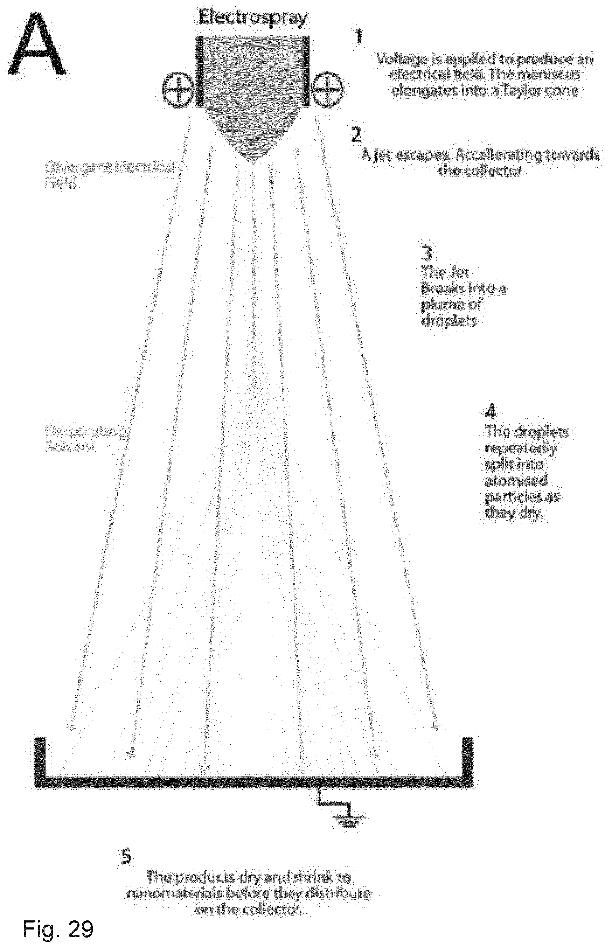

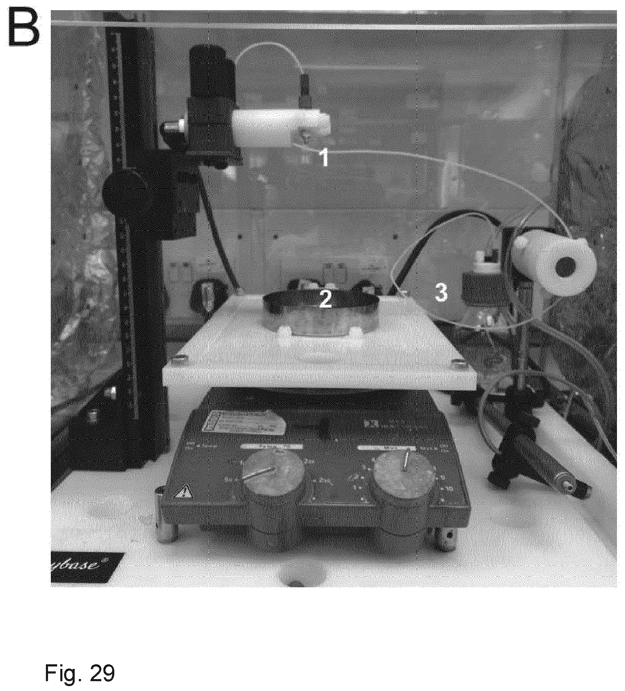

Figure 29 shows A: Schematic explanation of electrospraying using SprayBase. B: Current electrospraying set-up.

1: Emitter; 2: Collection plate; 3: Pressure vessel containing liquid to be sprayed. The collection plate is mounted on top of a magnetic stirrer.

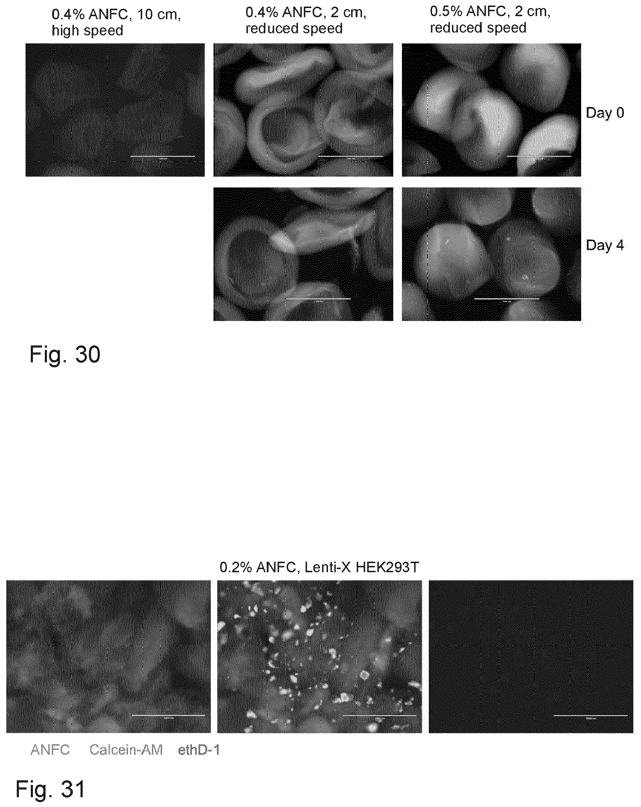

Figure 30 shows ANFC particles produced by using the electrosprayer to generate small droplets, followed by ionic cross-linking. Particles were stained with calcofluor white and imaged with a fluorescence microscope. Text above the images indicate % ANFC used, the distance between emitter and collector and an indication of the stirring speed used. Day 0: ~1 hour after cross-linking the particles. Day 4: Following 3 days in crosslinking solution and 4 days in DMEM after washing off cross-linking solution. White scale bar: 1 mm

Figure 31 shows HEK293T cells on ANFC particles, 5 days post-seeding. Calcein-AM indicates live cells, punctuate Ethidium-1 staining (largely absent in the images) indicates cell death. White scale bar = 1 mm.

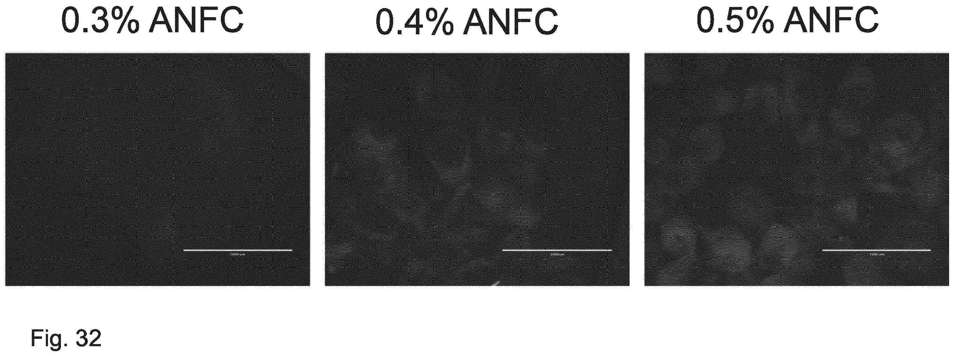

Figure 32 shows ANFC particles produced by electrospraying followed by ionic cross-linking. Particles were stained with calcofluor white and imaged with a fluorescence microscope. White scale bar: 1 mm

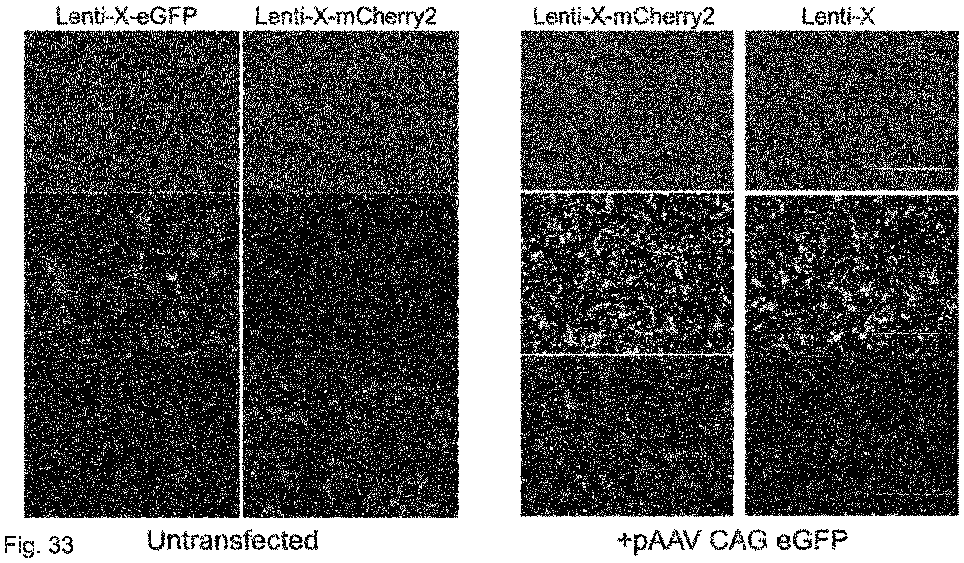

Figure 33 shows transfection of stable, fluorescent protein expressing LentiX cells. Left panel: Untransfected, clonal LentiX cells, stably expressing either GFP or mCherry. LentiX-GFP cells show fluorescence in the red channel, whereas LentiX-mCherry cells do not show fluorescence in the green channel. Right panel: LentiX and LentiX-mCherry cells were transfected with a plasmid encoding GFP (visualised in the green channel, middle row) to test transfection efficiency. White scale bar = 1000 $\mu$m. All images were taken at same magnification.

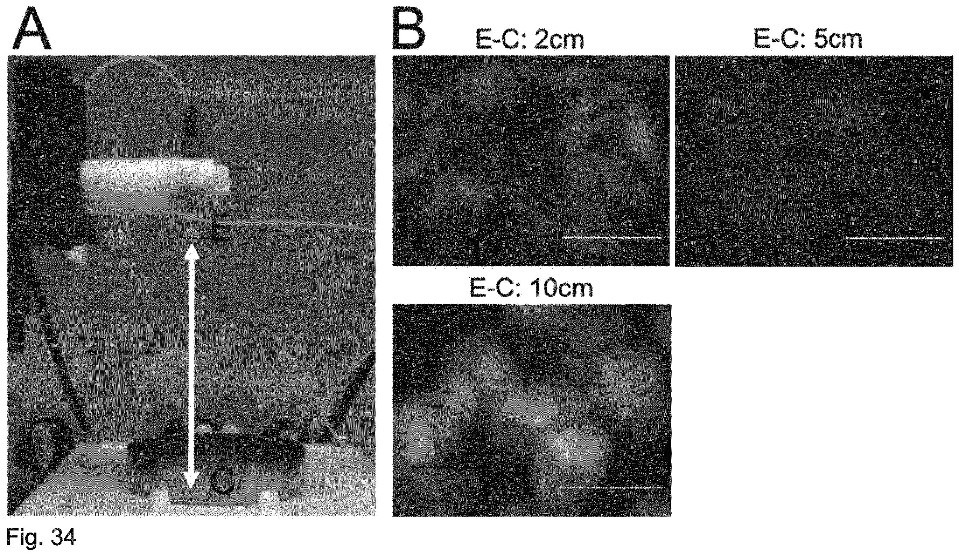

Figure 34 shows the effect of emitter-collector distance on the shape of electrosprayed ANFC particles A: Picture of the electrospraying setup, with needle emitter (E) and collector plate (C) indicated. Emitter-collector distance (E-C) is indicated by the white arrow. B: ANFC (0.5%) pre-stained with calcofluor white was electrosprayed in 0.1 M CaCl$_2$ at 10 kV with E-C distances shown above the images. Particles were imaged on an epifluorescence microscope. White scale bar = 1000 $\mu$m.

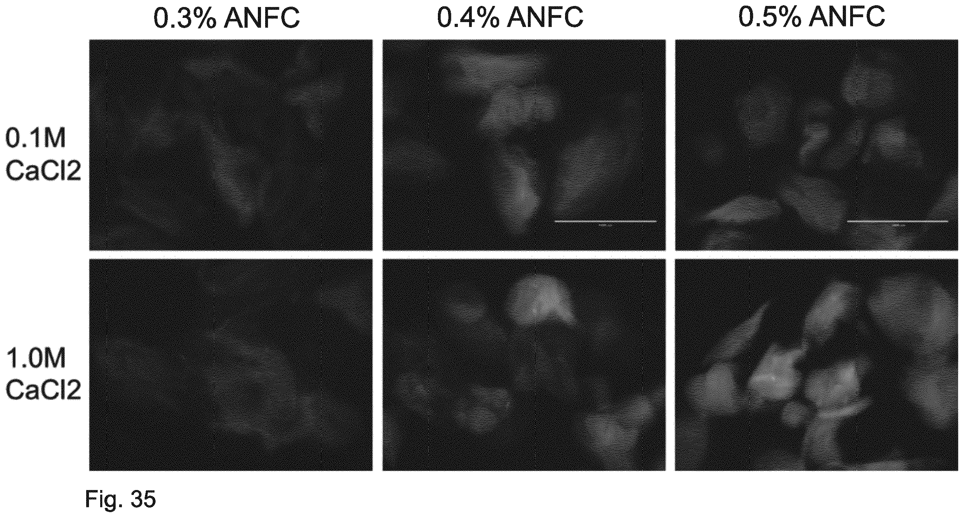

Figure 35 shows the effect of CaCl2 concentration on the size and shape of electrosprayed ANFC particles. ANFC pre-stained with calcofluor white was electrosprayed into 0.1 M or 1.0 M CaCl2 solution at 10 kV, using an emitter-collector distance of 5 cm. Particles were imaged on an epifluorescence microscope. White scale bar = 1000 $\mu$m.

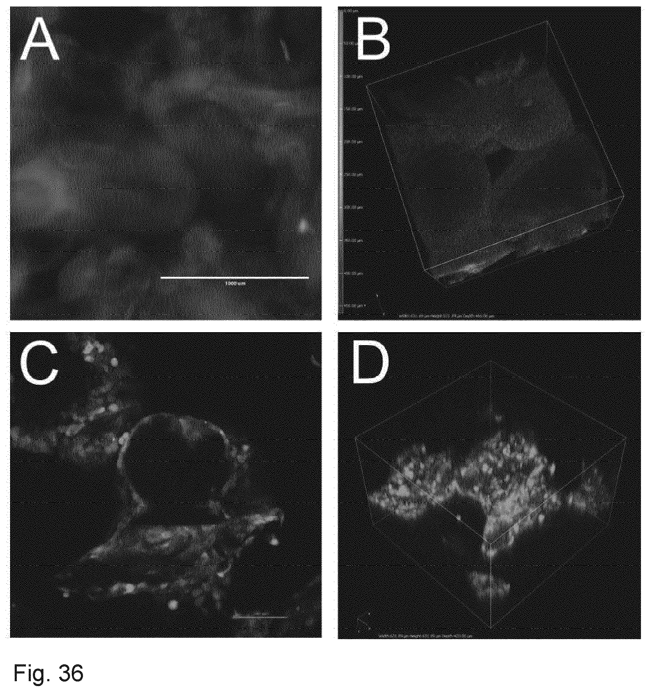

Figure 36 shows confocal imaging of electrosprayed pre-stained ANFC particles. A: ANFC (0.5%) pre-stained with calcofluor white was electrosprayed into 0.1M CaCl$_2$ solution at 10 kV, emitter-collector distance: 2 cm. Particles were imaged on an epifluorescence microscope. White scale bar = 1000 $\mu$m. B: 3D reconstruction of confocal z-stack images of particles shown in A. Colour is depth-coded (blue: top, red: bottom). Dimensions of the white box: 631 $\mu$m $\times$ 631 $\mu$m $\times$ 460 $\mu$m. C: Confocal image of LentiX-mCherry cells growing on ANFC particles shown in A and B. Blue: ANFC, Orange: LentiX-mCherry cells. Red scale bar is 100 $\mu$m. D: 3D reconstruction of z-stack images of LentiX-mCherry cells growing on ANFC particles (3 days after seeding) shown in A and B. Blue: ANFC, Orange: LentiX-mCherry cells. Dimensions of the white box: 631 $\mu$m $\times$ 631 $\mu$m $\times$ 420 $\mu$m.

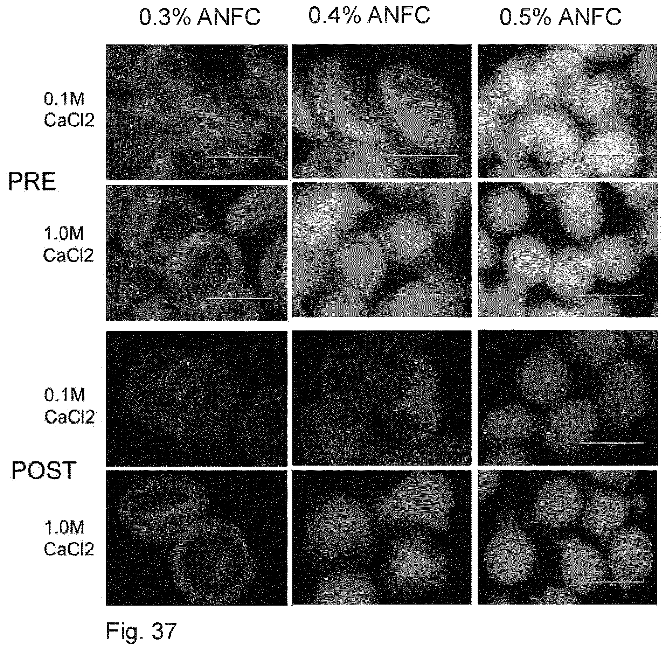

Figure 37 shows the effect of autoclaving on the ANFC particles. ANFC prestained with calcofluor white was electrosprayed in 0.1 M or 1.0 M CaCl2 at 4.5 kV, using an emitter-collector distance of 2 cm. Particles were imaged on an epifluorescence microscope. White scale bar = 1000 $\mu$m. All images taken at the same magnification. Particles are shown before (PRE) and after autoclaving (POST).

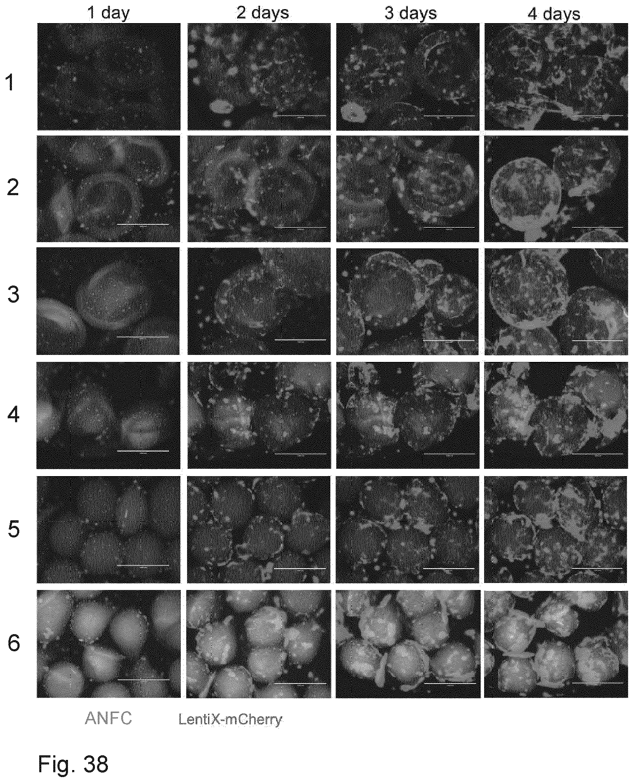

Figure 38 shows LentiX-mCherry cell growth on pre-stained, autoclaved ANFC particles. Example images of LentiX-mCherry cells growing on ANFC particles. Images were taken 1-4 days after seeding as indicated at the top of the figure. 1) 0.3% ANFC, 0.1 M CaCl2, 2) 0.3% ANFC 1.0 M CaCl2, 3) 0.4% ANFC, 0.1 M CaCl2, 4) 0.4% ANFC, 1.0 M CaCl2, 5) 0.5% ANFC, 0.1 M CaCl2, 6) 0,5% ANFC, 1.0 M CaCl2. Blue = ANFC, red = LentiX-mCherry cells. White scale bar is 1000 $\mu$m.

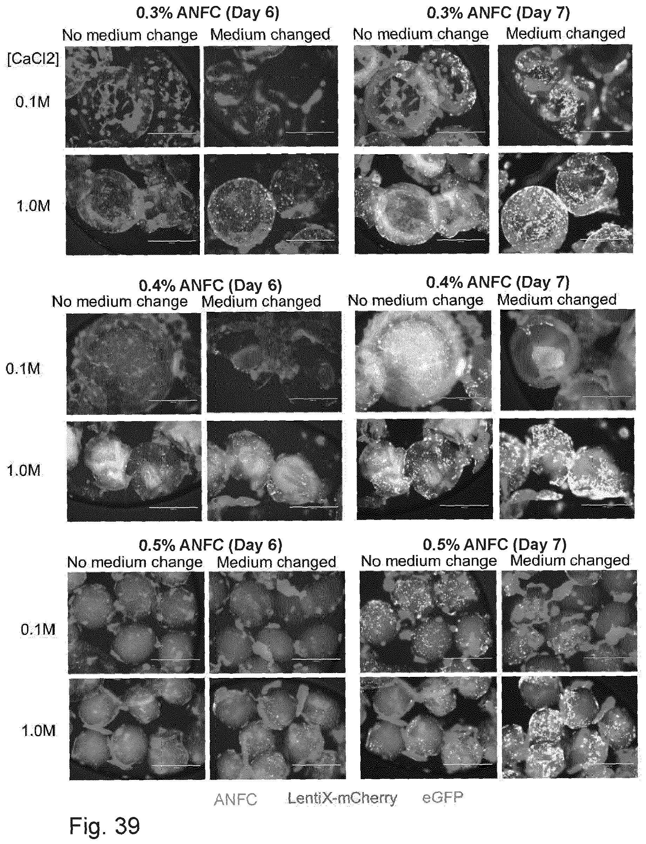

Figure 39 shows transfection of LentiX-mCherry cells growing on ANFC particles. Epifluorescence images showing GFP expression in LentiX-mCherry cells growing on pre-stained ANFC particles on day 1 and day 2 after transfection (day 6 and day 7 of the culture, respectively). For each condition, the same particles were imaged on day 6 and day 7. White scale bar = 1000 $\mu$m.

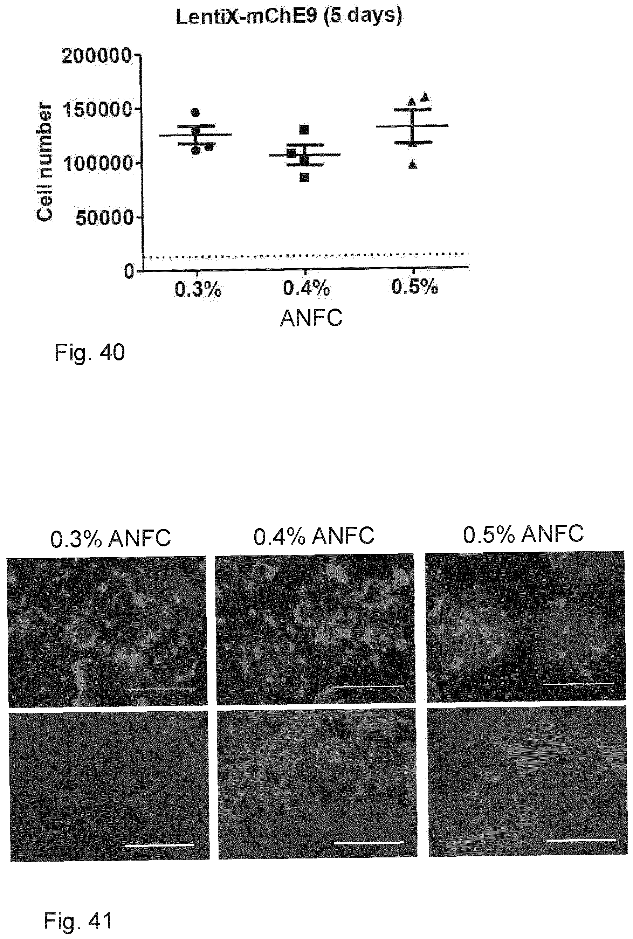

Figure 40 shows LentiX cell proliferation on ANFC beads. LentiX cells were seeded in a low-adherence 96-well TC plate at 12600 cells/well (indicated by the dotted line in the graph) on 75 $\mu$l of ANFC beads suspension (150 $\mu$l total

culture volume). ANFC beads were produced using 0.3%, 0.4% or 0.5% ANFC. Cells were grown for 5 days, before addition of 0.8 mg/ml dispase (final concentration) to detach the cells from the beads. Cells were counted on a haemocytometer. N=4 for each condition. Data are shown as mean $\pm$ SD and as individual data points. Differences between groups were tested by One-Way ANOVA followed by Tukey's post-hoc test with $p<0.05$ as the threshold for statistically significant differences. No significant differences were detected.

Figure 41 shows LentiX-mCherry cells on ANFC beads before transfection with lentiviral plasmids. Epifluorescence and phase contrast imaging of lentix-mCherry cells (red in top image row) on beads (blue in top image row) after 5 days culture, before being transfected with lentiviral plasmids. White scale bar is 1000 $\mu$m.

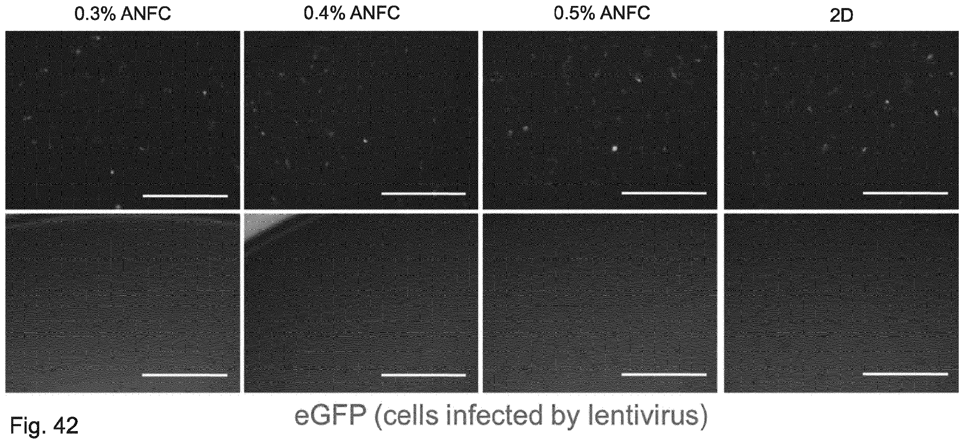

Figure 42 shows Lenti-X cells infected with viral supernatants from transfected cells on ANFC beads. Epifluorescence and phase contrast imaging of Lenti-X cells infected with viral supernatant (1:2 dilution in growth medium) containing GFP-encoding recombinant lentivirus particles. Cells were imaged after overnight incubation with the viral supernatant. The culture condition from which the viral supernatant was obtained is indicated above the images. All seeded cells displayed GFP expression. White scale bar = 400 $\mu$m

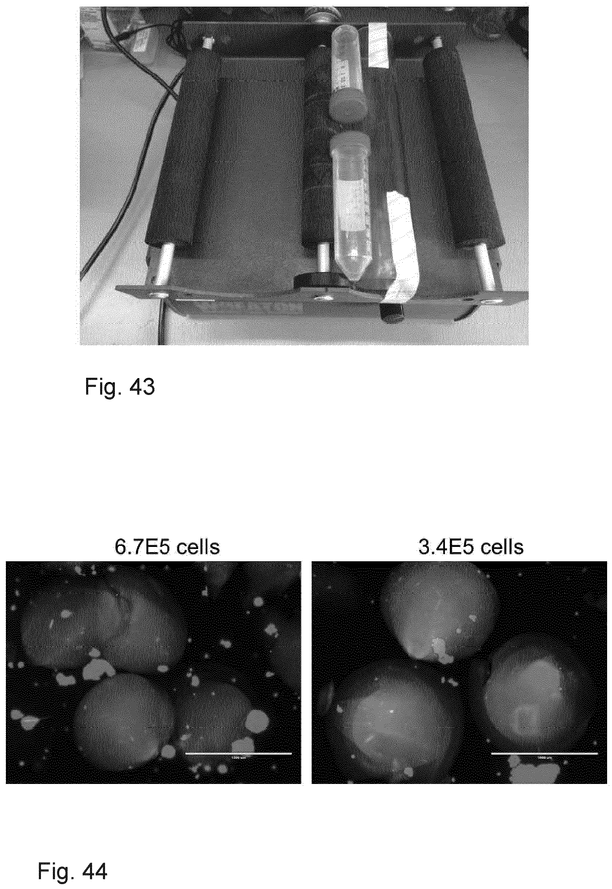

Figure 43 shows mini-roller bottle set-up. A roller bottle system has been adapted to fit 50 ml tubes, to allow low volume cultures under continuous rotation. Tube will be fitted with ventilated caps and the set up placed into a humidified and temperature/$CO_2$-controlled tissue culture incubator for culture of the cells.

Figure 44 shows LentiX cells on ANFC beads after overnight 'mini-roller' culture $6.7 \times 10^5$ or $3.4 \times 10^5$ cells were seeded in 50 ml filter cap tubes on 5 ml or 7.5 ml 0.5% ANFC bead suspension, respectively, in growth medium. Total culture volume was 10 ml. Tubes were placed on a roller bank, and rotated at ~1 RPM overnight. A small amount of beads plus medium was removed from the tubes the next day and imaged on an epifluorescence microscope. Red: LentiX-mCherry cells. Blue: ANFC. Scale bar is 1000 $\mu$m.

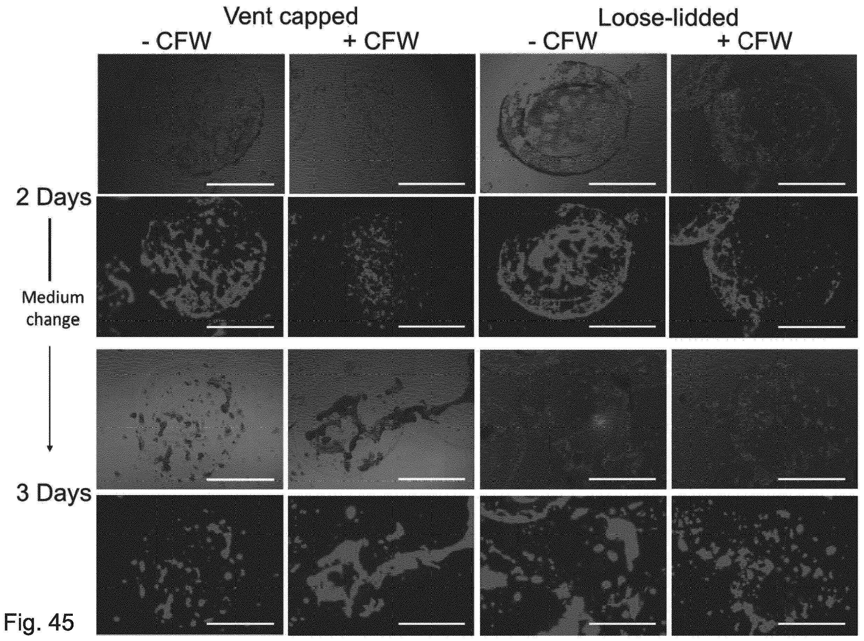

Figure 45 shows LentiX-mCherry cells growing on 0.4% ANFC beads in 50 ml tubes. Phase contrast and epifluorescence imaging of LentiX-mCherry cells on 0.4% ANFC beads. $2 \times 10^6$ Cells were seeded on 3 ml of 0.4% ANFC beads in growth medium in a total culture volume of 10 ml in a 50 ml tube. The medium was changed after 2 days of culture. Tubes were vent capped or loosened regular caps. Beads were produced with or without calcofluor white (CFW) prestaining. White scale bar is 1000 $\mu$m.

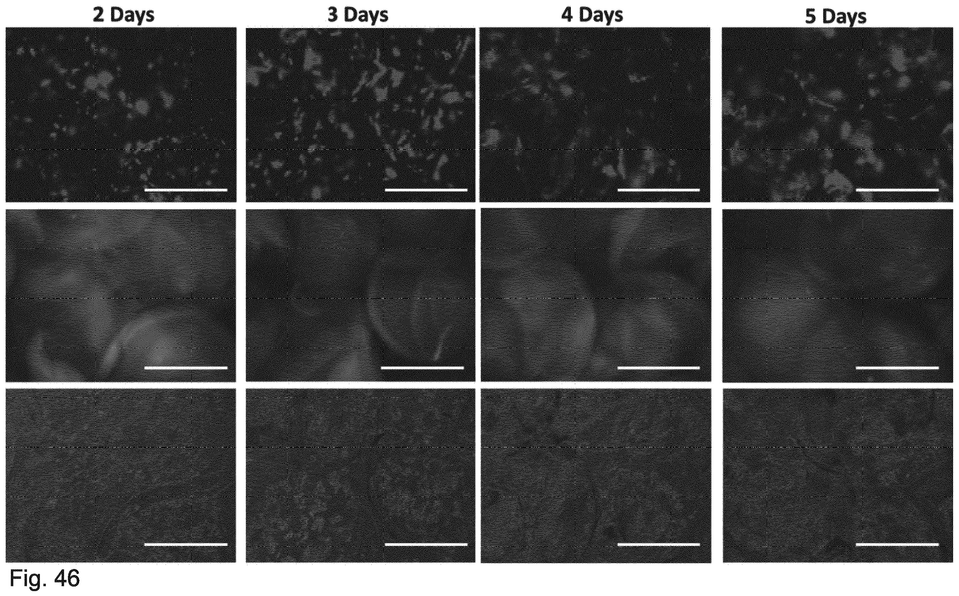

Figure 46 shows LentiX-mCherry cells growin on 0.4% ANFC beads in T25 flasks. Epifluorescence and phase-contrast imagiong of LentiX-mCherry cells (red) on 0.4% ANFC beads pre-stained with calcofluor white (blue). Culture time is indicated above the images. In the experiment shown here, $2 \times 10^6$ cells were seeded on 3 ml of beads in a 10 ml total culture volume in a T25 flask. Daily medium changes were performed on the cells from day 3 onwards. White scale bar = 1000 $\mu$m.

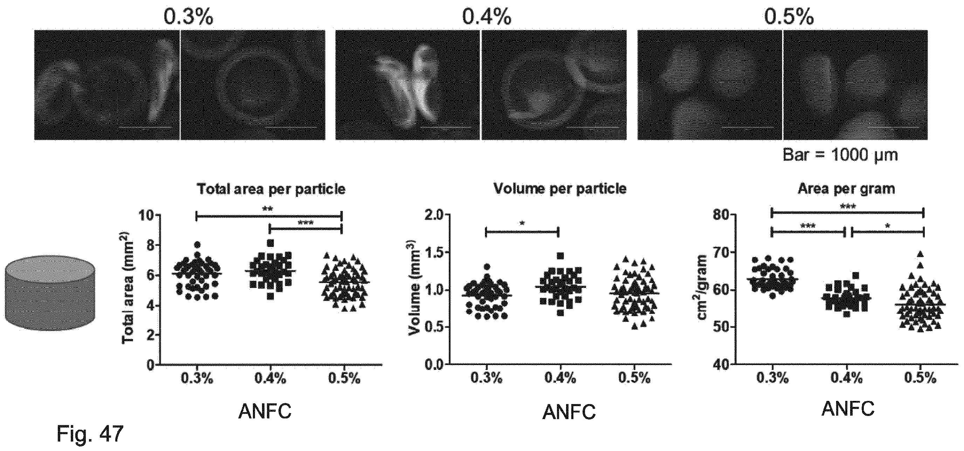

Figure 47 shows measurement of ANFC beads surface area and volume. Top row shows epifluorescence images of ANFC beads stained with calcofluor white (blue). The shape of the beads was approximated by a cylinder and the surface area and volume of individual beads were calculated from measurements of the top area and an average height of the beads of different percentages of ANFC. The density of the beads was assumed to be 1.05 g/ml, i.e. slightly higher than that of water. Graphs show data as mean (horizontal lines) and individual data points. Differences between beads of different percentages of ANFC were tested by One-Way ANOVA with Tukey's multiple comparison's post-hoc test. *$p<0.05$; **$p<0.01$; ***$p<0.001$.

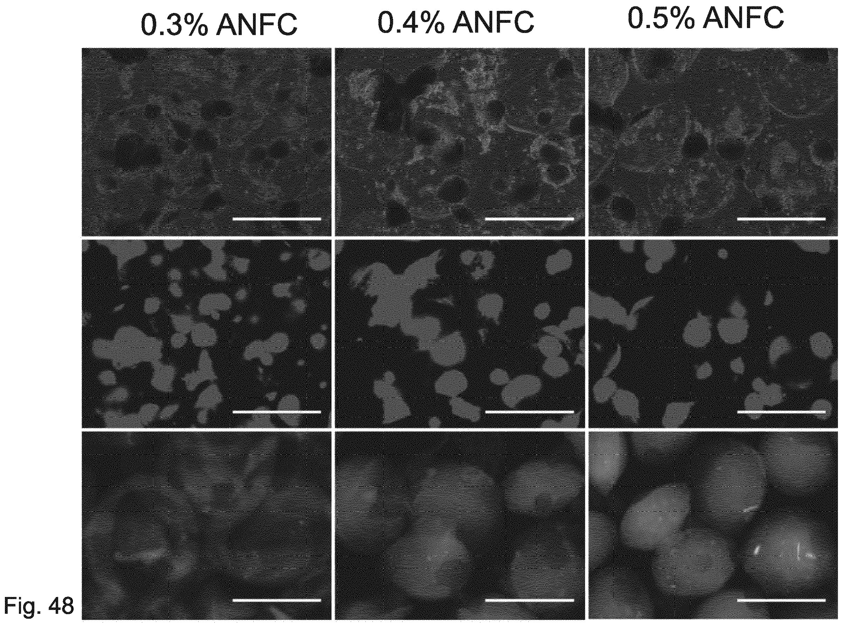

Figure 48 shows LentiX-mCherry cells after growing on 0.4% ANFC beads for 7 days. Epifluorescence and phase-contrast imaging of LentiX-mCherry cells (red) on beads made of different percentages ANFC and pre-stained with calcofluor white (blue). In the experiment shown here, $0.5 \times 10^6$ cells were seeded on 1.5 ml of beads in a 10 ml total culture volume in a T25 flask. Daily medium changes were performed on the cells from day 3 onwards. Cells were imaged after 7 days of culture. White scale bar = 1000 $\mu$m.

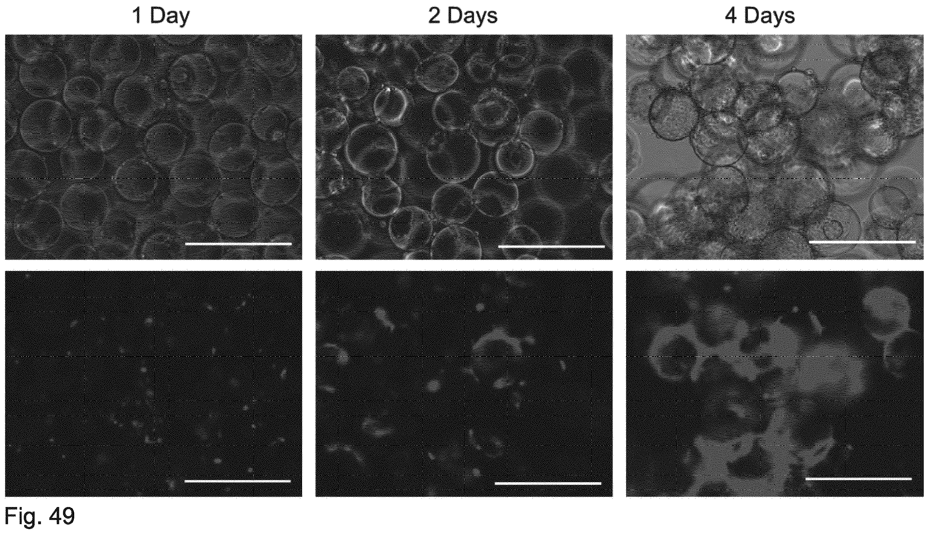

Figure 49 shows LentiX-mCherry growth on Cytodex1 beads. Phase contrast and epifluorescence imaging of LentiX-mCherry cells (red) on Cytodex 1 microcarriers. 3 g/l Cytodex was used in growth medium (GM) with a total culture volume of 4 ml. $1 \times 10^5$ cells were seeded in 2 ml GM and another 2 ml of GM was added on the second day of culture. Culture time is indicated above the images. White scale bar = 400 $\mu$m.

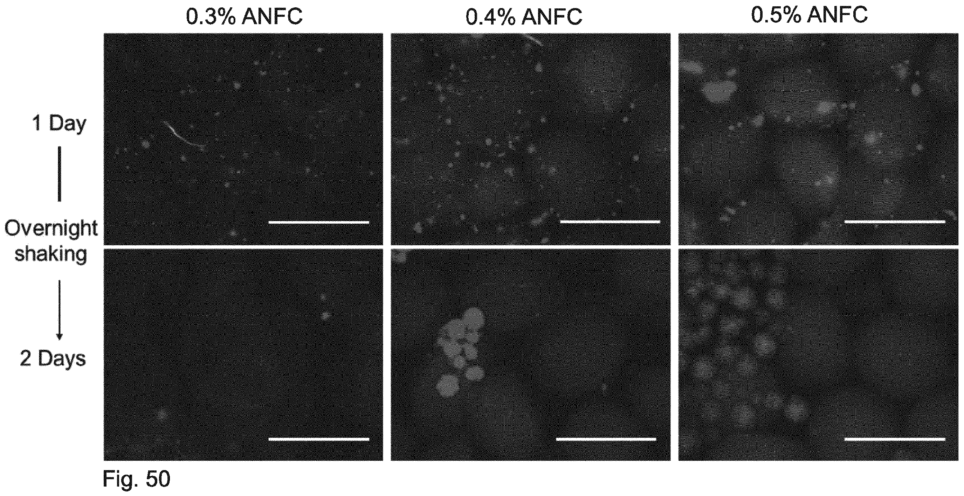

Figure 50 shows LentiX-mCherry cells after culture under agitation 1 day after seeding on ANFC beads. Epifluorescence imaging of LentiX-mCherry cells (red) on ANFC beads (blue) made with different percentages of ANFC. Culture time is indicated on the right of the images. $1 \times 10^5$ cells were seeded on 0.6 ml ANFC beads in growth medium (total volume: 4 ml), kept stationary overnight and transferred to an orbital shaker one day after seeding. White scale bar = 1000 $\mu$m.

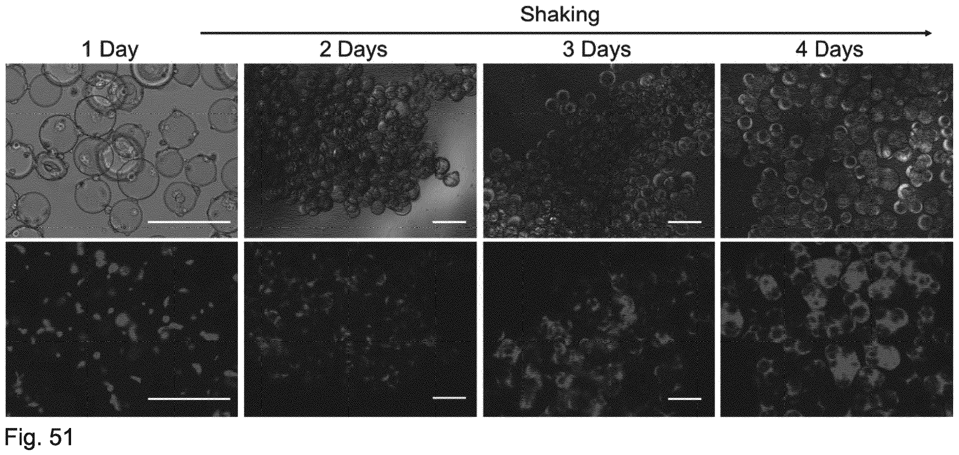

Figure 51 shows LentiX-mCherry cells after culture under agitation 1 day after seeding on Cytodex 1 beads. Phase contract epifluorescence imaging of LentiX-mCherry cells (red) grown on Cytodex 1 microcarriers. Culture time is indicated above the images. 3 g/l Cytodex was used in growth medium (GM) with a total culture volume of 4 ml. $1 \times 10^5$ cells were seeded in 2 ml GM and another 2 ml of GM was added on the second day of culture. Cells were kept stationary overnight and transferred to an orbital shaker one day after seeding. White scale bar = 400 $\mu$m. Note the difference in magnification between images taken after 1 day and images taken after 2, 3 and 4 days.

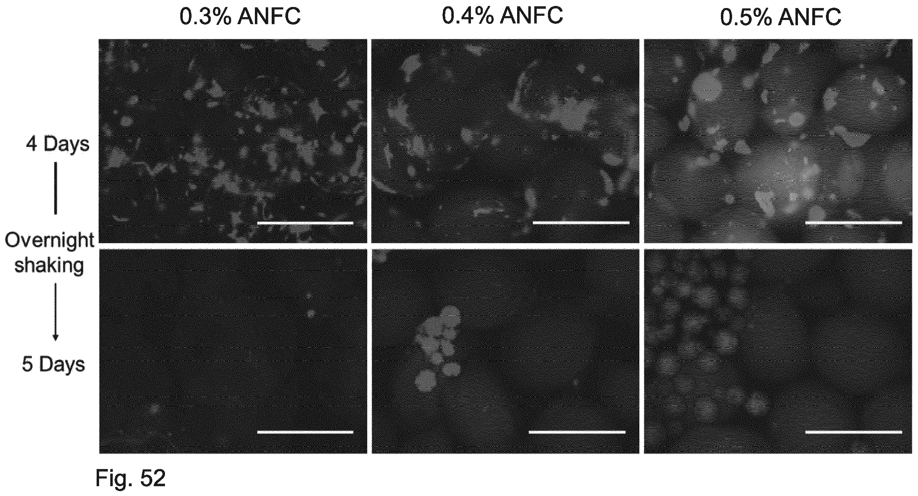

Figure 52 shows LentiX-mCherry cells after culture under agitation from 4 days after seeding on ANFC beads. Epifluorescence imaging of LentiX-mCherry cells (red) on ANFC beads (blue) made with different percentages of ANFC. Culture time is indicated on the right of the images. $1 \times 10^5$ cells were seeded on 0.6 ml of ANFC beads in growth medium (total volume: 4 ml), kept stationary for 4 days and then transferred to an orbital shaker. White scale bar = 1000 $\mu$m.

## Detailed description

In this specification, percentage values, unless specifically indicated otherwise, are based on weight (w/w, by weight, or wt%). If any numerical ranges are provided, the ranges include also the upper and lower values. The open term "comprise" also includes a closed term "consisting of" as one option. The diameters disclosed herein, unless specifically indicated otherwise, refer to the smallest diameter, and may be presented as average or number-average diameter and may be determined microscopically.

The materials and products described herein may be medical and/or scientific materials and products, such as life science materials and products, and may be used in the methods and the applications involving living cells and/or bioactive material or substances, such as described herein.

The present application provides a method for preparing microbeads comprising nanofibrillar cellulose, the method comprising

- providing a dispersion of chemically anionically modified nanofibrillar cellulose (ANFC) having a number-average diameter of 200 nm or less,
- forming the nanofibrillar cellulose into microbeads,
- obtaining microbeads comprising nanofibrillar cellulose in the range of 0.2-2% by weight.

With the present methods it is possible to obtain microbeads, which comprise chemically anionically modified nanofibrillar cellulose having a number-average diameter of 200 nm or less having a concentration in the range of 0.2-2% by weight. The microbeads may be therefore obtained by the method. The microbeads may be provided as an aqueous dispersion or suspension.

For the purposes of the present materials and applications thereof, the nanofibrillar cellulose should have adequate degree of fibrillation so that the desired properties and effects are obtained. The nanofibrillar cellulose may have a number-average diameter of the fibrils and/or fibril bundles in the range of 1-200 nm. The nanofibrillar cellulose may be further characterized with rheological properties, such as viscosity and/or yield stress.

In one embodiment the nanofibrillar cellulose, when dispersed in water, provides a zero shear viscosity in the range of 100-50000 Pa s, in the range of 300-10000 Pa·s, such as in the range of 300-8000 Pa·s, 1000-10000 Pa·s or 1000-8000 Pa·s, and a yield stress in the range of 1-50 Pa, such as in the range of 2-15 Pa, determined by rotational rheometer at a consistency of 0.5% by weight in aqueous medium at 22±1°C. Such material is fibrillated into such degree and has such properties that especially facilitates cell culturing, incubating, cell-derived product production, cell storage, encapsulating cells, and other applications discussed herein. Particularly the zero shear viscosity in the range of 300-8000 Pa·s and a yield stress in the range of 2-15 Pa are especially suitable for the present applications. In an especially advantageous embodiment the nanofibrillar cellulose comprises cellulose fibrils and/or fibril bundles having a number-average diameter in the range of 2-100 nm, such as 2-50 nm, 2-20 nm or 10-50 nm, and wherein the nanofibrillar cellulose, when dispersed in water, provides a yield stress in the range of 1-50 Pa, such as in the range of 2-15 Pa, determined by rotational rheometer at a consistency of 0.5% by weight in aqueous medium at 22±1 °C. This material is highly fibrillated, which facilitates the desired properties of the obtained products.

The rheological properties, as well as other properties, such as turbidity, as disclosed and discussed herein, are usually determined at standard conditions, including a standard consistency, for example 0.5 wt% or 0.8 wt% consistency or concentration in water, such as in pure water, or in aqueous medium. The aqueous medium may be water, such as pure or purified water, or an aqueous medium obtained by adding said water. The nanofibrillar cellulose may be therefore dispersed in water to obtain desired standard consistency for determining one or more rheological property pr other property. A homogenous dispersion of NFC is obtained. Dispersing may be carried out by using a suitable mechanical disintegration treatment which causes homogenous dispersion of the NFC into the aqueous solution without further fibrillating the cellulose, for example in case of microbeads the structure of the beads is disintegrated. In case the nanofibrillar cellulose has a lower consistency than the desired measuring consistency, it can be concentrated to obtain the desired consistency.

The nanofibrillar cellulose may be the only matrix material in the microbeads, such as the only polymeric material and/or the only cellulosic material. However, it is also possible to include other polymeric materials in addition to NFC, such as nanocrystalline cellulose, hyaluronan, hyaluronic acid and its derivates, peptidebased materials, proteins, other polysaccharides such as alginate or polyethylene glycol. Compositions forming a semi-interpenetrating network (semi-IPN) may be obtained, where nanofibrillar cellulose provides structural stability. The content of the other polymeric materials in the total composition as dry weight may be in the range of 20-80% (w/w), such as 40-60% (w/w), or 10-30% (w/w) or 10-20% (w/w).

The anionically modified nanofibrillar cellulose is generally provided as an aqueous dispersion, which may be a dispersion formed in water only, such as pure water, or purified water, or other aqueous solution, which may or may not contain other ingredients, such as salt, buffering agent(s), medium or the like, such as any aqueous solutions or mediums discussed herein. For example, PBS solution or essential medium, such as DMEM, may be used for dispersing the ANFC, depending on the application. The anionically modified nanofibrillar cellulose may be provided in the form obtained directly from the preparation process, such as any disintegrating process disclosed herein, so it may be dispersed in water. However, in later steps the material may be dispersed to other aqueous solutions as well.

The dispersion of chemically anionically modified nanofibrillar cellulose may be formed into microbeads with any suitable method, which preferably results in the formation of microbeads having desired properties, such as a desired diameter, size distribution, degree of uniformity, shape, robustness, coherence and/or percentage. The ANFC as non-toxic material containing no animal-based material, is especially suitable for the uses discussed herein. Such microbeads shall be suitable for desired uses, such as for use as cell culture material and/or cell storage material.

When trying to find suitable method for preparing microbeads with desired properties, there were several challenges in finding successful method. For example, there were problems in achieving suitable uniformity of beads, as well as shape, size distribution, diameter and the like properties. Also obtaining separate beads which are not fused together or coalesced, and which maintained their shapes was difficult. Further properties such as desired percentage, inner structure, robustness and other features, which have impact on the integrity, stability and cell permeability and permeability to substances such as nutrients and cell-derived products, were also challenging to obtain.

It was also found out that certain properties and the combination of the properties of the obtained beads have an impact to properties such as ability of the material to encapsulate cells and to store the cells in a stable form, such as in a paused state and/or in an undifferentiated form, for example in the case of stem cells.

Two suitable methods for preparing acceptable microbeads especially from anionic NFC were found, namely membrane emulsification and electro spraying. With these methods it was possible to obtain microbeads having desired integrity, uniformity, stability, size, size distribution, robustness, and other properties discussed herein. For example, it was possible to obtain separate microbeads small enough. The obtained microbeads may have an average diameter in the range of 100-1200 $\mu$m. Microbeads at this diameter range could be applied in the uses discussed herein. A specific average diameter may be at a relatively narrow area in this range and can be affected by the selection of preparation method and by controlling the method setup and/or process conditions.

Average particle diameter ($D_{av}$) may be detected from a sample microscopically. A specific analysis software may be used, such as Matlab, or additionally ImageJ, which were used in the Examples. The average particle diameter of the microbeads may be determined as a volume median particle size or diameter. Particle sizes or diameters of non-spherical beads can be determined or presented as equivalent spherical diameter (ESD). The equivalent spherical diameter of an irregularly shaped object is the diameter of a sphere of equivalent volume.

A coefficient of variation (CV) of the obtained microbeads may be in the range of 15-30%, such as 20-30%, for example in the range of 15-27%, 15-25%, 20-27% or 22-27%. The coefficient of variation (CV), also known as relative standard deviation (RSD), is a standardized measure of dispersion of a probability distribution or frequency distribution. It is often expressed as a percentage and is defined as the ratio of the standard deviation $\sigma$ to the mean $\mu$ (or its absolute value, $\mu$). A smaller percentage value means narrower size distribution, i.e. better uniformity. The coefficient of variation may be presented also as an average coefficient of variation, for example over a time period, such as 60 for minutes.

The microbeads are suitable entities for culturing, storing or carrying cells. Methods for applying cells with the microbeads are described herein. The cells may be combined with the final microbeads, or the cells may be combined with nanofibrillar cellulose during the preparation of the microbeads. In one example the cells are mixed with the nanofibrillar cellulose dispersion or emulsion before forming the dispersion or emulsion into microbeads. It was found out that cells can tolerate the formation conditions in many cases.

More particularly it was possible to obtain microbeads containing or having a concentration of nanofibrillar cellulose (NFC) in the range of 0.2-2.0% by weight. The concentration could be controlled, and a desired concentration could be obtained for a desired use. A suitable preparation method may be selected to obtain microbeads with desired percentage of NFC, for example in the range of 0.2-1.0% by weight, such as in the range of 0.2-0.8% by weight, 0.2-0.7% by weight, 0.25-0.7% by weight or 0.25-0.6% by weight.

First of all, it was found out that it was very difficult, even impossible, to formulate chemically unmodified (native) nanofibrillar cellulose (NFC) into microbeads having the desired properties. It may be possible to obtain beads of certain type from native NFC, but such beads may not be suitable for the specific uses, such as for culturing and/or storing the cells in the beads, especially as encapsulated and/or at certain specific conditions and/or when using specific cells, such as stem cells.

It was found out that anionic NFC (ANFC) was suitable starting material for forming microbeads with desired properties. It was found out that ANFC has a good ability to be ionically crosslinked. The microbeads formed from ANFC showed enhanced robustness and allowed further processing. Anionic NFC or anionically modified NFC as used herein refers to chemically anionically modified NFC, such as oxidized NFC, for example TEMPO oxidized NFC, which usually has a high fibrillation degree and therefore a high aspect ratio and especially a low fibril diameter. The number-average fibril diameter of such chemically anionically modified nanofibrillar cellulose may be 100 nm or less, or lower, such as 50 nm or less, or even 20 nm or less. When ANFC was used, it was possible to obtain microbeads having the desired properties which could be used for the specific purposes discussed herein, but even in this case many challenges had to be overcome. Merely using ANFC without specific process steps resulted in some cases even in worse problems during the preparation process than using native NFC as starting material. For example, the high viscosity and shear thinning properties of ANFC caused by the low fibril diameter and high aspect ratio could raise problems in handling and processing, such as clumping or blocking problems. As forming microbeads may require pushing the NFC hydrogel through very small tubes, apertures and/or orifices, the extremely high viscosity of ANFC does not make it an obvious choice for starting material, and such problems were faced in the experiments.

In one embodiment the chemically anionically modified nanofibrillar cellulose is oxidized nanofibrillar cellulose, such as TEMPO oxidized nanofibrillar cellulose. It may be desired to use wood cellulose as starting material, as wood cellulose can be efficiently modified according to needs, for example by the chemical modifications and by mechanical treatments.

The method comprises providing a dispersion of chemically anionically modified nanofibrillar cellulose. This dispersion may be obtained directly from a disintegration process of chemically anionically modified cellulose, or the ANFC may be provided as prepared and packed, i.e., a ready-made, product. The concentration of the dispersion may be initially any suitable concentration, for example a concentration in the range of 0.2-5%, which in many cases is the concentration the material is obtained from the fibrillating process. The dispersion may be diluted and/or concentrated during the process, so the concentration may be therefore adjusted and controlled. In many cases similar concentrations are used for the starting material as may be the concentration of final products, for example in the range of 0.2-2% by weight in the range of 0.2-1% by weight, such as in the range of 0.2-0.8% by weight, 0.3-0.6% by weight or 0.2-0.5% by weight, for example about 0.5% by weight, which was used in many successful experiments.

Before forming the microbeads, the dispersion of chemically anionically modified nanofibrillar cellulose may be homogenized, especially in a diluent, to obtain homogenous distribution of material and remove areas of discontinuity from the gel, which has an impact to further method steps. This homogenization may be a non-fibrillating homogenization step, which can be achieved by using a suitable homogenizing device, suitable settings of the device, suitable homogenizing energy and/or suitable processing time. Therefore, in one embodiment the method comprises homogenizing the aqueous dispersion of chemically anionically modified nanofibrillar cellulose with a non-fibrillating homogenization. This step is carried out before forming the ANFC into microbeads. A skilled person is able to select suitable device and suitable process conditions to obtain a non-fibrillating homogenization or disintegration treatment, as well as fibrillating treatment in cases it is required. The improved continuity and homogeneity of the obtained hydrogel may be detected from the material for example microscopically and/or by determining its rheological properties and can be recognized for example by comparing to similar material which is not homogenized with similar non-fibrillating homogenization.

Homogenisation of ANFC was found advantageous, in some cases even essential, for producing microbeads of ANFC that have an even cellulose distribution and spherical shape. Especially this was the case when membrane emulsification was used for forming the microbeads, but also for electro spraying. The homogenization step facilitated processing the highly viscous anionic NFC in the microbead-forming processes. The homogenization may be carried out in a diluent, which may be a suitable aqueous solution, such as a buffer solution or medium, for example PBS or DMEM. The homogenization may be carried out briefly prior to forming the ANFC into microbeads.

One or more emulsifying agent(s) may be added to further facilitate the process, such as a non-ionic emulsifying agent, for example ethyl PEG/PPG-1/10 dimethicone type of emulsifying agent. Especially an emulsifying agent may be useful if the dispersion contains oil. An emulsion is obtained, which may be further processed in further process steps as disclosed herein. The emulsifying agent may be added to the homogenized dispersion.

In one embodiment the forming the dispersion into an emulsion comprises adding one or more oil(s) and/or one or more emulsifying agent(s), preferably to obtain the emulsion.

Also, one or more surfactant(s) may be added, either to the emulsion or to the formed microbeads, to control the particle variety and/or to facilitate removing or washing the oil. The surfactant may be non-ionic surfactant. Examples of surfactants include silicone-based non-ionic surfactants, sorbitan oleate (polysorbate), such as polysorbate 20 (Tween 20) or polysorbate 80 (Span 80), etyl PEG/PPG-1/10 dimethicone (ABIL EM 90) and polyglycerol polyricinoleate (PGPR).

The homogenization and/or adding an emulsifying agent (forming an emulsion) may be carried out before any further steps, such as forming the ANFC into microbeads and/or crosslinking.

The microbeads may be further coated with a polymer, such as poly-d-lysine, agarose, collagen or hyaluronic acid, or derivatives thereof, to enhance cell adhesion or other properties. The coating may help immobilizing the cells, enhancing flow of gas and substances, and also otherwise enhance the processes. The polymer may be provided as a solution. The microbeads, or prebeads, may be treated with the polymer solution to coat the beads.

*Crosslinking*

It was found out that the properties of the beads or already the ANFC dispersion, as well as the success of the processes, could be enhanced by crosslinking the material ionically by using multivalent cations, such as calcium cations or with other suitable cations. Ionical crosslinking does not result in formation of covalent bonding, which could have harmful effect, such as forming permanent crosslinking in the NFC itself. Also, if cells are present, either before or after the crosslinking, covalent crosslinking or the reagents used therein could harm the cells and/or bind the cells to the NFC matrix, which is not desired. When the ANFC was crosslinked ionically using multivalent cations, such as divalent cations, for example calcium chloride, the obtained crosslinking can be undone or adjusted, and the cells can tolerate the crosslinking conditions. However, it was also found out that even though the ionical crosslinking appears to be a simple method, there were still challenges in obtaining desired crosslinking of ANFC and obtaining microbeads with desired properties. For example, to obtain a desired robustness of the microbeads, it was necessary to find suitable crosslinking conditions, such as mixing conditions and subsequent washing and filtering conditions, as well as resuspending and centrifuging. Any possible still remaining debris can be removed by further washing with a surfactant. It was noticed that the crosslinked particles tolerated harsh conditions, such as centrifuging and autoclaving.

In one embodiment the method comprises treating the chemically anionically modified nanofibrillar cellulose, such as in the form or dispersion, emulsion and/or microbeads, with multivalent cations, such as multivalent metal and/or earth alkali metal cations, for example selected from one or more of cations of calcium, magnesium, barium, zinc, aluminum, gold, platinum and titanium to ionically crosslink the nanofibrillar cellulose, preferably to obtain microbeads comprising ionically crosslinked nanofibrillar cellulose. However, as gold may provide antimicrobial properties, and platinum and titanium may provide catalytic properties, such as photocatalytic properties in the case of titanium, it may not be desired to use these cations when in it desired to obtain materials to be used to maintain cells. In the cell culture and storage applications metal cations and/or earth alkali metal cations are useful, such as cations of calcium, magnesium and/or zinc. The cations are preferably divalent cations. For example, barium and calcium may be useful in biomedical application, and especially barium may be used in labelling. The amount of the multivalent cations may be in the range of 0.1-3% (w/w), for example 0.1-2% (w/w) calculated from the dry content of the hydrogel. The cations may be provided as chloride salts, such as magnesium chloride ($MgCl_2$) and/or calcium chloride ($CaCl_2$). Calcium chloride was found preferable in preliminary tests. For example, microbeads crosslinked with calcium were found to further enhance the adherence of cells to the microbeads, which adherence is a useful feature in several applications, such as in cell culturing applications, microcarrier applications, and cell storage applications. It is not necessary to treat the surfaces of the beads with further methods or substances to facilitate cell adherence

In one embodiment the nanofibrillar cellulose in the microbeads is ionically crosslinked nanofibrillar cellulose.

The ionical crosslinking may be obtained by

- providing anionically modified nanofibrillar cellulose,
- providing multivalent cations, such as one or more types of multivalent cations,
- contacting the anionically modified nanofibrillar cellulose with the multivalent cations, and
- allowing reacting for a period of time to obtain crosslinked nanofibrillar cellulose product. The period of time may be a period required at least for the desired crosslinking to take place, which may be fully or partial crosslinking, or a longer period, and in may be minutes or hours, such as at least one minute, for example at least in the range of 1-240 minutes, or 30-240 minutes, such as 1-3 hours. However even longer incubation times could be applied, such as about 12, 24, 48 or 72 hours. The ANFC or microbeads formed from the ANFC may be soaked and/or mixed or agitated in an aqueous solution of multivalent cations.

In one example the ANFC in dispersion or in emulsion form is contacted with the multivalent cations. This may be carried out with mixing, such as by stirring, for example for 10-30 minutes, such as 10-20 minutes. A concentration of multivalent cations in the range of 5-15% by weight, such as 8-12% by weight, in the dispersion or the emulsion may be used. After the crosslinking the dispersion or the emulsion may be formed into microbeads. This process was found to be especially successful in membrane emulsification method, and resulted in microbeads retaining their shape, being robust and solvent resistant. Such microbeads were also especially suitable for cell culture and cell storage applications.

In one example the ANFC in microbead form is contacted with the multivalent cations. This option can be used for example in electrospraying method. A similar procedure may be used as in the previous. The microbeads (or preforms thereof) may be first filtered, for example by using any suitable filter or filtering device, and/or the microbeads may be filtered afterwards. This may depend on the used method.

The method may further comprise washing the fibrillar cellulose material or microbeads after the period of time, i.e., after crosslinking, such as washing with aqueous solution.

In one example the ANFC dispersion, emulsion or microbeads is/are diluted with buffer solution, such as PBS, mixed with multivalent cations, filtered and/or centrifuged, and resuspended into buffer solution. This may be repeated, for example once or twice.

*Membrane emulsification*

In membrane emulsification a dispersed phase is forced through pores of a microporous membrane directly into a continuous phase. Emulsified droplets are formed and detached at the end of the pores with a drop-by-drop mechanism. For applying membrane emulsification principle to nanofibrillar cellulose several challenges needed to be solved. There were problems in obtaining microbeads with desired properties, such as robustness, shape, uniformity, integrity, and durability. Further it was challenging to pass the viscous ANFC through the micropores of the membranes.

In one embodiment the forming the nanofibrillar cellulose into microbeads comprises membrane emulsification. The membrane emulsification preferably comprises forming the dispersion into an emulsion and passing the emulsion through a porous membrane to form microbeads comprising chemically anionically modified nanofibrillar cellulose.

The ANFC or the formed microbeads may be provided or mixed in buffer solution, such as phosphate buffered saline (PBS), or cell culture medium, such as DMEM. The type of solution was found to have an impact to the properties of the formed microbeads, such as to robustness or coalescence. However, the type of solution may also have an impact to the crosslinkability of the ANFC, which crosslinkability could be further facilitated with a non-fibrillating homogenisation treatment.

The membrane emulsification may be carried out with a setup comprising one or more suitable membranes and means for providing the emulsion through the membrane(s), such as means for providing pressure or injecting means. A continuous phase containing the microbeads may be obtained from the membrane. Constant stirring of the continuous phase obtained from the membrane may be required to exert shear at the surface of the membrane, causing droplets to detach from the membrane and be suspended in the continuous phase. The setup may include further parts such as one or more injectors, pumps and/or syringes, for example a syringe pump, vials or other containers for the dispersion and/or emulsion, stirrer(s) and/or other mixer(s), for example connected to a blade or the like rotor, and/or containers for receiving the formed microbeads. For example, a dispersion cell such as one disclosed in Figure 24 may be applied. The method and/or setup may be modified with one or more features disclosed in the examples.

Oil may be used to facilitate the formation of the microbeads. The method may comprise providing oil, such as to the ANFC dispersion, preferably to obtain an emulsion, wherein the forming the nanofibrillar cellulose into microbeads is carried out in the presence of the oil. The oil may be for example kerosene or propylene glycol dicaprylocaprate. Kerosene was found to be preferred in certain respects, such as for producing uniform emulsions, to prevent coalescence and to increase particle size.

Especially when oil is used, it may be necessary to add one or more emulsifying agent(s), as discussed in previous. An emulsion is obtained, which is then further processed. The emulsion may be crosslinked with the methods disclosed herein.

The emulsion comprising ANFC and oil is passed through a membrane with a suitable pore size to obtain microbeads. The pore size may be in the range of 10-50 μm, such as 15-20 μm. The membrane may have a pore spacing in the range of 100-250 μm, such as 150-250 μm. A suitable membrane type may be selected, such as a ring membrane or a full membrane.

It was found out that the mixing or rotation speed in the mixing of the continuous phase had an impact to the average microbead size and size distribution. The continuous phase may be mixed or stirred with a mixing or rotation speed in the range of 1000-1500 rpm, such as 1300-1400 rpm, which was found to product most uniform particle size distribution. The injection rate of the emulsion to the membrane may be in the range of 0.5-10 ml/min, such as 0.5-2 ml/min, for example about 1 ml /min. The injection of the emulsion may be obtained by using a suitable injecting means, such as a syringe pump or other pumping device or injector, and controlling the injecting means to obtain a desired injection rate.

When oil, such as kerosene, is used, it may be necessary to include a surfactant to prevent coalescence of the microbeads. Nonionic surfactants were successfully used, such as silicone-based water/oil emulsifiers, for example ABIL EM90, and polysorbates, for example polysorbate 80. Such surfactants may also act as emulsifying agents in the present use.

It may be necessary to remove the oil after the beads have been formed, for example by washing and filtering. In this way any excess continuous phase, including the oil, can be removed.

In one embodiment the method comprises filtering the obtained microbeads, especially to remove excess continuous phase, preferably comprising washing with a solution comprising surfactant, such as polysorbate 20 or 80, to remove oily residues. The method may comprise one or more resuspending steps of the obtained microbeads, for example in combination with a centrifuging or other concentrating step, for example two, three or four cycles of concentrating and resuspending.

With membrane emulsification it was possible to obtain microbeads with relatively small diameter, such as below 250 μm. It was also possible to obtain a relatively narrow size distribution. In embodiment the obtained microbeads have an average diameter in the range of 100-230 μm, such as in the range of 100-200 μm, in the range of 100-190 μm for example in the range of 100-170 μm. Microbeads with such a low diameter, for example 200 μm or less, may be desired in certain end uses, such as in microcarrier use.

The membrane emulsification process may be monitored and facilitated by staining the material with a suitable dye. Calcofluor white was used successfully in the experiments. The dye may be visualized with UV light, where applicable.

*Electro spraying*

In one embodiment the forming the nanofibrillar cellulose into microbeads comprises electro spraying.

Electrospraying is a liquid atomization-based technique that can be used to produce and formulate micro or nanoparticular cargo carriers for various applications. For the purposes of polymeric particle production, a common setup of electrospraying may comprise a high-voltage power supply, a plastic/glass syringe capped by a metallic capillary, such as a 16- to 26-gauge needle, to hold a polymer solution, a syringe pump to control the flow of the solutions, and a grounded collector. When a high electric field is applied at the needle, a charged liquid jet will break up into droplets, which form small particles with generally narrow size distribution on the collector. The process can be performed at ambient conditions (temperature and pressure), which is beneficial for sensitive biomolecules and even living cells; secondly, due to the possible absence of an external medium that allows the dissolution or migration of water-soluble cargos, the encapsulation efficiency using electrospraying can be maximized.

Even though obtaining desired microbeads by electro spraying was challenging, it was possible to finally find suitable conditions for producing ANFC microbeads exhibiting properties discussed herein. For example, it was possible to obtain a relatively narrow bead size distribution at a desired range.

It was found out that the formation of desired microbeads by electro spraying could be facilitated by using a voltage just below the critical voltage for jet formation, which led to formation of droplets rather than spray at the emitter. When the droplets were stirred with crosslinking multivalent cations, stable and relatively large beads of about 1000 μm could be obtained. When the voltage was raised above the threshold required to produce a jet, a spray was obtained leading to formation of smaller beads.

The method for forming the nanofibrillar cellulose into microbeads by electrospraying may comprise

- providing the dispersion of chemically anionically modified nanofibrillar cellulose.
- providing a flow of the dispersion, such as by pressure, through a needle or the like capillary,
- providing electricity or electric field to the needle or capillary, preferably metallic, to obtain a charged liquid jet of the dispersion directed to a grounded collector or any other applicable receiving part or container. The method may comprise adjusting the voltage and/or current to obtain desired spray or output leading to formation of desired beads. The method may comprise selecting the needle or capillary having a suitable orifice, preferably a diameter of the orifice, for outputting such jet which results in formation of desired microbeads.

A device setup containing suitable parts may be provided, such as one or more (metallic) needles or the like capillaries, preferably with the suitable orifice, one or more pumps for providing the flow, a syringe or other vial for the dispersion connected to the needle or capillary, a source of electric current connected to the setup, preferably an adjustable source of electric current, a grounded collector, any required tubes, wiring, valves, switches, control units, and/or the like parts. The method and/or setup may be modified with one or more features disclosed in the examples.

The droplets, which may be called prebeads, directly obtained by electrospraying could be crosslinked afterwards, i.e., after formation of the droplets or prebeads. The crosslinking may be carried out with the methods disclosed herein. For example, the ANFC dispersion could be electrosprayed into a crosslinking solution and mixed, such as stirred, to obtain the final microbeads, which may be washed, for example with buffer or medium as discussed in previous. The method may comprise forming prebeads with electro spraying and crosslinking the formed prebeads to obtain final microbeads.

The obtained microbeads may have an average diameter in the range of 100-1200 $\mu$m, such as in the range of 200-1000 $\mu$m, such as in the range of 200-500 $\mu$m. Larger microbeads having an average diameter above 200 $\mu$m, such as 500 $\mu$m or more, may be desired in applications, such as cell encapsulation applications, such as in cell culture, production of cell-derived products including viruses, and/or cell storage. Such microbeads could be efficiently produced by electro spraying methods.

The concentration of nanofibrillar cellulose may be in the range of 0.2-1% by weight, such as 0.2-0.8% by weight. In the present test best results were obtained with a concentration in the range of 0.3-0.5% by weight.

It was found out that the microbeads obtained with the methods discussed herein could be sterilized, for example they tolerated sterilization by autoclaving without losing their shape or other properties. Also irradiating or the like sterilizing methods can be applied.

In one embodiment the method comprises sterilizing the obtained microbeads, such as by autoclaving, for example for 15 minutes at 121°C. This applies to microbeads obtained by membrane emulsification and electro spraying, and especially to crosslinked microbeads.

It is possible to analyze microbeads to detect or recognize the preparation method used for producing the microbeads, as well as to detect or recognize used materials. The microbeads can be studied microscopically, such as by using microscopical techniques and devices disclosed herein. The microbeads may be stained or dyed to facilitate the analysis. Direct and/or indirect properties can be detected and/or analysed from the microbeads, such as size (diameter), size distribution, shape, composition, percentage, rheological properties, grammage, spectroscopic properties, crosslinking and degree and/or depth thereof, robustness, and/or the like properties, such as discusses herein, which properties can be detected and/or analyzed by using any suitable methods and devices.

*Nanofibrillar cellulose*

The starting material for forming the hydrogel may be nanofibrillar cellulose, also called as nanocellulose, which refers to isolated cellulose fibrils and/or fibril bundles derived from cellulose raw material. Nanofibrillar cellulose is based on a natural polymer that is abundant in nature. Nanofibrillar cellulose has a capability of forming viscous hydrogel in water. Nanofibrillar cellulose production techniques may be based on disintegrating fibrous raw material, such as grinding of aqueous dispersion of pulp fibers to obtain nanofibrillated cellulose. After the grinding or homogenization process, the obtained nanofibrillar cellulose material is a dilute viscoelastic hydrogel.

The obtained material usually exists at a relatively low concentration homogeneously distributed in water due to the disintegration conditions. The starting material may be an aqueous gel at a concentration of 0.2-10% (w/w), for example 0.2-5% (w/w). The nanofibrillar cellulose may be obtained directly from the disintegration of fibrous raw material, such as cellulose fibers. Examples of commercially available nanofibrillar cellulose hydrogels include GrowDex® variants by UPM.

Because of its nanoscale structure nanofibrillar cellulose has unique properties which enable functionalities which cannot be provided by conventional non-nanofibrillar cellulose or for example synthetic fibers or fibrils. It is possible to prepare materials and products which exhibit different properties than conventional products or products using conventional cellulosic materials or other polymeric materials. However, because of the nanoscale structure nanofibrillar cellulose is also a challenging material. For example, dewatering or handling of nanofibrillar cellulose may be difficult.

The nanofibrillar cellulose may be prepared from cellulose raw material of plant origin, or it may also be derived from certain bacterial fermentation processes. The nanofibrillar cellulose is preferably made of plant material. Nanofibrillar cellulose is preferably obtained from plants by mechanical disintegration of cellulose fibers. The raw material may be based on any plant material that contains cellulose. In one example the fibrils are obtained from non-parenchymal plant material. In such case the fibrils may be obtained from secondary cell walls. One abundant source of such cellulose fibrils is wood fibres. The nanofibrillar cellulose may be manufactured by homogenizing wood-derived fibrous raw material, which may be chemical pulp. Cellulose fibers can be disintegrated to produce fibrils which may have an average diameter of only some nanometers and gives a dispersion of fibrils in water. The fibrils originating from secondary cell walls are essentially crystalline with degree of crystallinity of at least 55%. Such fibrils may have different properties than fibrils originated from primary cell walls. For example, the dewatering of fibrils originating from secondary cell walls may be more challenging. In general, in the cellulose sources from primary cell walls, such as sugar beet, potato tuber and banana rachis, the microfibrils are easier to liberate from the fibre matrix than fibrils from wood, and the disintegration requires less energy. However, these materials are still somewhat heterogeneous and mainly consist of large fibril bundles.

Non-wood material may be from agricultural residues, grasses or other plant substances such as straw, leaves, bark, seeds, hulls, flowers, vegetables or fruits from cotton, corn, wheat, oat, rye, barley, rice, flax, hemp, manila hemp, sisal hemp, jute, ramie, kenaf, bagasse, bamboo or reed. The cellulose raw material could be also derived from the cellulose-producing micro-organism. The microorganisms can be of the genus Acetobacter, Agrobacterium, Rhizobium, Pseudomonas or Alcaligenes, preferably of the genus Acetobacter and more preferably of the species Acetobacter xylinumor or Acetobacter pasteurianus.

It was found out that nanofibrillar cellulose obtained from plant cellulose, especially wood cellulose, is preferable for life science, medical and/or scientific products described herein. Wood cellulose is available in large amounts, and the preparation methods developed for wood cellulose enable further producing nanofibrillar materials suitable for such products. The nanofibrillar cellulose obtained by fibrillating plant fibers, especially wood fibers, differs structurally from nanofibrillar cellulose obtained from microbes, and it has different properties. For example, compared to bacterial cellulose, nanofibrillated wood cellulose is homogenous and more porous and loose material, which is advantageous in applications involving living cells. Bacterial cellulose is usually used as such without similar fibrillation as in plant cellulose, so the material is different also in this respect. Bacterial cellulose is dense material which easily forms small spheroids and therefore the structure of the material is discontinuous, and it is not desired to use such material in the applications relating to living cells, especially when homogeneity of the material is required. Wood cellulose on the other hand may be used for preparing a variety of different nanofibrillar products, and the properties of the final nanofibrillar cellulose can be affected by the choice of process conditions, used methods, modifications and other conditions and method steps.

Nanofibrillar cellulose derived from plant material requires disintegrating cellulose fibers into fibrils and/or fibril bundles, which enables controlling the fibrillation process and the properties of the obtained fibrillated cellulose material. It is possible to modify the cellulose before the disintegration process and control the fibrillation type and efficiency for example by selecting a suitable device, suitable process conditions and time, and other related parameters, to obtain nanofibrillar cellulose with desired properties such as fibrillation degree, aspect ratio, modification type and continuity. This is not possible with bacterial nanocellulose, which is usually present as already fibrillar form. Plant cellulose is also preferred for methods, wherein cells and/or cell-derived products are cultured, maintained and/or prepared and it is desired to have a controlled production without any disturbing or contaminating substances. When using plant cellulose there would be no risk of presence of any substances of bacterial origin.

Wood may be from softwood tree such as spruce, pine, fir, larch, douglas-fir or hemlock, or from hardwood tree such as birch, aspen, poplar, alder, eucalyptus, oak, beech or acacia, or from a mixture of softwoods and hardwoods. In one example the nanofibrillar cellulose is obtained from wood pulp. The nanofibrillar cellulose may be obtained from hardwood pulp. In one example the hardwood is birch. The nanofibrillar cellulose may be obtained from softwood pulp. In one example said wood pulp is chemical pulp. Chemical pulp may be desired for the products disclosed herein. Chemical pulp is pure material and may be used in a wide variety of applications. For example, chemical pulp lack the pitch and resin acids present in mechanical pulp, and it is more sterile or easily sterilisable. Further, chemical pulp is more flexible and provides advantageous properties for example in medical and scientific materials. For example, very homogenous nanofibrillar cellulose materials may be prepared without excess processing or need for specific equipment or laborious process steps.

Nanofibrillar cellulose, including the cellulose fibrils and/or fibril bundles, is characterized by a high aspect ratio (length/diameter). The average length of nanofibrillar cellulose (the median length of particles such as fibrils and/or fibril bundles) may exceed 1 $\mu$m, and in most cases it is 50 $\mu$m or less. If the elementary fibrils are not completely separated from each other, the entangled fibrils, such as fibril bundles, may have an average total length for example in the range of 1-100 $\mu$m, 1-50 $\mu$m, or 1-20 $\mu$m. This applies especially for native grades of fibrils which are not shortened or digested, for example chemically, enzymatically or mechanically. However, if the nanofibrillar material is highly fibrillated, the elementary fibrils may be completely or almost completely separated and the average fibril length is shorter, such as in the range of 1-10 $\mu$m or 1-5 $\mu$m. Strongly derivatized nanofibrillar cellulose may have a shorter average fibril length, such as in the range of 0.3-50 $\mu$m, such as 0.3-20 $\mu$m, for example 0.5-10 $\mu$m or 1-10 $\mu$m. Especially shortened fibrils, such as enzymatically or chemically digested fibrils, or mechanically treated material, may have an average fibril length of less than 1 $\mu$m, such as 0.1-1 $\mu$m, 0.2-0.8 $\mu$m or 0.4-0.6 $\mu$m. The fibril length and/or diameter may be estimated microscopically, for example using CRYO-TEM, SEM or AFM images. A suitable imaging software may be used. If high aspect ratio is desired, it is not desired to shorten the fibril length so it may not be desired to use chemically and/or enzymatically digested cellulose, or such highly mechanically fibrillated material that the fibrils are already shortened. A low aspect ratio usually can be detected as decreased viscosity of an NFC dispersion.

The average lengths and/or diameters disclosed herein may be number-average lengths and/or diameters, in regard of the NFC but also in regards of other applicable entities, such as microbeads, particles, cells, cell-derived products, aggregates and the like discussed herein. However, for spherical or substantially spherical entities or particles a volume median particle size or diameter may be used. These dimensions may be determined microscopically, as discussed herein. A specific software may be used. The term "fibrils" also includes fibril bundles, where applicable. The mechanically disintegrated cellulosic material may contain at least a small amount of cellulose which is not fully separated into elemental fibrils but is still in the form of fibril bundles.

The average diameter (width) of nanofibrillar cellulose is less than 1 $\mu$m, or 500 nm or less, such as in the range of 1-500 nm, but preferably 200 nm or less, even 100 nm or less or 50 nm or less, such as in the range of 1-200 nm, 2-200 nm, 2-100 nm, or 1-50 nm, even 1-20 for highly fibrillated material. The diameters disclosed herein refer to fibrils and/or fibril bundles. The smallest fibrils are in the scale of elementary fibrils, the average diameter being typically in the range of 4-12 nm. The dimensions and size distribution of the fibrils depend *inter alia* on chemical modification and the refining method and efficiency. In case of highly refined native nanofibrillar cellulose, the average fibril and/or fibril bundle diameter may be in the range of 2-200 nm or 2-100 nm, for example in the range of 10-50 nm. Nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose obtained from plants, especially wood, may also contain small amounts of other plant components, especially wood components, such as hemicellulose or lignin. The amount is dependent on the plant source.

In general cellulose nanomaterials may be divided into categories according to TAPPI W13021, which provides standard terms for cellulose nanomaterials. Not all of these materials are nanofibrillar cellulose. Two main categories are "Nano objects" and "Nano structured materials". Nanofibrillar materials include "Cellulose microcrystals" (sometimes called as CMC) having a diameter of 10-12 $\mu$m and length:diameter ratio (L/D) <2, and "Cellulose microfibrils" having a diameter of 10-100 nm and a length of 0.5-50 $\mu$m. Nano objects include "Cellulose nanofibers", which can be divided into "Cellulose nanocrystals" (CNC) having a diameter of 3-10 nm and L/D >5, and "Cellulose nanofibrils" (CNF or NFC), having a diameter of 5-30 nm and L/D >50.

Different grades of nanofibrillar cellulose may be categorized based on three main properties: (i) size distribution, length and/or diameter (ii) chemical composition, and (iii) rheological properties. To fully describe a grade, two or more properties may be used in parallel. Examples of different grades include native (chemically and/or enzymatically unmodified) NFC, oxidized NFC (high viscosity), oxidized NFC (low viscosity), carboxymethylated NFC and cationized NFC. Within these main grades, also sub-grades exist, for example: extremely well fibrillated vs. moderately fibrillated, high degree of substitution vs. low degree of substitution, low viscosity vs. high viscosity etc. The fibrillation technique and the chemical premodification have an influence on the fibril size distribution. Typically, non-ionic grades have wider average fibril and/or fibril bundle diameter (for example in the range of 10-100 nm, or 10-50 nm) while the chemically modified grades are a lot thinner (for example in the range of 2-20 nm). Distribution is also narrower for the modified grades. Certain modifications, especially TEMPO-oxidation, yield shorter fibrils.

Depending on the raw material source, e.g., hardwood vs. softwood pulp, different polysaccharide composition exists in the final nanofibrillar cellulose product. The non-ionic grades may be prepared for example from bleached birch pulp, which yields high xylene content (25% by weight). Modified grades may be prepared either from hardwood or softwood pulps. In the modified grades, the hemicelluloses are also modified together with the cellulose domain. Most probably, the modification is not homogeneous, i.e., some parts may be more modified than others. Thus, a detailed chemical analysis is usually not possible as the modified products are complicated mixtures of different polysaccharide structures.

In an aqueous environment, a dispersion of cellulose nanofibrils forms a viscoelastic hydrogel network. The gel is formed already at relatively low concentrations of for example 0.05-0.2% (w/w) by dispersed and hydrated entangled fibrils. The viscoelasticity of the NFC hydrogel may be characterized for example with dynamic oscillatory rheological measurements.

The nanofibrillar cellulose hydrogels exhibit characteristic rheological properties. For example, they are shear-thinning or pseudoplastic non-Newtonian materials, which may be considered as a special case of thixotropic behavior, which means that their viscosity depends on the speed or force by which the material is deformed. When measuring the viscosity in a rotational rheometer, the shear-thinning behavior is seen as a decrease in viscosity with increasing shear rate. At low enough shear rates, shear thinning fluids will show a constant viscosity value, $\eta0$, termed the zero-shear viscosity or zero-shear viscosity plateau. The zero-shear-rate viscosity is a limiting value that cannot be measured directly; rather, it must be estimated by extrapolation from several measurements at different shear rates. The zero-shear viscosity value is determined from the constant region of the measured curve and it represents the situation wherein the shear rate approaches zero.

Rheometers that control the applied shear stress or shear strain are called rotational or shear rheometers, and can be used to measure the way in which a liquid, suspension or slurry flows in response to applied forces. They are used for shear-thinning fluids which cannot be defined by a single value of viscosity. The rheometer may use a rotor with a specific geometry, such as vane or plate geometry.

The hydrogels show plastic behavior, which means that a certain shear stress (force) is required before the material starts to flow readily. This critical shear stress is often called the yield stress. The yield stress can be determined from a steady state flow curve measured with a stress-controlled rheometer. When the viscosity is plotted as function of applied shear stress, a dramatic decrease in viscosity is seen after exceeding the critical shear stress. The zero-shear viscosity and the yield stress are the most important rheological parameters to describe the suspending power of the materials. These two parameters separate the different grades quite clearly and thus enable classification of the grades. A stress-controlled rotational rheometer can be used to measure both zero shear viscosity and yield stress as well as other rheological properties. The measurement may be carried out in pure water at pH 7 at 25±1°C or 22±1°C.

The dimensions of the fibrils and/or fibril bundles, rheological properties as well as other properties of the nanofibrillar cellulose, are dependent for example on the raw material, modification type and degree, the disintegration method and number of disintegration runs (passes). Mechanical disintegration of the cellulose raw material may be carried out with any suitable equipment such as a refiner, a grinder, a disperser, a homogenizer, a colloider, a friction grinder, a pin mill, a rotor-rotor disperser, an ultrasound sonicator, a fluidizer such as a microfluidizer, a macrofluidizer or a fluidizer-type homogenizer. The disintegration treatment is performed at conditions wherein water is sufficiently present to prevent the formation of bonds between the fibers. The disintegration may be called fibrillation, wherein fibrils are separated from fibrous cellulose.

In one example the disintegration is carried out by using a disperser having at least one rotor, blade or similar moving mechanical member, such as a rotor-rotor disperser, which has at least two rotors. In a disperser the fiber material in dispersion is repeatedly impacted by blades or ribs of rotors striking it from opposite directions when the blades rotate at the rotating speed and at the peripheral speed determined by the radius (distance to the rotation axis) in opposite directions. Because the fiber material is transferred outwards in the radial direction, it crashes onto the wide surfaces of the blades, i.e., ribs, coming one after the other at a high peripheral speed from opposite directions; in other words, it receives a plurality of successive impacts from opposite directions. Also, at the edges of the wide surfaces of the blades, i.e., ribs, which edges form a blade gap with the opposite edge of the next rotor blade, shear forces occur, which contribute to the disintegration of the fibers and detachment of fibrils. The impact frequency is determined by the rotation speed of the rotors, the number of the rotors, the number of blades in each rotor, and the flow rate of the dispersion through the device.

In a rotor-rotor disperser the fiber material is introduced through counter-rotating rotors, outwards in the radial direction with respect to the axis of rotation of the rotors in such a way that the material is repeatedly subjected to shear and impact forces by the effect of the different and/or separate counter-rotating rotors, whereby it is simultaneously fibrillated. One example of a rotor-rotor disperser is an Atrex device.

Another example of a device suitable for disintegrating is a pin mill, such as a multi-peripheral pin mill. One example of such device includes a housing and in it a first rotor equipped with collision surfaces; a second rotor concentric with the first rotor and equipped with collision surfaces, the second rotor being arranged to rotate in a direction opposite to the first rotor; or a stator concentric with the first rotor and equipped with collision surfaces. The device includes a feed orifice in the housing and opening to the center of the rotors or the rotor and stator, and a discharge orifice on the housing wall and opening to the periphery of the outermost rotor or stator.

In one example the disintegrating is carried out by using a homogenizer. In a homogenizer the fiber material is subjected to homogenization by an effect of pressure. The homogenization of the fiber material dispersion to nanofibrillar cellulose is caused by forced through-flow of the dispersion, which disintegrates the material to fibrils. The fiber material dispersion is passed at a given pressure through a narrow through-flow gap where an increase in the linear velocity of the dispersion causes shearing and impact forces on the dispersion, resulting in the removal of fibrils from the fiber material. The fiber fragments are disintegrated into fibrils in the fibrillating step. In order to obtain fibrillated cellulose, the homogenization process is carried out with such adjustment of the device, such as the size of the gap, and with such process conditions, that the cellulose is disintegrated. A homogenizer can also be used for homogenizing cellulosic material without fibrillating, depending on said adjustment and process conditions.

As used herein, the term "fibrillation" generally refers to disintegrating fiber material mechanically by work applied to the particles, where cellulose fibrils are detached from the fibers or fiber fragments. The work may be based on various effects, like grinding, crushing or shearing, or a combination of these, or another corresponding action that reduces the particle size. The expressions "disintegration" or "disintegration treatment" may be used interchangeably with "fibrillation".

The fiber material dispersion that is subjected to fibrillation is a mixture of fiber material and water, also herein called "pulp". The fiber material dispersion may refer generally to whole fibers, parts (fragments) separated from them, fibril bundles, or fibrils mixed with water, and typically the aqueous fiber material dispersion is a mixture of such elements, in which the ratios between the components are dependent on the degree of processing or on the treatment stage, for example number of runs or "passes" through the treatment of the same batch of fiber material.

One way to characterize the nanofibrillar cellulose is to use the viscosity of an aqueous solution or dispersion containing said nanofibrillar cellulose. The viscosity may be for example Brookfield viscosity or zero shear viscosity. The specific viscosity, especially zero shear viscosity, as described herein, can be used to distinguish nanofibrillar cellulose from non-nanofibrillar cellulose, and/or to define the fibrillation degree. A high zero shear viscosity usually corresponds to a high fibrillation degree, to a high aspect ratio and/or to a low fibril diameter. Brookfield viscosity cannot properly characterize the shear-thinning properties, so it can be used for example for comparing different batches and in quality control.

The nanofibrillar cellulose may also be characterized by the average diameter (or width), or by the average diameter together with the viscosity, such as zero shear viscosity. In one example nanofibrillar cellulose suitable for use in the products described herein has a number average fibril diameter in the range of 1-200 nm, or 1-100 nm. In one example said nanofibrillar cellulose has a number average fibril diameter in the range of 1-50 nm, such as 2-20 nm or 5-30 nm. In one example said nanofibrillar cellulose has a number average fibril diameter in the range of 2-15 nm, such as in the case of TEMPO oxidized nanofibrillar cellulose.

The diameter of a fibril may be determined with several techniques, such as by microscopy. Fibril thickness and width distribution may be measured by image analysis of microscope images, such as images from a field emission scanning electron microscope (FE-SEM), a transmission electron microscope (TEM), such as a cryogenic transmission electron microscope (CRYO-TEM), or an atomic force microscope (AFM). In general AFM and TEM, especially CRYO-TEM, suit best for nanofibrillar cellulose grades with narrow fibril diameter distribution. From Cryo-TEM images, also the bundled structure can be seen.

Degree of fibrillation can be evaluated by using fiber analysis where number of larger, only partially fibrillated, entities are evaluated. For example, in the case of derivatized nanofibrillar cellulose the number of those particles per mg of dry sample may be in the range of 0-10000, such as in the range of 0-5000, for example in the range of 0-1000. However, in non-derivatized NFC the number of non-fibrillated particles/mg is typically somewhat higher in the range of 0-20000, such as in the range of 0-10000, for example in the range of 0-5000. The fiber analysis may be carried out using Fiberlab method.

The stiffness of the nanofibrillar cellulose hydrogels can be evaluated from viscoelastic measurements of the gels. Typically, the storage modulus for 0.5% (by weight) nanofibrillar cellulose hydrogel in pure water at pH 7 at $25\pm1°C$ or $22\pm1°C$ is between 1 to 50 Pa, preferably 2 to 20 Pa. Often the derivatized NFC builds up stiffer hydrogels, but extensive fibrillation of these grades may lead also to lower storage modulus.

The rheometers and methods disclosed herein may be used for determining zero-shear viscosity, yield stress/fracture strength, storage modulus, and loss modulus.

A rheometer viscosity of the nanofibrillar cellulose dispersion may be measured according to one example at 22°C with a stress controlled rotational rheometer (AR-G2, TA Instruments, UK) equipped with a narrow gap vane geometry (diameter 28 mm, length 42 mm) in a cylindrical sample cup having a diameter of 30 mm. After loading the samples to the rheometer, they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s$^{-1}$ is exceeded. This method may be used for determining the zero-shear viscosity.

In another example rheological measurements of the hydrogel samples were carried out with a stress-controlled rotational rheometer (AR-G2, TA instruments, UK) equipped with 20 mm plate geometry. After loading the samples to the rheometer, 1 mm gap, without dilution, they were allowed to settle for 5 min before the measurement was started. The stress sweep viscosity was measured with gradually increasing shear stress in a range of 0,001-100 Pa at the frequency 10 rad/s, strain 2%, at 25°C. Storage modulus, loss modulus and yield stress/fracture strength may be determined.

In one example the nanofibrillar cellulose, for example provided as a starting material in the method, when dispersed in water, provides a zero shear viscosity ("plateau" of constant viscosity at small shearing stresses) in the range of 1000-100000 Pa·s, in the range of 1000-10000 Pa·s, such as in the range of 5000-50000 Pa·s, and a yield stress (shear stress where the shear thinning begins) in the range of 1-50 Pa, such as in the range of 2-15 Pa, determined by rotational rheometer at a consistency of 0.5% (w/w) by weight in aqueous medium at $22\pm1°C$. Such nanofibrillar cellulose may also have an average fibril diameter of 200 nm or less, such as in the range of 1-200 nm, or 1-100 nm.

Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry is used. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample.

In one turbidity measurement method, a nanofibrillar cellulose sample is diluted in water, to a concentration below the gel point of said nanofibrillar cellulose, and turbidity of the diluted sample is measured. Said concentration where the turbidity of the nanofibrillar cellulose samples is measured is 0.1%. HACH P2100 Turbidometer with a 50 ml measuring vessel is used for turbidity measurements. The dry matter of the nanofibrillar cellulose sample is determined and 0.5 g of the sample, calculated as dry matter, is loaded in the measuring vessel, which is filled with tap water to 500 g and vigorously mixed by shaking for about 30 s. Without delay the aqueous mixture is divided into 5 measuring vessels, which are inserted in the turbidometer. Three measurements on each vessel are carried out. The mean value and standard deviation are calculated from the obtained results, and the final result is given as NTU units.

One way to characterize nanofibrillar cellulose is to define both the viscosity and the turbidity. Low turbidity refers to small size of the fibrils, such as small diameter, as small fibrils scatter light poorly. In general, as the fibrillation degree increases, the viscosity increases and at the same time the turbidity decreases. This happens, however, until a certain point. When the fibrillation is further continued, the fibrils finally begin to break and shorten, and therefore cannot form a strong network anymore. Therefore, after this point, both the turbidity and the viscosity begin to decrease.

In one example the turbidity of anionic nanofibrillar cellulose is lower than 90 NTU, for example from 3 to 90 NTU, such as from 5 to 60, for example 8-40 measured at a consistency of 0.1% (w/w) in aqueous medium and measured by nephelometry. In one example the turbidity of native nanofibrillar may be even over 200 NTU, for example from 10 to 220 NTU, such as from 20 to 200, for example 50-200 measured at measured at 20°C±1°C a consistency of 0.1% (w/w) in aqueous medium and measured by nephelometry. To characterize the nanofibrillar cellulose these ranges may be combined with the viscosity ranges of the nanofibrillar cellulose, such as zero shear viscosity, storage modulus and/or yield stress.

Nanofibrillar cellulose may be or comprise non-modified nanofibrillar cellulose. The drainage of non-modified nanofibrillar cellulose is significantly faster than for example anionic grade. Non-modified nanofibrillar cellulose generally has a Brookfield viscosity in the range of 2000-10000 mPa·s, measured at 20°C±1°C, at a consistency of 0.8% (w/w) and at 10 rpm.

The disintegrated fibrous cellulosic raw material may be modified fibrous raw material. Modified fibrous raw material means raw material where the fibers are affected by the treatment so that cellulose nanofibrils are more easily detachable from the fibers. The modification is usually performed to fibrous cellulosic raw material which exists as a suspension in a liquid, i.e., pulp.

The modification treatment to the fibers may be chemical, enzymatic and/or physical. In chemical modification the chemical structure of cellulose molecule is changed by chemical reaction ("derivatization" of cellulose), preferably so that the length of the cellulose molecule is not affected but functional groups are added to β-D-glucopyranose units of the polymer. The chemical modification of cellulose takes place at a certain conversion degree, which is dependent on the dosage of reactants and the reaction conditions, and as a rule it is not complete so that the cellulose will stay in solid form as fibrils and does not dissolve in water. In physical modification anionic, cationic, or non-ionic substances or any combination of these are physically adsorbed on cellulose surface.

The cellulose in the fibers may be especially ionically charged after the modification. The ionic charge of the cellulose weakens the internal bonds of the fibers and will later facilitate the disintegration to nanofibrillar cellulose. The ionic charge may be achieved by chemical or physical modification of the cellulose. The fibers may have higher anionic or cationic charge after the modification compared with the starting raw material. Most commonly used chemical modification methods for making an anionic charge are oxidation, where hydroxyl groups are oxidized to aldehydes and carboxyl groups, sulphonization and carboxymethylation. Chemical modifications introducing groups, such as carboxyl groups, which may take part in forming a covalent bond between the nanofibrillar cellulose and the bioactive molecule, may be desired. A cationic charge in turn may be created chemically by cationization by attaching a cationic group to the cellulose, such as quaternary ammonium group.

Nanofibrillar cellulose may comprise chemically modified nanofibrillar cellulose, such as anionically modified nanofibrillar cellulose or cationically modified nanofibrillar cellulose. In one example the nanofibrillar cellulose is anionically modified nanofibrillar cellulose. In one example the anionically modified nanofibrillar cellulose is oxidized nanofibrillar cellulose. In one example the anionically modified nanofibrillar cellulose is sulphonized nanofibrillar cellulose. In one example the anionically modified nanofibrillar cellulose is carboxymethylated nanofibrillar cellulose. The material obtained with the anionical modification of cellulose may be called anionic cellulose, which refers to material wherein the amount or proportion of anionic groups, such as carboxylic groups, is increased by the modification, when compared to a non-modified material. It is also possible to introduce other anionic groups to the cellulose, instead or in addition to carboxylic groups, such as phosphate groups or sulphate groups. The content of these groups may be in the same ranges as is disclosed for carboxylic acid herein.

In the chemical derivatization process a desired degree of substitution of the cellulose can be obtained. This may be carried out by controlling the chemical derivatization process, such as by selecting suitable raw material(s), reaction conditions, equipment, reaction time, reagents and/or the like properties. For example, TEMPO or N-oxyl mediated oxidation is typically conducted to charge values in the range of 300-1500 micromol/g, preferably 600-1200 micromol/g, most preferably 700-1100 micromol/g. The oxidized NFC may contain also aldehyde functional groups, typically in the range of 10-250 micromol/g, such as 50-250 micromol/g. Derivatization via carboxymethylation is typically conducted for cellulose pulp to ds levels in the range of 0.05-0.3, preferably 0.08-0.25, most preferably 0.10-0.2 prior to fibrillation. If the derivatization is conducted by cationization, the DS levels are typically in the range of 0.05-0.4, preferably 0.15-0.3.

The cellulose may be oxidized. In the oxidation of cellulose, the primary hydroxyl groups of cellulose may be oxidized catalytically by a heterocyclic nitroxyl compound, such as through N-oxyl mediated catalytic oxidation, for example 2,2,6,6-tetramethylpiperidinyl-1-oxy free radical, generally called "TEMPO". The primary hydroxyl groups (C6-hydroxyl groups) of the cellulosic β-D-glucopyranose units are selectively oxidized to carboxylic groups. Some aldehyde groups are also formed from the primary hydroxyl groups. Regarding the finding that low degree of oxidation does not allow efficient enough fibrillation and higher degree of oxidation inflicts degradation of cellulose after mechanical disruptive treatment, the cellulose may be oxidized to a level having a carboxylic acid content in the oxidized cellulose in the range of 0.5-2.0 mmol COOH/g pulp, 0.6-1.4 mmol COOH/ g pulp, or 0.8-1.2 mmol COOH / g pulp, preferably to 1.0-1.2 mmol COOH/ g pulp, determined by conductometric titration. When the fibers of oxidized cellulose so obtained are disintegrated in water, they give stable transparent dispersion of individualized cellulose fibrils, which may be, for example, of 3-5 nm in width. With oxidized pulp as the starting medium, it is possible to obtain nanofibrillar cellulose where Brookfield viscosity measured at a consistency of 0.8% (w/w) is at least 10000 mPa·s, for example in the range of 10000-30000 mPa·s.

Whenever the catalyst "TEMPO" is mentioned in this disclosure, it is evident that all measures and operations where "TEMPO" is involved apply equally and analogously to any derivative of TEMPO or any heterocyclic nitroxyl radical capable of catalyzing selectively the oxidation of the hydroxyl groups of C6 carbon in cellulose.

Auxiliary agents for enhancing the manufacturing process or improving or adjusting the properties of the NFC or products formed from the NFC may be included in the nanofibrillar cellulose dispersion. Such auxiliary agents may be soluble in the liquid phase of the dispersion, they may form an emulsion, or they may be solid. Auxiliary agents may be added already during the manufacturing of the nanofibrillar cellulose dispersion to the raw material or they may be added to a formed nanofibrillar cellulose dispersion or gel. The auxiliary agents may be also added to the final product, i.e. the microbeads, for example by impregnating, spraying, dipping, soaking or the like method. The auxiliary agents are usually not covalently bound to the nanofibrillar cellulose, so they may be releasable from the nanocellulose matrix. A controlled and/or sustained release of such agents may be obtained when using NFC as matrix. Examples of auxiliary agents include therapeutic (pharmaceutic) agents and other agents affecting to the properties of the nanofibrillar cellulose or to the properties of the cells and/or cell products, such as buffers, surfactants, plasticizers, emulsifiers, bioactive agents or the like. In one example the dispersion contains one or more salts, which may be added to enhance the properties of the final product. Examples of salts include chloride salts, such as sodium chloride. The salt may be included in a concentration in the range of 0.01-1.0% (w/w) of the dispersion. The NFC product may also be dipped or soaked in a solution of sodium chloride, such as in an aqueous solution of about 0.9% sodium chloride. Desired salt content in the NFC product may be in the range of 0.5-1%, such as about 0.9%, of the weight of the wet product. The salts, buffers and the like agents may be provided to obtain physiological conditions.

One example provides a method for preparing such a hydrogel, the method comprising providing pulp, disintegrating the pulp until nanofibrillar cellulose is obtained, forming the nanofibrillar cellulose into a hydrogel

The nanofibrillar cellulose may be fibrillated into a desired fibrillation degree and adjusted into desired water content, or otherwise modified, so that it forms a hydrogel having desired properties as described herein. In one example the nanofibrillar cellulose in the hydrogel is anionically modified nanofibrillar cellulose.

The hydrogel to be used in the present applications is preferably homogenous. Therefore, the method may include homogenizing a hydrogel comprising nanofibrillar cellulose, preferably with a homogenizing device such as ones described herein. With this preferably non-fibrillating homogenizing step it is possible to remove areas of discontinuity from the gel. Especially it is possible to control the properties of wood cellulose with such treatment. A homogenous gel having better properties for the applications is obtained. The hydrogel may be further sterilized, for example by using heat and/or radiation, and/or by adding sterilizing agents, such as antimicrobials. The hydrogels obtained as discussed in previous may be used for forming the entities.

The present application discloses use of nanofibrillar cellulose for preparing the hydrogels and entities, such as microbeads, disclosed herein. The nanofibrillar cellulose may be any suitable nanofibrillar cellulose disclosed herein. Specifically disclosed is use of chemically anionically modified nanofibrillar cellulose for preparing the microbeads disclosed herein.

*Use of the microbeads*

Providing the nanofibrillar cellulose in the form of microbeads, which may be also called as separate or discontinuous entities, help controlling the conditions of a cell culture or a cell storage system. A so-called three-dimensional cell culture or cell system is obtained, wherein the microbeads may be present as three-dimensional discontinuous entities. The term "three-dimensional" is used to distinguish from "two dimensional", which refers to a system based on a layer, such as cell culture in a layer, which has a low thickness. The cells can be combined with the present microbeads in any suitable way. The expressions such as "cells combined with microbeads", "cells with microbeads" and "cells in and/or on the microbeads" refer to any suitable combinations of any applicable cells with the microbeads, such as cells on the microbeads, cells partly embedded in the microbeads, cells completely embedded or encapsulated in the microbeads, or combinations thereof. The cells may be any applicable cells, such as any cells disclosed herein.

For example, the viscosity of a suspension of separate nanofibrillar cellulose microbeads is much lower than a suspension of homogenous nanofibrillar cellulose, especially nanofibrillar cellulose, which in most cases is present as a viscous hydrogel. This makes mixing of such viscous suspension challenging, especially when the concentration increases. The separate microbeads on the other hand may each have a relatively high nanofibrillar cellulose concentration, fibrillation degree and/or aspect ratio, but still they can be handled without problems. The same may apply also for properties such as migration of substances, such as nutrients, gases, metabolites and/or cell-derived products, which may be more easily released from the separate microbeads compared to homogenous nanofibrillar cellulose hydrogel with a large volume. The microbeads also enable culturing the cells on a surface and/or below the surface of the microbeads, i.e., embedded or encapsulated, which terms may be used interchangeably, which may be desired in some cases. For example, the exchange of substances, including release of the cell-derived products, may be faster and/or more efficient in such cases. It is also possible to better maintain the structure of produced and released extracellular vesicles and the like cell-derived structures, especially ones having a lipid monolayer or bilayer. It was also noted that using such microbeads may be desired for cells incubated, cultured and/or provided at a stressed state. A high stress threshold can be maintained in such case. When cells are incubated or cultured on and/or in the microbeads, especially inside the microbeads, the cells can tolerate higher flow and stronger mixing or agitation. This enables for example providing more efficient culture reactor and conditions, more efficient mixing, and/or more efficient production of cell-derived products.

One example provides the microbeads for culturing cells comprising an aqueous medium and the microbeads in the aqueous medium. The microbeads may be applied to and/or they may be in a cell culture container or a bioreactor. The aqueous medium may be cell culture medium.

The present disclosure provides a cell culture comprising cells in the microbeads discussed herein. The cells may be on the surface of the microbeads and/or the cells may be inside the microbeads, either partly or fully. The cells may be embedded or encapsulated in the microbeads.

Using microbeads and combining the cells with the microbeads in a selected manner facilitates and helps controlling cell culturing, cell incubating, cell-derived product formation and/or release, exchange of nutrients, liquids and/or gases, and the like properties. By using the microbeads the mixing of the mixture of cells and microbeads can be facilitated as the suspension of entities has a relatively low viscosity.

The microbeads may be used for culturing cells with known methods, and they may also be used for producing cell-derived products. The cells may be cultured in a cell culture container, which may be any suitable cell culture container, such as a cell culture plate, a multiwell plate, a flask, or in a bioreactor, which comprises one or more containers, one or more compartments in a container, any required devices such as one or more pumps, mixers or other actuators to facilitate the process and movement of liquids, suspensions and/or cells, one or more filters or other separating surfaces to separating cells and other substances, such as cell products and/or other suitable parts. A cell culture method may comprise providing cells, microbeads and a cell culture container and/or a bioreactor for culturing the cells.

The microbeads can be used to produce and/or separate, isolate and/or harvest cell-derived products. The cell-derived products are obtained from the cells, i.e. the cells provide or produce the cell-derived products. The cell-derived products may be any products derived from the cell, such as excreted and/or secreted by the cells, and/or otherwise released from the cells. The cells are not cell-derived products. The cell-derived products are preferably bioactive substances, which may be called in general as "biologics", so cell-derived products and biologics as used herein are interchangeable terms. It is desired to maintain the bioactivity of such substances.

The cell-derived products may be or comprise vesicles, such as extracellular vesicles, for example exosomes, but they may also be or comprise other applicable cell-derived products, such as (macro)molecules, such as proteins, carbohydrates, lipids, nucleic acids, antibodies, hormones, other applicable molecules, cell organelles, viruses, parts thereof and/or virus-like particles, and the like substances and/or entities obtained from the cells. The cell-derived products may be recombinant products, such as recombinant molecules or viruses. The present method is mainly explained in the context of extracellular vesicles, more particularly exosomes, but the embodiments and examples may be applied to other cell-derived products as well. The cells may be treated with recombinant methods or recombinant methods or other production methods may be applied in the cells. For example, the cells may contain a plasmid or a virus or other vector designed to produce a product.

The microbeads may be used in methods and uses for capturing, stabilizing, storing, transporting, providing, delivering, and/or administering cells and/or cell-derived products, and/or bioactive compounds, agents or substances in general.

In one embodiment the cell-derived products are or comprise extracellular vesicles, such as exosomes, preferably wherein the cells are mammalian cells.

The present cell-derived products may have a number-average diameter of 1000 nm or less, 800 nm or less, or 500 nm or less, such as a number-average diameter in the range of 5-1000 nm, such as 20-1000 nm or 20-500 nm, or less, and they may have a relatively narrow size distribution. The cell-derived products may comprise extracellular vesicles and smaller substances and/or molecules, such as one or more of those mentioned herein.

In one example the cell-derived products comprise or are viruses, parts thereof, virus-like particles, viral products and/or viral vectors. These may be used for example for manufacture of vaccines or in gene therapy. In such case the cells are hosts cells for the viruses.

In one example the cell-derived products comprise or are proteins, such as therapeutic proteins, for example inti-inflammatory cytokines and other molecules, insuline, hormones etc. In one example the cell-derived products are or comprise antibodies. The cell-derived products may be or comprise a secretome, which according to one definition is a set of proteins expressed by an organism and secreted into the extracellular space. However also other cell-derived products discussed herein may be included in the definition of secretome.

Blood cells may be excluded from the cell-derived products, such as red blood cells, white blood cells and/or platelets may not be considered as the cell-derived products. Already the smallest blood cells platelet (thrombocytes) have the greatest diameter of 2-3 $\mu$m, and red and white blood cells are even larger. However, even as these are significantly larger than the cell-derived products discussed in previous, in some cases it may be possible to use the present materials and methods also for extracting blood cells.

The size of the cell-derived products may have an impact to the migration speed and efficiency in the microbeads. For example, vesicles, viruses and the like entities are larger than for example certain macromolecules, and as lipid monolayer or bilayer containing entities they may be fragile or otherwise such dynamic structures that the microbeads may have a substantial impact to the migration and maintenance of such cell-derived products. Therefore, it may be desired to culture cells for such cell-derived products on the surface of the microbeads and/or embedded in the microbeads, and/or use ANFC of such type that allows the migration of the cell-derived products.

With the present microbeads a three-dimensional cell system can be provided enabling incubation and/or culturing cells to produce cell-derived products. The three-dimensional cell system stimulates the secretion of *in vivo* like cell-derived products, such as extracellular vesicles, so it is desired to use three-dimensional (3D) cell system obtained from the microbeads. The amount of produced cell-derived products is therefore higher with 3D cell system compared to 2D systems. For example, three-dimensional spheroid system increases exosome secretion from mesenchymal stem cells. It was observed that 3D system derived EVs showed significantly different profiles in terms of secretion dynamics and signalling molecular contents (RNAs and DNAs) compared to 2D system derived EVs. There have been however challenges to implement such three-dimensional cell culture in efficient and reliable way, especially in bioreactors and the like environments. Even with 3D systems the risk of phenotypic alterations at the cellular level due to shear stress has been an issue. The present microbeads help avoiding these problems.

In one example a bioreactor, for example for extracting cell-derived products from cultured cells, comprises

- a container,
- an inlet for inputting cell culture medium into the container,
- an outlet for outputting cell culture medium comprising cell-derived products from the container,
- the container comprising, or being connected to, a compartment comprising microbeads configured to receive cells, said compartment comprising a first separating surface separating the microbeads from the outlet and allowing cell culture medium comprising cell-derived products to pass through the first separating surface.

On one example a method for extracting cell-derived products from cultured cells comprises

- providing the bioreactor or a cell culture container,
- providing microbeads,
- providing cell culture medium, preferably serum-free, animal origin free, feederfree and/or xeno-free cell culture medium,
- providing cells,
- incubating the cells in the bioreactor, such as in a compartment of the bioreactor, comprising the microbeads or in the cell culture container comprising the microbeads to form cell-derived products, and optionally mixing the microbeads and the cells,
- allowing cell-derived products to diffuse from the cells into the cell culture medium,
- harvesting the cell culture medium comprising the cell-derived products from the bioreactor or the cell culture container, preferably via the outlet for extracting the cell culture medium comprising the cell-derived products, to separate the cell-derived products from the incubated cells.

In one example a process of production of cell-derived products, such extracellular vesicles (EV), is as follows. Microbeads are mixed with culture medium and cells to final concentration to obtain a mixture of cells and microbeads. The mixture of cells and microbeads is added to a bioreactor. It is possible to further control the cultivation, as the cells may be cultured on top or on the surface of the microbeads, or they may be allowed to penetrate and embed the microbeads. The mixture of cells and microbeads will either be maintained by the addition of new media during culture or removed, such as siphoned off, and replaced with fresh cells and/or microbeads. The cells are allowed to produce EVs and secrete them to the medium. New medium may be added to the system as EV-enriched medium is removed. EV-enriched medium may be harvested at a rate equal to addition of new medium, where a permeabilized membrane may be used to separate cells and microbeads from the EV-enriched medium.

In one example the process of production of cells on top of microbeads, is as follows. ANFC is provided as microbeads or made into microbeads, mixed with culture medium and cells to a final concentration obtain a mixture of cells and microbeads. The mixture of cells and microbeads is added to a bioreactor. The cells are allowed to produce EVs and secrete them to the medium. The mixture of cells and microbeads is either maintained by the addition of new medium during culture, or removed, such as siphoned off, and replaced with fresh cells and/or microbeads. New medium may be added to the system as EV-enriched medium is removed. EV-enriched medium may be harvested at a rate equal to addition of new medium. A permeabilized or semi-permeable membrane may be used to separate cells and microbeads from the EV-enriched medium.

The cells on and/or in the microbeads may be transfected, for example with a plasmid, and/or infected with a virus, and incubated and/or cultured to produce a desired product, such as a recombinant product, a virus or a virus particle. This can be carried out in the 3D systems discussed herein, for example by using a suitable bioreactor or cell culture container.

In one example the process of production of cells embedded within microbeads is as follows. ANFC is mixed with medium and cells, and then formed into microbeads. The mixture of cells and microbeads is added to bioreactor. The cells are allowed to produce EVs and secrete them to the medium. The mixture of cells and microbeads is either maintained by the addition of new medium during culture, or removed, such as siphoned off, and replaced with fresh cells and/or microbeads. New medium may be added to the system as EV-enriched medium is removed. EV-enriched medium may be harvested at a rate equal to addition of new medium. A permeabilized or semi-permeable membrane may be used to separate cells and microbeads from the EV-enriched medium.

The microbeads may be provided as microcarriers. In general, microcarriers are one of the first methods used for supporting large-scale mammalian cell culture. Conventionally microcarriers are small beads (usually less than 500 $\mu$m in diameter) with surfaces treated to support cell attachment (adherence). These beads are then maintained in suspension in medium using very low stirring speeds in order to avoid mechanical cell damage, either due to shearing forces in the liquid or due to bead-bead collisions. A desired property of a microcarrier is a high surface area to volume ratio, allowing large populations of cells, such as stem cells, to be cultured in a relatively small vessel.

Examples of bioreactor configurations using microcarriers include packed and fluidized beds. Reactor volume is proportional to the microcarrier diameter, thus it is usually advantageous to reduce the microcarrier size as much as possible. However, packed beds with small beads may clog and the cells may have a tendency to accumulate in the channels between the microcarrier surfaces. Total flow rate is mainly dependent on cell number and the nutrient uptake rate of the cells.

With the present microbeads it was however not necessary to treat the surfaces of the beads to facilitate cell attachment. It was possible to obtain microcarrier beads with desired diameter and as the cells can be encapsulated in the microbeads, or as the cells can at least partly penetrate to the beads, the problems associated with clogging accumulating may be avoided or reduced.

In addition to culturing, carrying and storing, as discussed herein, the microbeads may be applied for studying the cells. For example, different examination methods may be applied, including microscopic methods, radiographic methods, for example when the cells or other substances are labelled or provided as labelled, wherein the label may be a radioisotope label, a fluorescent label or other applicable type of label, or other methods comprising monitoring and/or detecting a response of the cells to one or more external stimulus, to one or more substances and the like. Therefore, one example provides a method for studying, examining and/or monitoring cells, the method comprising providing cells in and/or on the microbeads, for example in any form or with any method disclosed herein, and studying and/or examining the cells in and/or on the microbeads. The method may also comprise incubating and/or culturing the cells in and/or on the microbeads, especially for a sufficient time to obtain a possible, an expected or an alleged response or reaction.

The microbeads may be applied to capturing bioactive substances from cells or tissue extracts, such as specific proteins or nucleic acids. The bioactive substances may be cell-derived or tissue-derived products. Such capturing properties may be obtained by adjusting the properties of the microbeads, such as concentration, cross-linking, type of NFC, preparation method and conditions, auxiliary agents, coating of the microbeads, and the like properties. The microbeads may be used in methods comprising providing cells or tissue extract, providing the microbeads, combining the cells or the tissue extract with the microbeads, and incubating a time period sufficient to capture biomolecules obtained from the cells or the tissue extract with the microbeads. The method may comprise washing the microbeads to remove the cells or the tissue extract. The method may comprise recovering the bioactive molecules from the microbeads.

The present disclosure provides use of the microbeads for providing bioactive substances to a target. The bioactive substances may be any of the cell-derived products disclosed herein, or other bioactive compounds or substances, such as therapeutic (pharmaceutic) agents, for example synthetic pharmaceutical molecules, or combinations thereof. The target may be or comprise cells, tissue and/or a patient, or the target may be or comprise a target environment such as solution or other environment, for example for research or medical purposes, for example in a test tube, microwell, or other applicable container or environment, wherein the method may be an *in vitro* method. The present disclosure provides the microbeads for use for providing bioactive substances to a target.

The microbeads may be brought into contact with the target, and preferably allowed to be in contact for a time period efficient for allowing migration of the bioactive substances from the microbeads to the target. It is also possible to bring the target, especially in the case of cells, into contact with microbeads, wherein the target may migrate or enter, partly or fully, into the microbeads.

The present disclosure provides use of the microbeads for culturing and/or incubating cells.

One example provides a method for providing bioactive substances to a target, the method comprising

- providing the microbeads,
- providing bioactive substances in the microbeads, and
- contacting a target with the microbeads to provide the bioactive substance(s) to the target.

The method may be *in vitro* or *in vivo* method. The method may comprise providing bioactive substances, and combining the bioactive substances with the microbeads. The method may be a method for producing and/or providing cell-derived products as the bioactive substances, the method comprising

- providing the microbeads,
- providing cells,
- combining the cells with the microbeads, and
- allowing the cells to produce cell-derived products, such as bioactive substances, for example with the methods disclosed herein.

The cell-derived products, or the bioactive substances, may be provided, such as released, from the microbeads as disclosed herein, for example by contacting or combining with a target. The cells may be incubated and/or culture for a time period enabling the production of the cell-derived products.

In one embodiment the bioactive substances are or comprise nucleic acids, such as DNA and/or RNA.

The microbeads may be used for purpose of the therapeutic delivery of nucleic acids to cells *in vivo,* for example for the purpose of the replacement of defective genes, or for the purpose of vaccination (analogous to the currently approved RNA vaccines against COVID-19). NFC is proved to be non-toxic material for cells, and thus, suitable for therapeutic delivery agent.

In addition, the microbeads may be used to delivery nucleic acids, or other bioactive compounds or substances as well, to cells *in vitro* for research or medical purposes, for example to manipulate intracellular processes and/or to investigate their function, or to prepare therapeutical cells and/or therapeutical substances, or to other targets.

*Cells*

The cells to be examined, studied, handled, stored, manipulated, treated, incubated and/or cultured with and/or in the microbeads and with the methods discussed herein can be any applicable cells.

In general, the cells may be cultured and/or incubated in the microbeads, and they can be maintained and proliferated on or in the microbeads without animal or human based agents or medium originating outside the cells. The cells may be evenly dispersed on or in the microbeads. The cells incubated or stored in the microbeads for the production purposes disclosed herein form a cell system.

Initially the cells may be pre-cultured in a separate culture in a first medium, and recovered and transferred into a new medium, which may be similar or different than the first medium. A cell suspension is obtained. This may be combined and/or mixed with the microbeads to obtain or form a cell system or a cell composition, which is a mixture of cells and microbeads. When cells are cultured in the cell system a cell culture is formed, preferably a 3D system or culture which refers to a system or culture in the microbeads, wherein the cells are permitted to grow and/or interact in all three dimensions. The nanofibrillar cellulose hydrogel matrix of the microbeads mimics the natural extracellular matrix structure and provides efficient transport of nutrients, gases and the like. When starting the cell incubation or culturing one or more propagation step(s) may be necessary to obtain a cell culture or a cell system which is optimal for production purposes. This may include inoculation and propagation of cells until a suitable cell density is obtained. After this the actual production can be initiated.

In one embodiment the cells are primary cells, such as stem cells. The cells may be eukaryotic cells, such as mammalian cells, for example human cells and/or non-human animal cells. In one embodiment the cells are human primary cells. In one embodiment the stem cells are human stem cells.

The term "cell culture" or "culturing of cells" may refer to one or more of maintaining, transporting, isolating, culturing, propagating, moving and/or differentiating of cells or tissues. The "cell system" may comprise the cell culture, but the cell system primarily aims to maintain the cells and to produce cell-derived products. It may be not desired to allow the cells to differentiate, and the propagation of the cells may be controlled and/or limited. It may also not be desired to transport, isolate and/or move the cells during the production. Cells may be maintained in any suitable arrangement, for example as individual cells, monolayers, cell clusters and/or spheroids, or as a tissue. In one embodiment the cells are present as aggregates, such as spheroids. The present microbead materials and arrangement thereof in a bioreactor or a container facilitate maintaining the cell system, such as maintaining the cells in a desired arrangement and exhibiting a desired phenotype.

The cells, especially eukaryotic cells, may be stem cells or differentiated cells, such as cells originated or derived from human or animal body. Specific examples of cells include stem cells, undifferentiated cells, precursor cells, as well as fully differentiated cells and combinations thereof. In some examples the cells comprise cell types selected from the group consisting of keratocytes, keratinocytes, fibroblast cells, epithelial cells and combinations thereof. In some examples the cells are selected from the group consisting of stem cells, progenitor cells, precursor cells, connective tissue cells, epithelial cells, muscle cells, neuronal cells, endothelial cells, fibroblasts, keratinocytes, smooth muscle cells, stromal cells, mesenchymal cells, immune system cells, hematopoietic cells, dendritic cells, hair follicle cells and combinations thereof. The cells may be cancer cells or cancer stem cells. The cells may be genetically modified cells, such as transgenic cells, cisgenic cells or knock-out cells, or pathogenic cells. Such cells may be used for example for drug research or in therapeutical applications, such as for providing therapeutic substances. Especially stem cells may be used in therapeutical applications, and in such case it is especially important to protect the cells and the obtained cell-derived products during the process by using the materials, devices and methods disclosed herein.

Eukaryotic cells may be animal cells, such as mammalian cells. Examples of animal and mammalian cells include human cells, and non-human animal cells, such as mouse cells, rat cells, rabbit cells, monkey cells, pig cells, bovine cells, chicken cells and the like.

In one embodiment the cells are stem cells, such as omnipotent stem cells, which may be non-human, pluripotent, multipotent, oligopotent or unipotent stem cells. Stem cells are cells capable of renewing themselves through cell division and can differentiate into multi-lineage cells. These cells may be categorized as embryonic stem cells (ESCs), induced pluripotent stem cells (iPSCs), and adult stem cells, also called as tissue-specific or somatic stem cells. The stem cells may be human stem cells, which may be of non-embryonic origin, such as adult stem cells. These are undifferentiated cells found throughout the body after differentiation. They are responsible for e.g. organ regeneration and capable of dividing in pluripotent or multipotent state and differentiating into differentiated cell lineages. The stem cells may be human embryonic stem cell lines generated without embryo destruction, such as described for example in Cell Stem Cell. 2008 Feb 7;2(2):113-7. The stem cells may be obtained from a source of autologous adult stem cells, such as bone marrow, adipose tissue, or blood.

Examples of stem cells include mesenchymal stem cells (MSC), multipotent adult progenitor cells (MAPC®), induced pluripotent stem cells (iPS), and hematopoietic stem cells.

In case of human stem cells the cells may be non-embryonic cells or embryonic cells, such as hESCs (human embryonic stem cells), which have been derived without destroying the embryo. In case of human embryonic stem cells the cells may be from a deposited cell line or made from unfertilized eggs, i.e. "parthenote" eggs or from parthenogenetically activated ovum, so that no human embryos are destroyed.

In one example the cells are mesenchymal stem cells (MSC). Mesenchymal stem cells (MSCs) are adult stem cells which can be isolated from human and animal sources, such as from mammals. Mesenchymal stem cells are multipotent stromal cells that can differentiate into a variety of cell types, including osteoblasts, chondrocytes, myocytes and adipocytes. Mesenchyme itself is embryonic connective tissue that is derived from the mesoderm and that differentiates into hematopoietic and connective tissue. However mesenchymal stem cells do not differentiate into hematopoietic cells. The terms mesenchymal stem cell and marrow stromal cell have been used interchangeably for many years, but neither term is sufficiently descriptive. Stromal cells are connective tissue cells that form the supportive structure in which the functional cells of the tissue reside. While this is an accurate description for one function of MSCs, the term fails to convey the relatively recently discovered roles of MSCs in the repair of tissue. The term encompasses multipotent cells derived from other non-marrow tissues, such as placenta, umbilical cord blood, adipose tissue, adult muscle, corneal stroma or the dental pulp of deciduous baby teeth. The cells do not have the capacity to reconstitute an entire organ

The International Society for Cellular Therapy has proposed minimum criteria to define MSCs. These cells (a) should exhibit plastic adherence, (b) possess specific set of cell surface markers, i.e. cluster of differentiation (CD)73, D90, CD105 and lack expression of CD14, CD34, CD45 and human leucocyte antigen-DR (HLA-DR) and (c) have the ability to differentiate in vitro into adipocyte, chondrocyte and osteoblast. These characteristics are valid for all MSCs, although few differences exist in MSCs isolated from various tissue origins. MSCs are present not only in fetal tissues but also in many adult tissues with few exceptions. Efficient population of MSCs has been reported from bone marrow. Cells which exhibit characteristics of MSCs have been isolated from adipose tissue, amniotic fluid, amniotic membrane, dental tissues, endometrium, limb bud, menstrual blood, peripheral blood, placenta and fetal membrane, salivary gland, skin and foreskin, sub-amniotic umbilical cord lining membrane, synovial fluid and Wharton's jelly.

Human mesenchymal stem cells (hMSC) display a very high degree of plasticity and are found in virtually all organs with the highest density in bone marrow. hMSCs serve as renewable source for mesenchymal cells and have pluripotent ability of differentiating into several cell lineages, including osteoblasts, chondrocytes, adipocytes, skeletal and cardiac myocytes, endothelial cells, and neurons in vitro upon appropriate stimulation, and in vivo after transplantation.

In one example the cells are multipotent adult progenitor cells (MAPC), which are derived from a primitive cell population that can be harvested from bone marrow, muscle and brain. MAPC are a more primitive cell population than mesenchymal stem cells, whilst they imitate embryonic stem cells characteristics they still retain adult stem cells potential in cell therapy. In vitro, MAPC demonstrated a vast differentiation potential to adipogenic, osteogenic, neurogenic, hepatogenic, hematopoietic, myogenic, chondrogenic, epithelial, and endothelial lineages. A key feature of MAPC is that they show large proliferative potential *in vitro* without losing their phenotype. MAPC may be used for treating a variety of diseases such as ischaemic stroke, graft versus host disease, acute myocardial infarct, organ transplant, bone repair and myelodysplasia. MAPC also enhance bone formation, promote neovascularisation, and have immunomodulatory effects.

Induced pluripotent stem cells (iPS) are a type of pluripotent stem cell that can be generated directly from adult cells. They can propagate practically indefinitely and may give rise to every other cell type in the body, including neurons, heart, pancreatic and liver cells. Induced pluripotent stem cells can be derived directly from adult tissues and they can be made in a patient-matched manner specific cell-derived products may be obtained which can be used without the risk of immune rejection. Human induced pluripotent stem cells are of special interest, and they can be generated from for example human fibroblasts, keratinocytes, peripheral blood cells, renal epithelial cells or other suitable cell types.

Hematopoietic stem cells (HSCs), also called as blood stem cells, are cells that can develop into all types of blood cells, including white blood cells, red blood cells, and platelets. Hematopoietic stem cells are found in the peripheral blood and the bone marrow. HSCs give rise to both the myeloid and lymphoid lineages of blood cells. Myeloid and lymphoid lineages both are involved in dendritic cell formation. Myeloid cells include monocytes, macrophages, neutrophils, basophils, eosinophils, erythrocytes, and megakaryocytes to platelets. Lymphoid cells include T cells, B cells, and natural killer cells.

The stem cells may be singe or separate stem cells, which are not aggregated, or they may be aggregated, such as stem cell spheroids, such as stem cell derived spheroids.

Cell spheroids refer to multicellular cell aggregates linked together by extracellular matrix, in this case multicellular cell aggregates linked together by nanofibrillar cellulose matrix. Spheroids are more complex than single cells present as separate cells due to dynamic cell-cell and cell-matrix interaction which makes them an important tool for resembling the *in vivo* tissues microenvironment *in vitro.* Cell spheroids can be formed by culturing or incubating cells in a matrix material to form three-dimensional cell culture system(s) containing multicellular aggregates or spheroids. When nanofibrillar cellulose was used as the matrix material cell spheroids could be obtained already after three days of culturing or incubating. In general, the diameter of cell spheroids may vary and range from tens of micrometres to over millimetre. However herein cell spheroids with a controlled diameter suitable for cell-derived product producing purposes could be obtained by controlling and adjusting the culturing and matrix formation materials and conditions. The cells may be pre-cultured in a separate culture in a first medium, and they are then formed into aggregates in the microbeads, and/or the cell aggregates are maintained in the microbeads.

## Examples

The percentages, such as the concentrations of nanofibrillar cellulose, are weight percentages unless otherwise indicated.

Wood cellulose from birch was selected as the raw material for the experimental work because it was found to provide desired properties for the present purposes relating to cell culture, cell-derived product production and handling and storing the cells. The wood cellulose could be modified efficiently by chemical oxidation and non-fibrillating and fibrillating mechanical treatments to obtain material with desired properties.

**Example 1: Using nanofibrillar cellulose to encapsulate cells and producing nanofibrillar cellulose microparticles by membrane emulsification**

Mesenchymal stem cells for cell-based therapies must be transported between the site of manufacture and site of clinical use without compromising therapeutic efficacy. Hypothermic storage of these cells in NFC is a promising method of short-term storage. However overall yield (% of live cells/original seeding density) is low, due to imperfect storage conditions at low temperature. Additionally, encapsulated cells in a 24 well plate format can be easily disrupted due to pipetting and cannot be efficiently released after storage due to mass-transfer limitations. The storage medium of encapsulated cells in NFC may be optimised for hypothermic conditions. In the present experiments small microparticles of NFC were produced in order to avoid mass-transfer limitations for cell release.

*Results*

In the present case process parameters and formulation for the production of uniform, spherical anionic nanofibrillar cellulose (ANFC) particles of controlled size via membrane emulsification for microcarrier and cell encapsulation applications were found.

NFC particles were produced by membrane emulsification, and the formulation was optimised for adequate particle uniformity to be achieved.

NFC concentration of 0.5% by weight and continuous phase formulation of 2 vol.% ABIL EM 90 in kerosene with process parameters of 1350 rpm rotation speed and 1 ml/min injection rate, using a ring membrane of 15 $\mu$m pore diameter, produced an emulsion with a $D_{av}$ of 115 $\mu$m and CV of 21.9% and was stable in size and uniformity over 150 minutes.

In order to produce successful ANFC particles, the process parameters were improved. An increased injection rate of 5 ml min$^{-1}$ and increased pore size to 40 $\mu$m did result in some ANFC being pushed through the membrane, however cellulose distribution was uneven, and some particles were misshapen. Homogenisation of ANFC with its diluent prior to emulsification proved to be essential for production of successful ANFC particles. These particles, produced with a 40 $\mu$m membrane pore size, had a $D_{av}$ of 220 $\mu$m and a CV of 26.4%. Homogenisation of ANFC also allowed use of the smaller membrane pore size. Therefore, a ring membrane of 15 $\mu$m pore diameter could be used to make microparticles of both NFC and ANFC.

However, it is necessary to produce more robust particles in order to accommodate downstream processing such as washing. Increasing NFC concentration was unsuccessful due to particle clumping and increased polydispersity and did not increase particle robustness. Addition of DMEM as a diluent for NFC, in order to replicate cell-compatible conditions, caused a decrease in particle size and an increase in polydispersity. However, uniformity was not adversely affected when DMEM was used to dilute ANFC, and even prevented particle coalescence.

ANFC particle washing with PBS was somewhat successful, however some particles adhered to the nylon mesh used for filtration and remaining particles were not robust enough to survive several rounds of washing. Therefore calcium-ion induced crosslinking of ANFC particles was attempted by several methods, however this was unsuccessful in increasing particle robustness. As revealed by calcofluor white staining, this was due to the entrapment of the cellulose fibres in ANFC below the membrane. It is hypothesised that the DMEM used to dilute ANFC causes some crosslinking and therefore prevents extrusion of ANFC through the membrane pores. This was not a problem in NFC particles, as calcofluor white staining confirmed the presence and uniform distribution of cellulose fibres in these particles.

ANFC particles were successfully crosslinked by adding calcium chloride to the emulsion and stirring. In more detail, 2 M CaCl$_2$ was added to the emulsion immediately after emulsification at a final concentration of 10 vol/vol% and the emulsion was then stirred with an overhead stirrer at 440 rpm for 15 minutes. Washing could then be carried out as the particles were robust. Particles can be washed by filtering through a 40 $\mu$m pore size cell strainer and resuspending in aqueous solution, then centrifuging. Particles can be centrifuged without loss of particle integrity or shape and without clumping.

ANFC particles were produced for use as microcarriers and for cell encapsulation to a high degree of uniformity (23% CV for the former and 26% CV for the latter, after crosslinking and washing). However even after washing, there was a considerable amount of debris in the microcarrier suspension, which could be eliminated by washing with a surfactant. Cells seeded onto the microcarriers in static culture attached in cell aggregates rather than in a single cell layer over the surface area of the microcarriers and did not survive well over seven days. A preliminary experiment demonstrated that cells can be encapsulated within the ANFC microparticles.

*Methods*

- Stirred cell membrane emulsification using a dispersion cell for production of NFC/ANFC emulsions.
- Particle washing using filtration and centrifugation.
- Microcarrier inoculation and cell culture.
- PrestoBlue cell viability assay.

*Conclusions*

Optimized membrane emulsification process parameters and formulations were found for the emulsification of NFC and ANFC to produce uniform particles. ANFC is the better material for producing particles in this way due to its ability to be crosslinked, increasing particle robustness and allowing downstream processing. Crosslinking, washing and membrane emulsification process parameters were optimised for the production of uniform ANFC particles for applications as microcarriers and for cell encapsulation.

*Research questions background*

Hypothermic storage of therapeutically relevant cells such as mesenchymal stem cells is a promising method of short-term storage for transport of cellular therapy products from the site of manufacture to the clinic. Storage of cells in NFC has been attempted previously, however it is necessary to reduce mass-transfer limitations in order to successfully release the cells post-storage, therefore encapsulation in microparticles is a solution. Membrane emulsification is a simple and scalable method of producing uniform water-in-oil emulsions and can therefore be used to make particles of NFC in a suitable oil phase. Membrane emulsification can be used for cell encapsulation since it has low mechanical stress compared to other emulsification techniques. The particle size can be controlled by varying the process parameters, therefore can be tailored to different cell types.

*Results*

*Membrane emulsification of alginate*

For preliminary experiments, sodium alginate hydrogel was used as the dispersed phase to investigate the effect of rotation speed on average particle diameter ($D_{av}$) and size variation (coefficient of variation; CV). Sodium alginate powder was dissolved in distilled water to make a 1% solution. 5 wt.% PGPR in Miglyol 840 was used as the continuous phase. The dispersed phase was injected at a rate of 1 ml/min and the stirrer rotation speed was varied from 390 to 1950 rpm. The membrane used was a full membrane with a 15 $\mu$m pore diameter and 200 $\mu$m pore spacing. Figure 1 shows that increasing rotation speed causes a decrease in average particle diameter.

Particles produced with a rotation speed of 1350 rpm were the most uniform, with a CV of 26.5%. This rotation speed was therefore used in subsequent experiments with NFC.

*Membrane emulsification of NFC*

*Effect of NFC concentration and membrane type on microparticle formation*

For an initial test of emulsification of NFC, the hydrogel was diluted to 1% by weight with distilled water. The experiment was carried out using the same conditions as for the alginate experiments; continuous phase was 5 wt.% PGPR in Miglyol 840, the injection rate was 1 ml/min, the rotation speed was 1350 rpm and a full membrane was used as previously described. The emulsion was imaged immediately after emulsification had taken place. Representative images of particles produced are shown in Figure 2.

The average particle diameter was 67.9 $\mu$m and the CV was 42.4%. Particles were observed to leave trails of debris when moving through the continuous phase and micelles can be observed on the outsides of particles, leading to the hypothesis that the particles were expelling water and other matter, and potentially solidifying. These particles were also observed to rapidly coalesce, as seen in the right panel of Figure 2. Overall, these particles do not appear to be stable. The concentration of NFC was subsequently reduced to assess the effect of this on particle coalescence and trail formation.

NFC was therefore diluted to 0.5% with distilled water and all other parameters were kept the same. The emulsion was imaged immediately after emulsification and again after 30 minutes of standing to assess particle behaviour over time. Representative images of particles produced immediately after emulsification (Figure 3A) and after standing for 30 minutes (Figure 3B) are shown below.

Immediately after emulsification, $D_{av}$ was 57.1 $\mu$m and the CV was 35.4%. After 30 minutes of standing, $D_{av}$ reduced to 47.2 $\mu$m and the CV also reduced to 26.8%. This was due to an observed shrinking and shrivelled appearance of the particles after 30 minutes (Figure 3B), which could also suggest particle solidification. Although fewer trails were seen in the emulsion, micelles still formed on particle surfaces (Figure 3A) and coalescence was still a problem (not shown).

A further experiment was carried out using these conditions (0.5% NFC, continuous phase of 5 wt.% PGPR in Miglyol 840, injection rate of 1 ml/min, rotation speed of 1350 rpm) and a ring membrane of 15 $\mu$m pore diameter and

200 $\mu$m pore spacing. Representative images of the particles immediately after emulsification are shown (Figure 4A), then continuous stirring was employed to agitate the particles for 15 minutes after emulsification to prevent coalescence (Figure 4B), and this was compared to unstirred emulsion (Figure 4C).

$D_{av}$ of particles immediately after emulsification was 57.9 $\mu$m and the CV was 35.8%. The particles did not leave trails in the continuous phase (Figure 4B). After stirring for 15 minutes, particles were observed to have coalesced and therefore accurate size analysis was not possible due to the shape of the particles (see figure 4B). Emulsion that was left to stand for 15 minutes formed large clumps of NFC with a shrivelled appearance (Figure 4C). This demonstrates that stirring may be a good way of maintaining intact particles after emulsification, but coalescence still occurs and that an additional strategy is needed to combat coalescence. Therefore, different continuous phases were tested in subsequent experiments.

*Effect of continuous phase formulation*

Two oil phases (Miglyol 840 and kerosene) and three surfactants (PGPR, Span 80 and ABIL EM 90) were trialled in different combinations as the continuous phase; 5 wt.% PGPR in Miglyol 840, 5 wt.% PGPR in kerosene, 2 wt.% Span 80 in kerosene, 5 wt.% Span 80 in kerosene and 2 vol.% ABIL EM 90 in kerosene. Particle size (Figure 5A) and uniformity (Figure 5B) was monitored over time in the emulsions that were continuously stirred or left unstirred in order to determine particle stability. A 15 $\mu$m pore diameter ring membrane, 1350 rpm rotation speed and 1 ml/min injection rate were used for the emulsification procedure.

Firstly, the oil of the continuous phase was changed from Miglyol 840 to kerosene, with PGPR being kept as the surfactant. The emulsion was imaged immediately after emulsification (Figure 6A) then continuously stirred and photographed after 15 minutes (Figure 6B), 30 minutes (Figure 6C) and 60 minutes (Figure 6D) or left to stand and photographed after 15 (Figure 6E) and 60 minutes (Figure 6F). Average particle diameters and CV for each time point are shown in Figure 5.

Particles were of a higher uniformity (27.0%) immediately after emulsification (Figure 5B) than in previous experiments, suggesting that kerosene is a superior oil phase to Miglyol 840 in terms of producing uniform emulsions. Average particle size was also increased (Figure 5A). Continuous stirring after emulsification helped to maintain particle size and uniformity over 60 minutes, whilst the emulsion that was left to stand showed an increase in variation (Figure 5B). This was perhaps due to particles breaking up over time, as the $D_{av}$ for this emulsion also decreased over 60 minutes. Rings around the particles were observed after 15 minutes of stirring (Figure 6B) and developed to be more pronounced after 30 (Figure 6C) and 60 (Figure 6D) minutes. This may be solidification of the particles that is accelerated by stirring of the emulsion.

As kerosene is a promising oil phase for the emulsification of NFC, different surfactants were subsequently trialled with kerosene to try to reduce particle variability. Therefore 2% Span 80 was used as the surfactant for the next experiment, keeping all other variables the same. Images were taken immediately after emulsification (Figure 7A), then continuously stirred and imaged after 15 minutes (Figure 7B), 30 minutes (Figure 7C) and 60 minutes (Figure 7D) or left to stand and imaged after 15 minutes (Figure 7E). Average particle diameters and CV are shown in Figure 5.

The particles immediately after emulsification had high uniformity (CV 24.2%; Figure 5B) and a slightly lower average diameter (89.6 $\mu$m) (Figure 5A) than particles made with PGPR in kerosene. However, particles were observed to coalesce rapidly (not shown). Also, stirring reduced the average particle diameter over time and did not prevent further coalescence, as can be seen in Figure 7C, with a large clump of hydrogel forming after 60 minutes of stirring (Figure 7D). These data suggest that the type of surfactant has an effect on particle size, uniformity and propensity for coalescence.

As the particles were prone to coalescence, an increase in surfactant concentration was trialled. The continuous phase was kerosene with 5% Span 80 and all other variables were kept the same (Figure 8).

These particles had an average diameter of 71.9 $\mu$m and a CV of 27.0% (Figure 5). Unfortunately, this emulsion had a lot of debris in the continuous phase and particles surfaces were not smooth. Micelles could also be observed coming from the surface of the particles (Figure 8). Therefore, this strategy of increasing surfactant concentration was not pursued further.

Next, ABIL EM 90 was trialled as a surfactant. The continuous phase was 2 vol% ABIL EM 90 in kerosene and all other variables were kept the same. The emulsion was imaged immediately after emulsification (Figure 9A), then continuously stirred and imaged after 15 (Figure 9B), 30 (Figure 9C), 60 (Figure 9D) and 150 minutes (Figure 9E) or left to stand and imaged after 15 (Figure 9F), 30 (Figure 9G), 60 (Figure 9H) and 150 minutes (Figure 9I). Average particle diameters and CV are shown in Figure 5.

The rotation speed of the dispersion cell stirrer and therefore shear stress was varied in order to describe the relationship between particle size and shear stress (Figure 9J). As expected, increase in shear stress causes a decrease in particle size. Using mathematical modelling, this relationship can be extrapolated to predict particle size.

Particle uniformity was good (21.9%; figure 5B) immediately after emulsification and average diameter was higher than previously, at 115.0 $\mu$m (Figure 5A). Over 150 minutes, stirring helped to maintain uniformity to a CV of under 30% and maintained average diameter within a margin of 6 $\mu$m. The unstirred emulsion did not have a constant CV or $D_{av}$ over time and after 150 minutes the particles were very polydisperse and had either broken up or coalesced considerably (Figure 9I). Although coalescence did occur in both the unstirred and stirred emulsions over time, the remaining particles in the stirred emulsion were the most uniform of any emulsion yet produced. Therefore, kerosene and 2% ABIL EM 90 is a promising continuous phase to employ for further refinement of particle uniformity.

Experimental determination of the relationship between shear stress and particle size shows a slight trend of decreasing average particle diameter with increasing shear stress, caused by an increase in rotation speed. For mathematical modelling, the shear ($\tau$) is determined by:

$$\tau = 0.825 \mu \omega \, r_{trans} \frac{1}{\delta}$$

where $\mu$ = continuous phase viscosity (Pa s$^{-1}$)

$\omega$ = rotation speed (rpm)

$$r_{trans} = \frac{D}{2} 1.23 \left(0.57 + 0.35\frac{D}{T}\right)\left(\frac{b}{T}\right)^{0.036} n_b^{0.116} \frac{Re}{1000 + 1.43Re}$$

where D = stirrer width (m)

T = tank width (m)
b = blade height (m)
$n_b$ = number of blades
Re = $(p\omega D^2)/(2\pi\mu)$

where p = continuous phase density (kg m$^{-3}$)

$$\delta = \sqrt{\frac{\mu}{p\omega}}$$

Particle, or droplet, diameter $x$ is determined by:

$$x = \frac{18\tau^2 r_p^2 + 2\sqrt{81\tau^4 r_p^4 + 4r_p^2 \tau^2 \gamma^2}}{3\tau}$$

where $r_p$ = membrane pore radius (m)
$\gamma$ = interfacial tension (N/m)

However, this mathematical modelling does not take into account the injection rate, which can have an influence on particle size. Therefore, different injection rates would need to be investigated in order for the mathematical model to better fit the experimental data.

*Higher concentrations of NFC*

To produce firmer particles and prevent coalescence, emulsification with 1.5% NFC diluted with water as the dispersed phase was investigated. The continuous phase was 2 vol% ABIL EM 90 in kerosene and all other variables were kept the same. The emulsion was imaged immediately after emulsification (Figure 10A) then continuously stirred and imaged after 15 (Figure 10B), 30 (Figure 10C) and 60 (Figure 10D) minutes or left to stand and imaged after 15 (Figure 10E), 30 (Figure 10F) and 60 (Figure 10G) minutes. Average particle diameter and CV of the unstirred emulsion are shown in Figures 10H and 101 respectively.

Immediately after emulsification, particles were seen to clump together by eye, but some small particles remained intact (Figure 10A). Upon continuous stirring, particles clumped further and after 30 minutes, hardly any individual particles remained intact (Figure 10C). Conversely, emulsion that was left to stand maintained intact individual particles after 15 (Figure 10E), 30 (Figure 10F) and 60 (Figure 10G) minutes, although some clumping of NFC is seen at this time point. Average particle size (Figure 10H) was reduced compared to previous emulsions due to an increase in NFC viscosity, however variability in particle size was high, with a CV of over 50% immediately after emulsification (Figure 10I). Therefore, in order to create firmer particles of higher concentration NFC, conditions need to be optimised for greater uniformity.

Firstly, undiluted NFC straight from the syringe at concentrations of 1.5% and 2.5% was tried. These NFC particles could be observed clumping together immediately after production, and large clumps could be viewed under the microscope in both cases (Figure 11A). Average particle diameter (Figure 11B) is considerably smaller than 0.5% NFC particles (Figure 5A) due to the increase in viscosity. The emulsions were either stirred or left to settle for 60 minutes. Stirring the emulsions caused further clumping of the NFC such that after 60 minutes, very few NFC particles remained intact in both the 1.5% and 2.5% NFC emulsions, as most of the material had clumped together (Figure 11A). However, less clumping was observed in the unstirred emulsions and particles with a CV of 33.3% and 40.5% were observed in the 1.5 and 2.5% NFC emulsions respectively after 60 minutes (Figure 11C). Size and size distribution data for stirred 1.5% NFC emulsion could not be obtained due to insufficient number of particles.

2.5% NFC particles were very slightly more robust than particles of lower concentrations, however this was not enough to warrant further investigation of particle production at this concentration.

NFC was then diluted with basal medium (DMEM) to 2.0% to determine the effect of addition of culture medium to particle formation. Contrary to undiluted NFC, these particles did not clump together during emulsification or during stirring, however they were prone to coalescence after 60 minutes of stirring (Figure 12A). Average particle size ($D_{av}$) in the stirred sample varied widely over 60 minutes, presumably due to coalescence, whilst particle size in the unstirred sample remained fairly consistent (Figure 12B). However, particles that were left unstirred tended to break up after 60 minutes, resulting in large size variability in both samples; 68.2% and 61.0% CV for stirred and unstirred sample respectively (Figure 12C).

These data suggest that whilst higher concentrations of NFC taken straight from the syringe are unsuitable for particle production due to high levels of clumping (Figure 11A), dilution of NFC with DMEM prevents this problem. However, these particles have high size variability due to coalescence and break up therefore experimental parameters would need to be optimised in order to produce uniform particles of higher concentration NFC particles. When determining particle robustness by applying pressure to a coverslip placed over a sample of emulsion on a microscope slide, particles of 2% NFC were not noticeably more robust than lower concentration particles produced previously. Therefore, increasing the concentration of NFC may not be suitable for producing particles that are able to withstand downstream processing. Subsequent experiments therefore focused on lower concentration 0.5% NFC particles that were shown to have good uniformity (Figure 5B), and a suitable washing procedure, as well as ANFC as the dispersed phase.

*Effect of cell culture medium as a diluent on particle formation*

Dilution of NFC to 0.5% with either complete medium (DMEM, 10% FBS, 1% Ultraglutamine) or basal medium (DMEM only) was investigated to elucidate any effects on particle size and formation. Figures 13A and 13B show that the size of particles with basal and complete medium remain consistent over 60 minutes of stirring following emulsification, although coalescence did occur without stirring (data not shown). When compared to 0.5% NFC particles diluted with water and emulsified under the same conditions (2 vol.% ABIL EM 90 in kerosene continuous phase, 15 μm pore diameter ring membrane, 1350 rpm rotation speed, 1 ml/min injection rate; figure 9), particles diluted with medium were smaller (Figure 13B) and more polydisperse (Figure 13C), but there was little difference between medium types. Smaller particle size can be attributed to the increased ionic strength in cell culture media compared to distilled water. Particle size may be easily altered by differing the rotation speed of the stirrer to produce particles of a suitable size for cell encapsulation, therefore the decrease in particle size upon addition of medium does not pose a problem. Size variation, however, will need to be decreased if these conditions are selected for optimal microparticle production.

Particles with complete medium were slightly more robust than particles with basal medium or water. For future experiments, however, it was decided that NFC would be diluted with basal medium, as the proteins contained within the complete medium are surface active and may disrupt particles upon washing.

Washing of particles was first attempted by simply removing excess continuous phase, adding washing solution and gently agitating the particles by stirring or swirling manually. 0.5% NFC particles diluted with basal medium were washed in this way using either PBS or 2% Tween 20 in water.

Although particles appear to have survived washing in all conditions, there are some cases in which flattened, pink-tinged (owing to the presence of phenol red in basal medium) particles can be observed (indicated with black arrows, Figure 14). This casts doubt over the identity of the transparent particles observed, as nanocellulose fibres could not be observed in any particles under the microscope. Therefore, a different washing procedure was adopted in future experiments in order to capture NFC particles prior to addition of washing solution (see Figure 16).

*Membrane emulsification of ANFC*

*Effect of medium on ANFC particle formation*

ANFC particles were produced, either diluted with water or basal medium to a concentration of 0.5% by weight, and stirred over a period of 60 minutes. Coalescence was observed in particles diluted with water (see top left panel, Figure 15A) whereas this was not the case in particles diluted with basal medium. Similarly, to NFC (Figure 13B), addition of medium caused particle size to decrease (Figure 15B). Size uniformity was maintained over 60 minutes in both samples (Figure 15C) with a very good uniformity of particles diluted with basal medium immediately after emulsification (CV = 25.2%).

Immediately following emulsification, 0.5% ANFC particles with basal medium were filtered using a cell strainer (Figure 16Ai) to remove the continuous phase, and washing solutions (either water, basal medium or PBS) were added to resuspend the particles. However, particles were very prone to adhering to the cell strainer mesh (Figure 16Aii) and only the use of PBS was successful in recovering some particles from the strainer (Figure 16B).

Particles recovered from the strainer (Figure 16B) had an average diameter of 64.8 $\mu$m and CV of 25.1%. This suggests that washing with PBS causes particles to shrink, as average dimeter prior to washing is 78.8 $\mu$m (Figure 15B). Particle size variation, however, remained constant after washing. These data therefore suggest that a filtration system for washing of NFC/ANFC particles with PBS is useful in maintaining particle integrity whilst removing the continuous phase from the system.

In order to increase particle robustness, a higher concentration of ANFC was provided. 0.7% ANFC diluted with basal medium was used for particle production, then washed using PBS and a cell strainer as above and left in PBS overnight to assess the longer-term stability of particles in an aqueous environment. These particles were prone to coalescence immediately after emulsification and had an average diameter of 88.7 $\mu$m and CV of 43.3% (Figure 17C). Upon leaving overnight in PBS (Figure 17B), however, the particles were much more robust and bounced back when pressure was applied but were not resistant to acetone, indicating that whilst more stable, these particles had not solidified.

Whilst a promising avenue for increasing particle robustness, the 0.7% ANFC particles were not acetone resistant and therefore had not solidified. Furthermore, a concentration of 0.5% would be more suitable for cell culture applications, therefore crosslinking of 0.5% ANFC particles was explored.

*Crosslinking of ANFC particles*

At first, $CaCl_2$ at a final concentration of 0.05 M in 0.5% ANFC was added at the point of dilution, however this caused crosslinking too rapidly as the materials could not be mixed to homogeneity. Next, 0.5% ANFC particles were produced and washed with PBS before 0.1 M $CaCl_2$ was added dropwise to the particles on a microscope slide. No change in particle robustness was observed using this method. $CaCl_2$ was then added to PBS at a concentration of 0.1 M and this solution was used to wash 0.5% ANFC particles. Whilst this method had some success in increasing particle robustness, $CaCl_2$ solubility in PBS was an issue as it appeared to precipitate out of solution. Likewise, adding $CaCl_2$ to DMEM for use as a washing solution resulted in precipitation. This precipitation is perhaps due to dicalcium phosphate formation.

A number of crosslinking methods using a 1 M $CaCl_2$ solution were subsequently trialed. This included simple addition of $CaCl_2$ to the final emulsion, as well as a sedimentation method involving the ANFC particles in the continuous phase poured on top of the $CaCl_2$ solution and left to sediment into the aqueous phase. Another crosslinking technique was homogenization of the $CaCl_2$ solution in the continuous phase and stirring the resulting emulsion with the ANFC emulsion to allow the diffusion of $CaCl_2$ into the ANFC particles. Unfortunately, none of these techniques were successful in crosslinking the particles as no increase in robustness was observed.

*Calcofluor white*

*Calcofluor white staining and process improvement*

In order to understand the composition of NFC and ANFC particles and to understand the lack of crosslinking, calcofluor white staining was employed to visualise the cellulose fibres in the particles. 1% calcofluor white was added to the 0.5% NFC or ANFC solution before emulsification and staining was visualised using UV microscopy (Figure 18).

0.5% NFC particles stained well with calcofluor white (Figure 18A), with staining equally distributed throughout the particles and the fibrous structure able to be identified at higher magnification (Figure 18Aiii). However, when 0.5% ANFC particles diluted with DMEM were stained (Figure 18B), no particle fluorescence was observed under UV light (Figure 18Bii). Residual ANFC that was trapped underneath the membrane in the dispersion cell did fluoresce however (Figure 18Biii). This suggests that the ANFC cellulose fibres are not being pushed through the membrane pores and are getting caught behind the membrane. In order to remedy this, the experiment was repeated with a membrane of larger pore diameter (40 $\mu$m) and increased injection rate (5 ml/min; Figure 18C) to force the ANFC fibres through the membrane. This approach was only partly successful, as some particles did fluoresce (Figure 18Cii), however stain distribution was uneven and some particles were misshapen. Moreover, ANFC fibres were still caught behind the membrane (Figure 18Ciii). It was hypothesised that ANFC was surprisingly crosslinking to some degree when mixed with DMEM prior to emulsification, preventing movement through the membrane pores. ANFC was consequently diluted with water to 0.5% and the previous experiment repeated with 40 $\mu$m membrane pore size and 5 ml/min injection rate (Figure 18D). Particles fluoresced under UV (Figure 18Dii) and residual ANFC did not strongly fluoresce (Figure 18Diii). These data suggest that dilution of ANFC with DMEM is causing crosslinking and preventing effective extrusion of the material through the membrane. This issue therefore explains the lack of particle crosslinking induced by $CaCl_2$ through various methods described in previous.

Production of successful ANFC particles

The process was subsequently further improved by the addition of a homogenisation step before emulsification. Homogenisation of ANFC with its diluent proved to be successful, as uniform ANFC particles with an even cellulose fibre distribution were produced using PBS (Figure 19a) and DMEM (Figure 19b) as the diluent. Several methods of particle crosslinking were then trialled, including filtering particles using a cell strainer then adding $CaCl_2$ solution (figures 19c(i) and c(ii)), homogenising $CaCl_2$ solution with 2% ABIL EM 90 in kerosene and adding this to the emulsion (Figure 19c(iii)), and finally adding $CaCl_2$ to the emulsion and stirring for 15 minutes (Figure 19c(iv)). Detected $D_{av}$ ($\mu$m) and CV (%) are presented in Table 1.

Table 1

|  | PBS | DMEM |
| --- | --- | --- |
| $D_{av}$ ($\mu$m) | 220 | 168 |
| CV (%) | 26.4 | 26.8 |

Homogenisation of ANFC and its diluent is essential for producing particles of ANFC that have an even cellulose distribution and spherical shape. This was achieved using PBS (Figure 19a) and DMEM (Figure 19b) as the diluent. Since ANFC particles can now be produced in an emulsion successfully, different methods for crosslinking of the particles were subsequently tested. Calcium chloride solution added after filtration of the particles in a cell strainer did succeed in crosslinking the particles, however these particles tended to stick together (Figure 19ci), particularly when DMEM was used as the diluent for ANFC (Figure 19cii). A different approach was to produce small particles of calcium chloride by homogenising with the continuous phase, 2% ABIL EM 90 in kerosene, and stirring these calcium chloride particles with the ANFC emulsion. Unfortunately, this caused the ANFC particles to clump and stick together (Figure 19ciii). The successful method of crosslinking was to add 2M calcium chloride into the ANFC emulsion (10% final concentration) and stir this for 15 minutes. The resulting ANFC particles retained their shape, were separate, robust and acetone resistant (Figure 19civ).

*ANFC particles for cell culture applications*

Following from the successful crosslinking procedure, ANFC particles were produced for use as microcarriers (Figure 20a) or particles for cell encapsulation (Figure 20b) and their size and size distribution was analysed (Table 2).
Particles for use as microcarriers were produced using a 15 $\mu$m pore size membrane, exemplifying that homogenisation of the ANFC prior to emulsification is essential for producing particles and allows smaller pore sizes to be used. There particles were very uniform, with a CV of 16%. Particles were crosslinked as previously described (Figure 19civ) with 1 M calcium chloride for 8 minutes, then resuspended in PBS, centrifuged, and resuspended again in PBS. This was repeated twice further to wash the particles. The resulting crosslinked and washed particles (Figure 20aiii) remained robust, were slightly reduced in size and slightly decreased in uniformity (Table 2), but this was still very acceptable.
Particles for cell encapsulation applications were produced using the larger 40 $\mu$m full membrane in order for cells to be able to pass through the membrane pores. Full membranes will generally produce less uniform emulsions than ring membranes, therefore the decrease in uniformity compared to the microcarrier particles was expected (Table 2). Particles were crosslinked with 1 M calcium chloride for 12 minutes and washed as above. Again, the crosslinked and washed particles were slightly reduced in size and uniformity (Table 2) but remained robust and spherical (Figure 20biii).

Table 2.

| | Microcarriers | | Encapsulation particles | |
|---|---|---|---|---|
| | In emulsion | After crosslinking and washing | In emulsion | After crosslinking and washing |
| $D_{av}(\mu m)$ | 133 | 104 | 176 | 165 |
| CV (%) | 16 | 23 | 23 | 26 |

*Microcarriers*

ANFC and commercially available Solohill plastic microcarriers were sterilised by autoclaving and the ANFC microcarriers were then filtered to remove excess debris (Figure 21a). ANFC (Figure 21b) and plastic (Figure 21c) microcarriers were seeded with human Mesenchymal Stem Cells (hMSC) at a density of 5000 cells cm$^{-2}$. Both types of microcarriers were observed at day 1 without cells (Figure 21 bi and ci), then with cells at day 3 (Figure 21bii and cii) and day 7 (Figure 21biii and ciii) after seeding. Cell viability at each of these intervals was measured using the PrestoBlue resazurin-based colourimetric assay.

ANFC microcarriers were filtered due to the presence of a considerable amount of debris in the solution. This is perhaps some oily residues from the continuous phase that have not been adequately washed away. Filtration did slightly reduce the amount of debris, but some remained, therefore an improved microcarrier washing technique is required. ANFC and plastic microcarriers were inoculated with hMSC cells in a static well plate and observed over seven days. By day three, hMSC cells can be observed attaching to the plastic microcarriers and forming bridges between beads, causing their aggregation (Figure 21 cii) and is even more evident after seven days (Figure 21 ciii). In the ANFC microcarriers, cells can be observed attaching to the surface of some microcarriers and forming cell aggregates at day three (Figure 21bii) and day seven (Figure 21 biii). However, cells do not appear to attach evenly across the surface of the microcarriers and even form aggregates in the medium, perhaps around some debris in the system. Cell viability (Figure 21d) increased up to day three in the plastic microcarrier system and decreased at day seven, presumably due to the aggregation in the static system. In the ANFC microcarrier system, cell viability decreased over seven days. These data suggest that further work is needed to optimise the ANFC microcarriers to be suitable to support cell growth.

*Improved microcarrier washing*

As mentioned previously, an improved particle washing procedure is needed for eliminating oily residue left after filtering particles with a cell strainer and washing with PBS. Therefore, washing with Tween 20 in PBS after filtration was tried (Figure 22).

The resulting washed particles were cleaner and easier to identify under visible light (Figure 22a) with the absence of debris and retained their robustness and shape (Figure 22b). Therefore, it is recommended that the particles are first filtered to remove excess continuous phase, then washed with a solution containing 2% Tween 20 or another surfactant to remove oily residues.

*Cell encapsulation in ANFC particles*

Encapsulation of hMSCs inside the ANFC particles was trialled, using the parameters for particle production as those in figure 20b. A cell suspension was added to the homogenised 0.5% ANFC/DMEM gel to a cell density of 300,000 cells ml$^{-2}$ and this was used as the dispersed phase in the membrane emulsification process.

This shows that hMSCs can be successfully encapsulated within ANFC microparticles using the membrane emulsification process (Figure 23). However, it is important to note that the ANFC must be homogenised before the addition of cells, and that the cell suspension added must be of a small volume to not disrupt the structure of the homogenised ANFC. Mixing the cell suspension into the ANFC using a micropipette may not achieve a homogeneous gel, which can affect the success of the membrane emulsification process.

Materials and methods

*Used methods*

- Stirred cell membrane emulsification using a dispersion cell for production of NFC/ANFC emulsions.
- Particle washing using filtration and centrifugation.
- Microcarrier inoculation and cell culture.
- PrestoBlue cell viability assay.

*Protocol*

*Membrane emulsification*

A diagram of the dispersion cell used for membrane emulsification in shown below in figure 24. Stirred cell membrane emulsification is used to produce emulsions in batches. In this setup, the dispersed phase is pumped through a membrane with a regular array of pores into the continuous phase. As the dispersed phase is pushed through the pores, droplets form. Constant stirring of the continuous phase exerts shear at the surface of the membrane, causing droplets to detach from the membrane and be suspended in the continuous phase.

A detailed protocol is outlined in the following:
Three different membranes were used for the production of hydrogel microparticles. All were nickel membranes. For alginate microparticles, a full membrane of 15 $\mu$m pore diameter and 200 $\mu$m pore spacing was used. For NFC microparticles, a ring membrane with 15 $\mu$m pore diameter and 200 $\mu$m pore spacing was used. This membrane was also used to produce ANFC microparticles, as well as a full membrane of 40 $\mu$m pore diameter and 200 $\mu$m pore spacing for producing ANFC microparticles for cell encapsulation.

Membrane cleaning is carried out by acid and alkali washes and sonication in an ultrasonic bath. The membrane is covered with 2 M NaOH and sonicated for one minute, then left to soak for five minutes. Following this, the membrane is rinsed with tap water and then sonicated again for one to five minutes in distilled water. The membrane is then transferred to 2% citric acid and further sonicated for one minute and left to soak for five minutes. The membrane is then rinsed again with tap water, sonicated for one to five minutes in distilled water, then dried with acetone and compressed air.

The continuous phase is made by combining surfactant (PGPR, Span 80 0r ABIL EM 90) and oil phase (Miglyol 840 or kerosene) and stirring with a magnetic stirrer for at least one hour. The membrane is then soaked in the continuous phase for at least 30 minutes in order to avoid membrane wetting. The dispersed phase is made by diluting NFC or ANFC with distilled water, DMEM, hMSC complete medium or PBS or by dissolving sodium alginate in distilled water and stirring with a magnetic stirrer until homogeneous, or homogenising at 6500 rpm for 10 minutes. The dispersed phase is then drawn into a 25 ml syringe and mounted onto a syringe pump.

To set up the dispersion cell, PTFE tubing with a three-way valve is connected to the base of the dispersion cell. The syringe containing the dispersed phase is then connected to the valve and an empty waste syringe is also connected to the valve. 25 ml of continuous phase is poured into the base of the dispersion cell to fill it. Continuous phase is then drawn through the PTFE tubing until it reaches the waste syringe. The valve is then shut to the tubing and dispersed phased is then pumped through the valve until it also reaches the waste syringe. This ensures that the two liquid phases meet at the valve without air bubbles between them. The membrane is then placed into the base of the dispersion cell and secured using a PTFE ring stopper. The glass cylinder is screwed on the base of the dispersion cell and up to 100 ml of continuous phase is poured into this.

The dual-bladed overhead stirrer is then attached to a 24 V DC motor and calibrated using a tachometer. The stirrer is then attached to the top of the dispersion cell. The motor is switched on to start stirring at 1350 rpm and the syringe pump is also switched on to begin pumping of the dispersed phase through the tubing, into the base of the dispersion cell and through the membrane, at an injection rate of 1-10 ml min$^{-1}$.

The continuous phase is observed until the first few droplets of dispersed phase appear. The system is then left running for sufficient time to produce a final concentration of the dispersed phase in the emulsion of 2%. The emulsion is then transferred into a beaker and a sample is taken with a plastic Pasteur pipette for analysis using a light microscope.

*Particle size analysis*

A sample of particles (minimum 300 particles) is used for size analysis. Average particle diameter ($D_{av}$) was determined using a MATLAB R2019b program. Any particles that were too large to be detected by the MATLAB program were manually sized using ImageJ. Coefficient of variation (CV) was calculated by dividing the standard deviation by the mean and multiplying by 100.

*Particle stability over time*

Emulsions were either continuously stirred using an overhead stirrer or left to settle in the beaker for up to 150 minutes, and samples are taken for analysis as above at regular time intervals.

Particle washing

NFC particles in section "Effect of cell culture medium as a diluent on particle formation" were washed with a simple washing procedure. This consisted of leaving particles to settle at the bottom of the emulsion, then removing excess continuous phase and adding washing solution. Washing solutions used were 2 vol.% Tween 20 in distilled water or Phosphate Buffered Saline without calcium and magnesium (PBS). Particles in washing solution were agitated by gently stirring or manually swirling. Particles were then analysed by microscopy.

The filtration washing procedure in section *"Effect of medium on ANFC particle formation"* was carried out by pipetting the emulsion onto a nylon mesh cell strainer with 40 $\mu$m diameter pore size and letting the continuous phase drip through. The particles captured on the strainer were then washed over with washing solution to remove remaining continuous phase and then immersed in washing solution and photographed immediately or left in the washing solution for 24 hours. Washing solutions used were distilled water, basal medium or PBS.

Crosslinked particle washing in section "ANFC particles for cell culture applications" was carried out by filtering particles in a cell strainer (see figure 25 for schematic) resuspending in PBS and centrifuging at 300 x g for 5 minutes. Resuspension and centrifugation were repeated twice further, and particles were finally resuspended in PBS for autoclaving (microcarriers) or DMEM (particles for encapsulation).

Improved particle washing in section "Improved microcarrier washing" was carried out by filtering particles in a cell strainer, washing through with 2% Tween 20 in PBS, resuspending in PBS and filtering again through a cell strainer, washing through with 2% Tween 20 in PBS once more and resuspending in PBS.

*Estimation of particle robustness*

Particle robustness was tested by adding a coverslip on top of particles on a microscope slide and applying gentle pressure. Particle resistance to acetone was also used to determine robustness by adding a drop of acetone to particles on a microscope and observing particle behaviour.

*ANFC particle crosslinking*

Calcium ion induced crosslinking of ANFC particles described in previous. was tried using several methods. $CaCl_2$ at a final concentration of 0.05 M was added to the dispersed phase at the point of dilution. 0.1 M $CaCl_2$ solution was added dropwise to PBS-washed ANFC particles on a microscope slide. $CaCl_2$ was also added to PBS at a final concentration of 0.1 M and this solution was used to wash ANFC particles. 1 M $CaCl_2$ was added to the emulsion and stirred for one hour. Sedimentation of ANFC particles was performed by pouring the emulsion on top of an aqueous phase of 1 M $CaCl_2$ and allowing the particles in the oil phase to fall into the aqueous phase below. Homogenisation was performed by homogenising 5 vol.% 1 M $CaCl_2$ in the continuous phase (2 vol.% ABIL EM 90 in kerosene) for 90 seconds at 5000 rpm and adding this emulsion to an emulsion of 0.5% ANFC in the same continuous phase in a 1:1 ratio and stirring for with an overhead stirrer for two hours. Particle robustness was tested as described above.

Crosslinking was carried out using several methods. The emulsion was filtered using a 40 $\mu$m pore size cell strainer and resuspended in 1 M $CaCl_2$. Homogenisation of $CaCl_2$ in the continuous phase was carried out as described above. The successful method was adding 2 M $CaCl_2$ to the emulsion at a final concentration of 10 vol/vol% immediately after emulsification and stirring with an overhead stirrer at 440 rpm for 15 minutes before filtering in a cell strainer and resuspending in PBS (Figure 25).

*Calcofluor white staining*

Membrane emulsification was performed as above, with a final concentration of 1% Calcofluor white dye added to the dispersed phase and mixed with a magnetic stirrer until homogenous before emulsification. The stain was visualised under UV light.

*Cell culture*

Human bone marrow-derived Mesenchymal Stem Cells (hMSC) were cultured in hMSC complete medium (DMEM supplemented with 10% FBS and 1% Ultraglutamine) at 37°C, 5% $CO_2$, 95% humidity. Cell counting was achieved by trypsinising cells and using an automated cell counter.

*Microcarrier inoculation*

ANFC and SoloHill plastic microcarriers suspended in PBS were steam sterilized for 15 minutes at 121°C in an autoclave. ANFC microcarriers were then filtered using a Steriflip filter unit and resuspended in PBS. Both types of microcarrier were centrifuged at 200 x g for 5 minutes, resuspended once in fresh PBS, centrifuged again and resuspended in hMSC complete medium. Microcarriers in medium were added to an ultra-low attachment 6 well plate at 200,000 cm$^2$ ml$^{-1}$ and incubated for at least an hour in a cell culture incubator. A cell suspension was prepared and added to each well at a density of 5000 cells cm$^{-2}$. Culture medium was half-changed every 2-3 days.

PrestoBlue cell viability assay

1/10th volume of PrestoBlue reagent was added to each well, protected from light at incubated at 37°C for one hour. A sample of the medium was analysed in a microplate reader. Absorbance was measured at 570 nm, using 600 nm as a reference wavelength.

*Cell encapsulation*

A hMSC cell suspension was added to 0.5% ANFC diluted with DMEM that had been homogenised, at a final cell density of 300,000 cells ml$^{-1}$ and mixed by pipetting. This was used as the dispersed phase to create microparticles using membrane emulsification.

Materials

*Membrane emulsification*

- Dispersion cell with dual bladed paddle stirrer, glass cylinder and PTFE ring stopper (Micropore Technologies)
- Nickel membrane; full membrane with 15 $\mu$m pore diameter and 200 $\mu$m pore spacing, ring membrane with 15 $\mu$m pore diameter and 200 $\mu$m pore spacing or full membrane with 40 $\mu$m pore diameter and 200 $\mu$m pore spacing (Micropore Technologies)
- DC power supply for stirrer (INSTEK, PR3060)
- Syringe pump (World Precision Instrument Inc., AL-1000)
- PTFE tubing with three-way valve
- Sodium hydroxide (Fisher Scientific, A16037.36)
- Citric acid (Fisher Scientific, 10233470)
- NFC and ANFC from wood cellulose (UPM Biomedicals, 100103010 and 200103010)
- Sodium alginate (Health Leads, 0721)
- PGPR (Polyglycerol polyricinoleate; Abitec)
- Span 80 (Polysorbate 80; Sigma-Aldrich, S6760)
- ABIL EM 90 (Cetyl PEG/PPG-10/1 Dimethicone; Surfachem)
- Miglyol 840 (IOI Oleochemical, 50050)
- Kerosene, low odour (Sigma-Aldrich, 329460)
- Tachometer (Omega)
- Acetone (Sigma-Aldrich, 179124)
- Microscope (L2800FL LED GX microscope, GT Vision)
- Homogeniser (T18 digital ULTRA TURRAX, IKA, 0003720000)

*Particle washing*

- Nylon mesh cell strainer, 40 $\mu$m pore size (Falcon, 10737821)
- Tween 20 (Polysorbate 20; Sigma-Aldrich, P1379)

*ANFC crosslinking*

- Calcium chloride (Fisher Scientific, S25223 )

*Calcofluor white staining*

- Calcofluor white stain (Sigma-Aldrich, 18909)

*Cell Culture*

- Human Mesenchymal Stem cells, bone marrow derived (Lonza)
- Phosphate buffered saline without calcium and magnesium (PBS, Lonza, BE17-516F)
- Dulbecco's Modified Eagle Medium (DMEM) 1.0 g/l glucose w/o L-gln (Lonza, 12-707F)
- Fetal Bovine Serum (FBS; Sigma)
- Ultraglutamine (Lonza, BE17-605E/U1 )
- Nucleocounter NC-3000 (Chemometec)

*Microcarrier inoculation*

- SoloHill plastic microcarriers, 125-212 $\mu$m diameter (Pall)
- VX 95 Series Autoclave (Systec GmbH)
- Steriflip 60 $\mu$m pore size sterile centrifuge tube top filter unit (Merck Millipore, SCNY00060)
- Corning Costar Ultra-Low Attachment 6 Well Plate (Merck, CLS3471)

*PrestoBlue cell viability* assay

- PrestoBlue cell viability reagent (ThermoFisher Scientific, A13261)
- FLUOstar Omega microplate reader (BMG Labtech)

Table 3 presents some research questions and corresponding outcomes.

Table 3

| | Outcome |
|---|---|
| 1. Determine optimum process parameters and formulation for production of uniform NFC particles. | Process parameters and formulation of continuous phase for optimal uniformity of:<br>- 0.5% NFC emulsion determined.<br>- 1350 rpm rotation speed,<br>- 1 ml/min injection rate,<br>- Ring membrane of 15 $\mu$m pore size and 200 $\mu$m pore spacing<br>- 2 vol.% ABIL EM 90 in kerosene continuous phase used as standard in further experiments.<br>This is the best protocol for producing uniform NFC droplets. However, washing and further downstream processing was not possible as the droplets were not robust enough to survive the further procedures. |
| 2. Optimum process parameters and formulation for production of uniform ANFC particles. | - 0.5% ANFC, diluted with PBS or DMEM, homogenised at 6500 rpm for 10 minutes<br><br>- 1350 rpm rotation speed<br>- 5 ml/min injection<br>- Ring membrane of 15 $\mu$m pore size and 200 $\mu$m pore spacing or full membrane of 40 $\mu$m pore size and 200 $\mu$m pore spacing<br>- 2 vol.% ABIL EM 90 in kerosene continuous phase |

(continued)

| | Outcome |
|---|---|
| 3. Increase particle robustness | Higher concentration of NFC was not successful in producing more robust particles. Crosslinking of ANFC particles therefore explored as a method of increasing robustness. Process parameters altered to produce ANFC microparticles - increased injection rate and homogenisation of ANFC and diluent prior to emulsification. Crosslinking achieved by adding calcium chloride into the emulsion and stirring. |
| 4. Visualise cellulose fibres in particles | Staining successful in NFC particles and ANFC particles following process parameter optimisation. |
| 5. Washing of particles and resuspension in aqueous phase | Crosslinked particles can be filtered using a cell strainer and resuspended in an aqueous phase e.g. PBS or DMEM. Resuspended particles can be centrifuged multiple times without clumping or losing their integrity or shape. However, there is lots of debris left after this, which may be oily residue. Washing with 2% Tween 20 (in PBS) in aqueous phase can solve this problem. |
| 6. ANFC particles as microcarriers | ANFC particles can be autoclaved to sterilise without any detriment to shape or integrity. When used as microcarriers, hMSC cells tend to attach to the surface of the particle as aggregates rather than attaching in a single layer over the whole surface of the particle. Cells do not survive well over seven days. <br> Protocol for producing ANFC m icrocarriers: <br> 1. Dilute ANFC to 0.5% with $Ca^{2+}$ and $Mg^{2+}$-free PBS and homogenise at 6500 rpm for 10 minutes until smooth consistency <br> 2. Soak ring membrane of 15 $\mu$m pore size and 200 $\mu$m pore spacing in the continuous phase (2 vol.% ABIL EM 90 in kerosene) for at least 30 minutes <br> 3. Set up dispersion cell as described <br> 4. Homogenise with the following parameters: 5 ml/min injection rate, 1350 rpm rotation speed. <br> 5. Decant emulsion into a beaker, add a final concentration of 10 vol.% 2 M $CaCl_2$ solution to the emulsion and stir at 440 rpm for 15 minutes <br> 6. Filter through a 40 $\mu$m pore size cell strainer and wash through with 2 vol.% Tween 20 in PBS, resuspend in PBS <br> 7. Repeat step 6 <br> 8. Centrifuge at 300 x g for 5 minutes, remove supernatant and resuspend in PBS <br> 9. Repeat step 8 twice <br> 10.Autoclave for 15 minutes at 121°C <br> 11. Filter using a Steriflip filter unit <br> 12. Resuspend in PBS, centrifuge and resuspend in PBS twice, then resuspend in culture medium and incubate for one hour prior to cell inoculation |
| 7. ANFC particles for hMSC cell encapsulation | A preliminary experiment shows that membrane emulsfication can be used to encapsulate hMSC cells in ANFC particles. However further work is needed to investigate cell viability after washing and crosslinking of the particles and over time in culture. <br> Protocol for producing ANFC microcapsules for cell encapsulation: <br> 1. Dilute ANFC to 0.6% with basal culture medium e.g. DMEM and homogenise at 6500 rpm for 10 minutes until smooth consistency 2. Soak full membrane of 40 $\mu$m pore size and 200 $\mu$m pore spacing in the continuous phase (2 vol.% ABIL EM 90 in kerosene) for at least 30 minutes <br> 3. Prepare cell suspension and add to ANFC solution to get desired cell density and final ANFC concentration of 0.5% <br> 4. Set up dispersion cell as described <br> 5. Homogenise with the following parameters: 10 ml/min injection rate, 1350 rpm rotation speed. |

(continued)

| | Outcome |
|---|---|
| | 6. Decant into a beaker, analyse particles under microscope and incubate |

The following was found out in the experiments (tips and tricks):

- Homogenisation of ANFC with its diluent is essential before emulsification. Homogenise until no 'lumps' can be seen and the surface of the gel is smooth upon stirring it. The gel must be smooth and free flowing.
- When transferring a sample of the emulsion to a microscope slide, the plastic transfer pipette may cause coalescence of the NFC/ANFC particles on the microscope slide. It is recommended to aspirate and eject some of the emulsion a few times before taking a sample to image.
- When stirring the emulsion with aqueous $CaCl_2$ solution to crosslink the ANFC particles, some of the particles will aggregate together in bubbles of aqueous phase. This is nothing to worry about as the particles are not stuck together and will separate upon filtration and washing.
- After centrifugation of the particles, use a micropipette to resuspend them in aqueous solution in order to get separated particles.
- When adding a cell suspension to homogenised ANFC, add the smallest volume possible and mix very thoroughly by pipetting. It may be required to do this for up to 15 minutes in order to maintain the smooth consistency afforded by homogenisation.

**Example 2: Large-scale virus production using nanofibrillar cellulose microbeads as a cellular scaffold and producing nanofibrillar cellulose microbeads by electro spraying**

Goal of project

Many gene therapy products currently in development use a recombinant virus to delivery genetic information to cells. Manufacturing of gene therapy products requires scalable cell culture methods that allow the safe and efficient production of virus particles. The use of microbeads allows the culture of an increased number of virus producing adherent cells per volume of medium, thus facilitating the large-scale production of (therapeutic) virus particles.

In the present work it was aimed to explore the use of NFC and ANFC microbeads as cellular scaffolds to promote the growth and transfection of adherent HEK293T cells in solution to increase the virus yield and allow upscaling in a cost-efficient manner.

More particularly it was aimed to obtain a large-scale virus production using NFC and ANFC microbeads as a cellular scaffold, to find out the optimal NFC and ANFC microbead size and method for maximal HEK293T cell adherence and cell growth, to find out the optimal transfection conditions for adherent HEK293T cells on NFC and ANFC microbeads, and to compare the efficiency of virus production using NFC and ANFC vs standard 2D cell cultivation.

Results

Beads with a maximum diameter of ~1 $\mu$m could be made by ionically cross-linking ANFC droplets produced by electrospraying. The inventors did not find a way to make beads using NFC and therefore the project focused on ANFC. A preferred range of ANFC concentrations that can be used to make beads is 0.3-0.5%.

HEK293T cells can proliferate on the ANFC beads and the rate of proliferation is not different between beads made of different concentrations of ANFC. HEK293T cells growing on the beads can be successfully transfected by using standard methods. Lentivirus particles can be produced in, and recovered from, cultures on ANFC beads.

Methods

The tested methods included cell culture, epi-fluorescence microscopy, and electrospraying.

Conclusions

The inventors established a protocol to produce ANFC beads by electrospraying. The adherence and growth are not affected by the concentration of ANFC used to make the beads. It was identified that cell growth on the ANFC beads is likely the limiting factor for virus production and have investigated different methods to scale up ANFC bead cultures. The inventors showed that cells can be transfected regardless of the ANFC concentration used to make the beads. The inventors also showed that lentivirus particles can be produced on and recovered from ANFC bead cultures, and have made comparisons of cell growth on ANFC beads and a commercially available microcarrier.

Research questions background

Viruses such as lentiviruses and adeno-associated viruses are commonly used for biological research and have the potential be developed for gene therapy purposes. Currently, large-scale production of viruses for biological research relies on the use of adherent cells in large plastic vessels, called Cell Factories. These are multi-layered cell culture flasks that provide a large surface area on which to grow the adherent HEK293T cells. Collection of the virus containing media from these vessels can be challenging, increasing the risk of contamination of both the user and the conditioned media. Furthermore, growing cells in these Cell Factories uses large volumes of cell culture media, requires lots of laboratory space, large amounts of plasmid DNA and transfection reagents, thereby increasing the cost. Viruses must then be collected from large volumes of infectious media. The inventors explored the use of NFC and ANFC microbeads as cellular scaffolds to promote the growth and transfection of adherent HEK293T cells in solution. This will decrease the costs involved in virus production and may have the added advantage of increased cell viability, increased transfection efficiency and thus, increased virus production.

For NFC-based beads to function as cellular scaffolds that allow high density cell culture of adherent cells, they should ideally meet the following requirements:

1) Be small enough to significantly increase the total surface area for cells to adhere to compared to 2D cultures, but large enough to allow proliferation of the cell type to be cultured (HEK293T, length ~20 $\mu$m).

2) Be sufficiently coherent to allow physical manipulations such as medium changes and mixing with cells without disrupting the particles.

3) Be non-toxic to cells and allow efficient cell growth.

With these criteria in mind, the inventors considered a number of different approaches for producing ANFC microbeads, including producing millimetre scale ANFC beads and shrinking them by drying or dehydration, producing micrometre scale ANFC beads directly by electrospraying ANFC to produce small droplets, forming ANFC beads by chemical modification of ANFC through oxidation, and forming ANFC beads by dissolving ANFC in urea/NaOH, followed by dispersion by stirring in oil. The latter two options required use of solid form of cellulose, which was not found useful for the present purposes. For the discussed purposes producing beads by electrospraying was found most advantageous.

Results

Producing millimetre scale ANFC beads and shrinking them by drying or dehydration ANFC in cell culture medium was easily disturbed by pipetting, therefore a cross-linking or a coagulation step was found advantageous to allow the formation of sufficiently coherent ANFC particles. A simple solution finally was to ionically cross-link the anionic ANFC using a divalent cation such as $Ca^{2+}$.

It was first pursued the idea to produce ANFC beads by cross-linking ANFC droplets in a 0.1 M $CaCl_2$ solution. Initially, droplets were produced manually using a micropipette (droplet size: -10-20 $\mu$l). This produced particles that could easily be recovered when the ANFC was applied at a concentration of greater than 0.2%. However, using this method, small droplets could not be produced from 1% ANFC.

However, cross-linked droplets made from 0.2% ANFC were mechanically very unstable and clumped together when left in aqueous solutions. In contrast, $CaCl_2$ cross-linking of droplets of 0.5% ANFC resulted in particles with a diameter of ~3 mm which remain intact and separate during liquid exchanges and centrifugation (Figure 26). Therefore, it was decided to use ANFC concentrations in the region of 0.5% in experiments to optimise the size of cross-linked ANFC particles.

A hot air oven was used to shrink the cross-linked droplets formed using a micropipette. This produced flat, dry 'flakes' which adhered to the plastic tubes. ANFC droplets were also dehydrated by incubating them in 100% ethanol or acetone, followed by evaporation at room temperature. Initially this caused the particles to shrink in size whilst maintaining their spherical shape. However, over time a similar type of 'flakes' were produced after drying the particles in a hot air oven. As these particles adhered to the surfaces they were dried on, and ultimately result in a relatively small NFC surface area compared to the volume of NFC used to produce them, it was not attempted to grow cells on the 'flakes' and pursue this avenue of experimentation further.

Preliminary tests were performed to investigate if the HEK293T cells that will be used for virus production could be grown on the spherical non-dried $CaCl_2$ crosslinked particles. Particles formed by manually dripping ANFC followed by crosslinking were washed with two exchanges of complete medium (Dulbecco's Modified Eagle's Medium (DMEM) + 10% Foetal Calf Serum) to remove excess CaCl2.

Medium was poured off through a cell strainer to allow collection of the particles. HEK293T cells in complete medium were then seeded on the particles in a low-adherence cell culture plate at different densities and grown for five days. Cells were imaged by light microscopy and stained with a live/dead cell assay to determine cell viability five days after seeding. Cell adherence and increasing density on the particles over time was observed at all ANFC concentrations used (Figure 27), despite the daily medium changes required to prevent excessive medium acidification. However, cell densities on the ANFC particles appeared to be much lower after staining with the live/dead cell dyes (Figure 28), suggesting that the adherence of the cells was poor at the time of staining and/or that not all cells were being effectively stained. Nonetheless, cell death levels were low and these observations support the cytocompatibility of the washed ANFC particles.

*Production of micrometer scale ANFC beads by electrospraying.*

Rather than producing large ANFC particles and trying to shrink them, the inventors next used electrospraying to produce micrometre scale droplets to be cross-linked in a $CaCl_2$ solution.

Electrospraying relies on a strong electrical field to destabilise the meniscus of a solution being pumped through a conducting fine needle emitter, resulting in the formation of a Taylor cone. At certain threshold voltage, a liquid jet escapes from the meniscus and accelerates towards a collection plate while splitting into increasingly fine droplets under the influence of the electrical field (Figure 29). Parameters which influence the size and shape of the droplets include the properties of the liquid to be sprayed, the voltage applied, the speed at which the liquid is pumped through the emitter and the distance between the emitter and the collection plate. Two additional factors which may influence the shape of the particles is the force exerted by the cross-linking solution on the ANFC droplet when it hits the surface of the solution, and the speed at which the solution is stirred. Figure 29 contains a photo of the set-up that was used. The highest percentage ANFC that was able to run through the tubing and emitter of the set-up was 0.5%.

Figure 29 A shows a schematic explanation of electrospraying using SprayBase (https://www.spraybase.com/electrospraying). In step 1 voltage is applied to produce an electrical field. The meniscus elongates into a Taylor cone. A divergent electrical filed is created. In step 2 a jet escapes accelerating towards the collector. In step 3 the jet breaks into a plume of droplets. In step 4 the droplets repeatedly split into atomised particles as they dry. The solvent evaporates. In step 5 the products dry and shrink to nanomaterials before they distribute on the collector.

Figure 29 B shows the electrospraying set-up used in the experiments including 1: Emitter; 2: Collection plate; 3: Pressure vessel containing liquid to be sprayed. The collection plate is mounted on top of a magnetic stirrer.

It was found out that when the voltage applied to the system was just below the critical voltage for jet formation, small visible droplets were formed which drip from the emitter, rather than being sprayed. When dripping these particles into a stirred $CaCl_2$ solution particles of ~1 mm diameter were obtained (Figure 30). Reducing the stirrer speed, the distance between the emitter and collector and increasing the ANFC concentration altered the shape of the cross-linked particles (Figure 30). These particles remained intact and dispersed in the $CaCl_2$ cross-linking solution for at least 3 days. In addition, after replacing the $CaCl_2$ solution with DMEM, the particles retained their shape and size for at least four days (Figure 30), indicating their potential suitability for cell culture. Finally, these particles were stable enough to be transferred by pipetting with tips with a sufficiently wide bore and are negatively buoyant and may therefore be collected by centrifugation or by simply allowing the particles (plus cells) to settle.

To further decrease the size of the particles, the voltage was increased in the electrosprayer above the threshold required to produce a jet from the emitter leading to the spraying of the ANFC. Initial attempts using 0.2% ANFC without stirring resulted in clumps of cross-linked ANFC that were likely much larger than the droplets produced. HEK293T cells were seeded in complete medium, mixed with the ANFC clumps, as described above, cultured for five days and shown to be viable and able to proliferate in association with the ANFC clumps, although the cells themselves appeared to grow mostly as clumps (Figure 31).

The particles formed by spraying 0.3-0.5% ANFC while stirring the cross-linking solution showed much better dispersion and had a diameter of -200-500 $\mu$m, but there was considerable variation in their size and shape (Figure 32). It was observed that producing a consistent/continuous spray was difficult, especially with the highest concentration of ANFC used, and this may explain the inconsistency in the shape of the particles.

*Fluorescently labelled LentiX cell lines*

To achieve project aim 2 (Determine the optimal NFC and ANFC microbead size and method for maximal HEK293T cell adherence and cell growth), HEK293T cells (LentiX) were produced which stably express green fluorescent protein (GFP) or the red fluorescent protein mCherry. This allowed to visualise the density and morphology of HEK293T cells grown on the ANFC particles without requiring additional staining procedures.

LentiX cell lines stably expressing mCherry or GFP were made by infecting LentiX cells with a lentiviral vector encoding either of the fluorescent proteins, followed by antibiotic selection. Clonal cell lines were produced by limiting dilution cloning and clones showing a high growth rate were expanded and stored. A LentiX cell line expressing mCherry (LentiX-mCherry) was used for subsequent experiments as GFP expressing cells showed fluorescence in higher wavelength channels on our epifluorescent microscope, which makes it technically challenging to detect transfection of cells in our setup (Figure 33). Transfection efficiency of the LentiX-mCherry cell line was similar to that of control LentiX cells (Figure 33).

*Optimising the production of ANFC particles*

It was previously found out that electrospraying of ANFC was limited to ANFC concentrations of 0.5% or lower, likely because higher concentrations lead to a blockage of the needle emitter. Using a larger gauge needle emitter and larger diameter tubing there were no such issues, and ANFC could be pumped through the electrospraying system at a concentration of at least 0.8%. However, while the concentrated ANFC was able to move through the tubing and the emitter, it was found out that at concentrations of >0.5% entry of the ANFC into the tubing was limited by the viscosity of the ANFC. As found out previously, the lower limit was set by an observation that ANFC concentrations lower than 0.3% resulted in particles that clump together. Therefore, electrospraying ANFC using the set-up available to the inventors remained limited to concentrations of 0.3-0.5% by weight.

Though the inventors had already made small (~1000 $\mu$m maximal diameter), regularly shaped ANFC particles by using the electrospraying equipment to generate small droplets, it was attempted to make smaller particles to further increase the potential surface area per volume of ANFC, while maintaining the regular shape. Mainly, the inventors tried to use higher voltages to induce a stronger electrical field resulting in increased splitting of the ANFC droplets. In addition, the inventors varied the distance between the needle emitter and the collection plate (E-C distance, see Figure 27A), which alters both the strength and shape of the electric field the droplets are subjected to. To aid the visualisation of the particles, the ANFC suspensions were pre-stained with calcofluor white (final concentration: 10% v/v) before spraying. It was found that this doesn't affect the shape and size of the particles compared to particles stained after spraying.

It was found that the maximal diameter of the particles remained ~1000 $\mu$m, regardless of the E-C distance (Figure 34). In addition, the particles were irregularly shaped. It was believed that smaller droplets and droplets with a lower ANFC concentration are more likely to flatten upon impact with the cross-linking solution. Lowering the E-C distance does not seem to significantly alter this (Figure 34). The inventors considered that increasing the $CaCl_2$ concentration may result in an increase in the rate of crosslinking, thus preventing the irregular shaping of the particles. However, electrospraying the NFC ANFC into a 1.0 M $CaCl_2$ solution did not seem significantly alter the size and shape of the particles compared to spraying into a 0.1 M $CaCl_2$ solution (Figure 35). In addition, it was found out that a significant amount of the sprayed ANFC ended up in large clumps when using the 1.0M $CaCl_2$ solution, despite continuous stirring.

*Sterilisation of the ANFC particles*

Using the current set-up, it was not possible produce the particles under completely aseptic conditions. Therefore, it was initially attempted to grow cells on the pre-stained particles in growth medium containing antibiotics (penicillin/streptomycin). $CaCl_2$ was removed and particles were washed in the growth medium using a cell strainer as described previously. LentiX-mCherry cells were put through a cell strainer, seeded on top of the particles in a low-adherence 24-well plate, left to grow for 3 days and imaged by confocal microscopy (Figure 36). It was found out that cells adhered to the outside of the particles, and cell density on the particles increased during the culture period. This demonstrates that it is possible to grow cells on ANFC particles containing calcofluor white, which enables repeated imaging of the cells on the particles without any medium changes or staining procedures. However, it was found out that in most cases, it was difficult to visualise the cells on the particles, as the intensity of the calcofluor white staining was too low to result in a good signal-to-background ratio on the confocal microscope. Calcofluor signal remained easy to detect by epifluorescence, but the irregular shape of the particles made it difficult to determine the location of the cells relative to the particles. Finally, it was found out that cultures on non-sterile particles generally showed visible contamination after ~4 days.

A method for sterilising the particles was therefore required. It was found that the pre-stained particles can be autoclaved without affecting their shape (Figure 37). Autoclaving did result in the loss of some of the calcofluor white dye (Figure 37), but it was found that the fluorescence signal was still easily sufficient to image the particles over time by epifluorescence microscopy (See Figures 38 and 39). It was decided to use the consistently shaped particles produced by electrospraying at a voltage high enough to producing small droplets, but too low to result in further splitting by spraying for further experiments.

Particles were produced using 0.3%, 0.4% and 0.5% pre-stained ANFC, with 0.1 M or 1.0 M $CaCl_2$ as the cross-linking solution. Increasing the $CaCl_2$ concentration in the cross-linking solution affected the shape of the 0.4% particles more than that of the 0.3% and 0.5% particles (Figure 30). While the 0.3% and the 0.5% ANFC particles were disk and spherically shaped, respectively, regardless of the $CaCl_2$ concentration used, the 0.4% ANFC particles sprayed into a 1.0 M CaCl$_2$ solution appeared to be a more tightly folded variant of the 0.4% particles sprayed into a 0.1 M CaCl$_2$ solution (Figure 37).

*Growth and transfection of cells on the sterilised ANFC particles*

To grow LentiX-mCherry cells on the sterilised ANFC particles, the particles were washed twice in growth medium (DMEM with L-Glutamine/10% fetal calf serum) without antibiotics to remove excess CaCl$_2$, dispensed the particles in growth medium in a low adherence 96-well plate and seeded LentiX15 mCherry cells (1.1×10$^4$ cells/well) on top of the particles. The cells were imaged daily following seeding (Figure 38).

It was found out that all cells increased in density on all preparations of particles used and remained free of visible contamination for at least 7 days. On day 5, the cells were transfected with a plasmid encoding GFP under control of constitutively active promoter. DNA:polyethylenimine (at a 1:4 w/w ratio) complexes were prepared and either added to the growing cells, or added to cells after refreshing the medium. Cells were imaged by epifluorescence microscopy at 1 and 2 days post-transfection (Figure 39). Low GFP expression levels were detected on day 1 post-transfection, but a noticeable increase on day 2. The medium change appeared to result in the reduction of the number of cells associated with the particles (e.g. see Figure 39, 0.4% ANFC). From the images in Figures 38 and 39 it is apparent that part of the cells grows as clumps on the particles, and it is likely these cells that become detached during physical manipulations such as medium changes. Nonetheless, these data show that the cells can be grown and transfected on ANFC particles in antibiotic-free medium.

*Releasing cells from ANFC particles for analysis*

For an accurate and representative quantification of cell growth, all cells must be liberated from the ANFC particles and proliferation quantified using a haemocytometer or measuring dye dilution on a flow cytometer. Likewise, the cells must also be liberated from the ANFC in order to quantify transfection by flow cytometry. Therefore, a method which allows the efficient release from the ANFC particles, while maintaining cell viability, is required.

Ideally, the ANFC should be degraded with Growdase, which comprises a mixture of cellulases, and cells collected once the degradation of the ANFC is completed. However, it was found out that Growdase was ineffective on the present crosslinked ANFC. The inventors tried to use the Growdase after disrupting the particles with a pipette, and with or without addition of the calcium chelator EDTA to sequester the calcium ions that cross-link the ANFC particles, to increase the surface area of the ANFC that Growdase can react with. Growdase incubation was always done overnight or longer at 37°C. However, none of these conditions resulted in visible degradation of the ANFC, and cells remained attached to the disrupted ANFC fragments.

The next option was to release the cells from the ANFC by different enzymatic digestion. Washing the particles and cells with PBS and incubation with either trypsin or accutase for up to 30 minutes does not result in the release of the LentiX-cells seen when growing them on tissue culture plastics. However, direct addition of dispase at a final concentration of 0.8 mg/ml to the medium resulted in release of the cells from the ANFC particles after 30-60 minutes incubation at 37°C. Cells and beads have a similar density and are therefore difficult to separate by centrifugation. In addition, most of the cells detach from the ANFC beads as sheets, which makes it more difficult to use cell sieves or filters. Nonetheless, it was possible to perform cell counts of cells grown on ANFC beads to determine the effect of the percentage of ANFC used on LentiX cell proliferation. These data show there was no significant difference in cell number after 5 days of growth on ANFC beads made with different percentages ANFC (Figure 40).

*Production of lentivirus on ANFC beads*

It was tested whether lentivirus particles could be produced on, and recovered from, ANFC beads. LentiX-mCherry cells were grown on ANFC beads in a 24 well plate for 5 days before transfection with lentivirus helper plasmids (Figure 41) and a transfer plasmid to initiate the production of recombinant lentiviral particles encoding GFP. A separate population of LentiX-mCherry cells was seeded in wells without beads 1 day before transfection as a 2D culture control. Transfected cells were kept for another 2 days after which the medium (viral supernatant) was collected from the cells and filtered through a 0.45 μm filter to remove any cells. Non-fluorescent Lenti-X cells were seeded in a 96 well plate in growth medium with diluted viral supernatant and incubated overnight. Green fluorescence was detected as evidence of the presence of infectious lentiviral particles in the viral supernatant (Figure 42). This demonstrates that lentivirus particles can be recovered, from the ANFC bead cultures and are not completely trapped by the ANFC particles.

To determine if a fraction of the lentiviral particles get trapped by the ANFC beads, it was tried briefly to detect a method to develop detection of lentiviral particles in ANFC beads by staining with an anti-P24 antibody (which binds the P24 lentiviral capsid protein) or DiL stain, a lipophilic membrane dye which is weakly fluorescent unless inserted into lipid membranes. However, it was struggled to get these methods to work on 2D cultures and have not pursued this further.

*Scaling up ANFC bead cultures*

For the scale-up of ANFC beads cultures, a number of different systems were tested. It as reasoned it would be beneficial for cell growth if the cells were gently mixed with the beads during adherence period of the culture to facilitate the distribution of the cells over the beads. Therefore, LentiX-mCherry cells were seeded on ANFC particles in ventilated 50 ml tubes, which were slowly rotated on a rollerbank (Figure 43) in a TC incubator. However, imaging of the cells and beads the following day showed that almost all cells were present as freely floating clumps (Figure 44), suggesting that the continuous rotation of the culture prevented adherence of the cells to the beads. Therefore any scaled-up culture using the ANFC beads will need to include a stationary period.

The next step was to test adherence and growth of the LentiX-mCherry cells on ANFC beads in ventilated 50 ml tubes with tipped bottoms kept upright in a $CO_2$ incubator at 37°C. Tubes with vent caps and tubes with regular lids which were loosened slightly to allow ventilation were tested. Also beads with and without calcofluor white to pre-stain the beads were tested. Sieved LentiX-mCherry cells were seeded by mixing them gently with the beads in 10 ml of growth medium using a 25 ml glass pipette. In this set-up, the beads and the cell sink to the bottom of the tube, with most of the medium volume on top of the beads. After two days of culture, a 200 $\mu$l sample of the beads was carefully withdrawn with a widebore pipette tip and imaged. Examples of imaged beads are shown in Figure 45. A good cell adherence on the beads was observed in all conditions after two days of culture. Half of the growth medium was refreshed by careful pipetting and cells were imaged again the next day. At this point, a significant cell detachment in the bead sample in all culture conditions was observed (Figure 45). The observation of cell detachment after a medium change in 50 ml tubes was repeated for nonfluorescent Lenti-X cells and cells at a lower passage number (Data not shown).

It was reasoned that the sensitivity of the cells to detachment upon medium refreshment in the 50 ml tubes may be due to local exhaustion of medium factors important for cell adherence due the concentration of the cells in one compartment of the medium volume (i.e. the bottom of the tube) and/or to improper ventilation of the cells due the relatively large volume of medium covering the cells. Therefore, next the use of T-flasks to scale up ANFC bead cultures was investigated. The T-flasks are essentially a larger version of multi-well culture plates in which, compared to the 50 ml tubes, the same volume of beads and medium can be spread over a larger surface area. This means a better spread of the beads and cell within the culture medium and better ventilation because of the lower diffusion distance between the cells and the surface of the medium. It was found out that LentiXmCherry cells could be grown in T25 flasks with daily 50% medium changes from day 3 of culture onwards without significant cell detachment (Figure 46).

To determine the extent to which the cells could be expanded without subculturing in this system, cell counts were performed at the end of a number of different bead culture experiments in T25 flasks (See Table 4). The results confirm the previous observation that the growth rate of the cells is similar on beads made with different percentages of ANFC. Importantly, the final number of cells after 1 week of culture was very similar even when -4-fold different numbers of cells were seeded at the start of the cultures, and when different amounts of ANFC beads were used. These observations suggest that cell density in the culture becomes the limiting factor for cell growth. Under 2D conditions, a confluent T25 flask contains approximately $8 \times 10^6$ cells. Total cell numbers at the end of the ANFC bead cultures were significantly higher (Table 4). A quantification of the approximate surface area and volume of the ANFC beads shows the beads have a surface area of ~60 $cm^2$ per gram. Therefore, the quantities of ANFC beads used in the experiments reported in Table 4 can be estimated to have a total surface area of ~180 $cm^2$ (for 3 ml of beads) and ~90 $cm^2$ (for 1.5 ml of beads). Because the calculation of area and volume assumed the beads are cylindershaped, this is probably an underestimation of the real surface area. Confluent 2D cultures of LentiX cells on these surface areas can be estimated to have ~$60 \times 10^6$ cells and ~$30 \times 10^6$ cells, respectively. Therefore, although final cell numbers in the T25 ANFC bead cultures were higher than may be achieved in a 2D T25 culture, they are significantly lower than may be expected from the total surface area available on the beads. Figure 47 shows measurement of ANFC beads surface area and volume.

Table 4: LentiX cell proliferation on ANFC beads in T25 flasks

| | | | | |
|---|---|---|---|---|
| 3 ml, 0.4% | 10 ml | $1.0 \times 10^6$ cells | 6 days | $11.4 \times 10^6$ cells |
| 3 ml, 0.4% | 10 ml | $1.0 \times 10^6$ cells | 7 days | $11.5 \times 10^6$ cells |
| 3 ml, 0.3% | 10 ml | $2.2 \times 10^6$ cells | 6 days | $15.1 \times 10^6$ cells |
| 3 ml, 0.4% | 10 ml | $2.2 \times 10^6$ cells | 6 days | $12.3 \times 10^6$ cells |
| 3 ml, 0.5% | 10 ml | $2.2 \times 10^6$ cells | 6 days | $13.9 \times 10^6$ cells |
| 1.5 ml, 0.3% | 10 ml | $0.5 \times 10^6$ cells | 7 days | $14.9 \times 10^6$ cells |
| 1.5 ml, 0.4% | 10 ml | $0.5 \times 10^6$ cells | 7 days | $14.3 \times 10^6$ cells |
| 1.5 ml, 0.5% | 10 ml | $0.5 \times 10^6$ cells | 7 days | $11.4 \times 10^6$ cells |

Table 4 shows an overview of LentiX-mCherry culture in growth medium on ANFC beads in T25 flasks, with ANFC bead amounts, cell number seeded and culture times as reported in the table. Cells were detached from the beads with dispase (1 mg/ml), dispersed by pipetting and counted on a haemocytometer. N=1 for each condition.

It was found out that LentiX-mCherry cells on ANFC beads tended to grow in localised high density groups which, on most beads, only spread out across the surface of the bead to a limited extend. After >5 days in culture, the cells form clumps on the beads instead of spreading on the bead surface (Figure 49). At the same time, the medium acidifies rapidly after more than 4-5 days of culture and daily 50% medium changes are likely insufficient to maintain pH and nutrient levels to support cell growth in the bead cultures. Together, these two factors likely explain submaximal coverage of the ANFC beads by the Lenti-X cells. Whether the clumping of cells on the ANFC beads would occur if the medium pH and nutrient levels would be remain stable and sufficient throughout the culture (e.g. by continuous perfusion of the culture) is unclear.

It was attempted to test ANFC beads using $Mg^{2+}$ (in the form of $MgCl_2$) as the divalent cation to ionically cross-link the ANFC, as well as cell seeding in serumfree medium in comparison to the $Ca^{2+}$-cross-linked beads and seeding in high serum medium to determine whether this would improve the spreading of the cells on the surface of the ANFC beads. Unfortunately, LentiXmCherry cells did not adhere well to beads in any of the conditions used in the experiments for unknown reasons, and this was not pursued further.

It was noted important to maintain conditions supporting cell health during high density cultures in the microcarriers. Therefore, industrial applications requiring cell culture may be scaled up by using stirred bioreactors in which medium conditions can be controlled and mixing ensures consistent conditions for all cells in the culture, while allowing space efficient scale-up.

To investigate how LentiX-mCherry cells can be grown on ANFC beads under stirring conditions, the cultures were performed in 6-well plates on an orbital shaker placed in a temperature and humidity-controlled $CO_2$ incubator. Ideally, the cultures would be stirred instead of shaken but it was chosen to use a shaker as it allowed to perform more cultures in parallel in multi-well plates. ANFC beads were compared to Cytodex 1, a commercially available solid microcarrier consisting of a cross-linked dextran matrix with diethylaminoethyl groups, giving it a positive charge. LentiXmCherry cells were used. Stationary cultures of the cells on Cytodex 1 beads show good adherence and cell growth, though many of the cells are located in high density groups that clump the beads together (Figure 49).

LentiX-mCherry cells were then cultured on ANFC and Cytodex 1 beads and transferred to the orbital shaker on the day after cell seeding. The cultures were kept shaking at 120 RPM, which was the lowest RPM at which mixing of the beads throughout the medium in the wells was observed in the volume of medium used (4 ml per well of a 6 well plate). The cells were imaged the next day (Figure 50). In the cultures on ANFC beads, most cells had detached from the beads and were present in the wells as unattached clumps. A small fraction of the cells remained attached to the beads, mostly so on the beads made with 0.3% ANFC. In contrast, cells on the Cytodex 1 beads showed good adherence, with few detached cells visible in the wells (Figure 51). The cells and Cytodex 1 beads did form large clumps again despite the continuous shaking, although these appeared to break up into smaller clumps after 4 days of culture (Figure 51).

To determine if a longer stationary period before transfer to the shaker would improve LentiXmCherry cells adherence to ANFC beads under shaking, the experiment described above were repeated, but the cells were kept stationary for 4 days (when expanding cells showing angular, adherent morphology were observed) before transferring the culture plate to the orbital shaker. However, a similar degree of cell detachment was observed after an overnight shaking period, with a low fraction of cells attached to beads visible, mostly on the 0.3% ANFC beads (Figure 52).

These data show that adherence of LentiX-mCherry cells on ANFC beads is weaker compared to adherence to Cytodex 1 beads under the shaking conditions used in the experiments, suggesting the need for optimisation of culture conditions.

In the present work ANFC microcarriers were developed which were useful for large-scale cell culture and lentivirus production, for example. Lenti-X cells were used because they are an optimised cell line for the production of lentivirus. Ionically cross-linked ANFC beads were developed and produced by electrospraying and it was demonstrated that is possible to grow and transfect Lenti-X cells on these beads, and to produce and recover lentiviral particles from them. Finally, it was tested, on a small scale, how ANFC bead cultures may be scaled up.

Materials and methods

*Used methods*

LentiX cells were cultured using standard methods in Dulbecco's Modified Eagle Medium with GlutaMax™, pyruvate and 10% (v/v) fetal calf serum (GM). Cultures of LentiX cells on beads in microplates were done in low-adherence multi-well plates, 50 ml tubes or T-flasks.

Cells on ANFC beads were imaged on a LifeTech Evos FL epi-fluorescent microscope or a Nikon A1 confocal microscope.

Monoclonal LentiX cells expressing mCherry were made by transducing LentiX cells with a recombinant Lentivirus followed by selection in GM containing the appropriate antibiotic. Monoclonal cell lines were made by limited dilution cloning.

Cell counts were performed using a haemocytometer.

Cytodex 1 microcarriers were obtained from Scientific Laboratory Supplies Ltd and washed and sterilised according to the manufacturer's instructions.

*Protocol*

*Generation of ANFC beads*

ANFC was diluted to the desired concentration with water and calcofluor white (10% v/v) by mixing with a narrow-tip pipette tip for at least 1 minute. The pre-stained ANFC was then electrosprayed at ~4.5 kV using a 22G needle emitter into a gently stirred 0.1 M or 1.0 M calcium chloride solution in water. The resulting beads were collected from the collector plate of the electrosprayer with a 25 ml glass pipette and kept in the calcium chloride solution in a glass bottle. The beads were then autoclaved in a bench top autoclave on liquid cycle.

*Seeding of LentiX cells onto ANFC beads (for microplate/50 ml tubes)*

The autoclaved beads are pipetted through a cell strainer using a 10 or a 25 ml glass pipette to remove the calcium chloride solution. The beads are then washed in growth medium by pipetting the medium into the cell strainer containing the beads while it's kept in its sterile wrapper. The cells are then collected along with the medium using a glass pipette and kept in a 50 ml tube for at least 5 minutes. This is repeated twice. For cultures in microwell plates, the washed beads can then be dispensed into a microwell plate using a wide bore pipette tip, and the desired number of freshly passaged cells can be seeded on top of the beads. For cultures in 50 ml tubes or T-flasks, passaged cells were sieved with a 40 $\mu$m cell strainer and mixed with the beads and the final volume of growth medium in the tube used for washing the cells before seeding.

*Transfection of LentiX cells on ANFC beads*

Transfection of cells was done by complexing plasmid DNA with PEI in a 1:4 (w/w) ratio in serum free medium for 15 minutes at room temperature, before adding the complexes to the cultures by pipetting directly into the wells.

*Release of cells from ANFC beads*

Dispase II stock (Sigma #D4693) is dissolved at 10 mg/ml in 50 mM HEPES/KOH (pH: 7.4) + 9 g/l NaCl. The stock added to cultures in growth medium at a final concentration of 1 mg/ml. Cultures were then incubated at 37°C for 60 minutes to dissociate the cells from the ANFC beads. Cell suspensions for counting were prepared by thoroughly disrupting the ANFC beads and dissociating cell clumps by pipetting.

*Materials*

- ANFC from wood cellulose
- ddH$_2$O (autoclaved)
- CaCl$_2$ solution (autoclaved)
- Growth medium (Dulbecco's Modified Eagle Medium with pyruvate, GlutaMax and 10% fetal calf serum)
- Calcofluor white
- Tubing + emitter needle (autoclaved) (Spraybase)
- Electrospray equipment (cleaned with 70% ethanol) (Spraybase)
- Cell strainers
- Sterile/pyrogen-free 50 ml tube with vented or regular caps
- T -Flasks

**Claims**

1.  A method for preparing microbeads comprising nanofibrillar cellulose, the method comprising

    - providing an aqueous dispersion of chemically anionically modified nanofibrillar cellulose having a number-average diameter of 200 nm or less, such as 100 nm or less,
    - forming the nanofibrillar cellulose into microbeads having an average diameter in the range of 100-1200 $\mu$m, to obtain microbeads comprising chemically anionically modified nanofibrillar cellulose having a concentration in the range of 0.2-2% by weight in the microbeads, such as in the range of 0.2-1% by weight, such as in the range of 0.2-0.8% by weight,
    - the method further comprising treating the chemically anionically modified nanofibrillar cellulose or the microbeads with multivalent cations to ionically crosslink the nanofibrillar cellulose.

2.  The method of claim 1, comprising homogenizing the aqueous dispersion of chemically anionically modified nanofibrillar cellulose with a nonfibrillating homogenization.

3.  The method of claim 1 or 2, wherein the microbeads have a coefficient of variation (CV) in the range of 15-30%.

4.  The method of any of the preceding claims, wherein the multivalent cations are selected from multivalent metal and earth alkali metal cations, for example selected from cations of calcium, magnesium, barium, zinc, aluminum, gold, platinum and titanium, to ionically crosslink the nanofibrillar cellulose.

5.  The method of any of the preceding claims, wherein the forming the nanofibrillar cellulose into microbeads comprises membrane emulsification, comprising forming the dispersion of chemically anionically modified nanofibrillar cellulose into an emulsion and passing the emulsion through a porous membrane to form microbeads comprising chemically anionically modified nanofibrillar cellulose, preferably wherein the forming the dispersion into an emulsion comprises adding one or more oil(s) and/or one or more emulsifying agent(s).

6.  The method of any of the preceding claims, wherein the obtained microbeads have an average diameter in the range of 100-230 $\mu$m, such as in the range of 100-190 $\mu$m.

7.  The method of any of the claims 1-4, wherein forming the nanofibrillar cellulose into microbeads comprises electro spraying.

8.  The method of claim 7, wherein the obtained microbeads have an average diameter in the range of 200-1000 $\mu$m, such as in the range of 200-500 $\mu$m, and preferably a concentration of nanofibrillar cellulose in the range of 0.2-1% by weight, such as in the range of 0.3-0.5% by weight.

9.  Microbeads having an average diameter in the range of 100-1200 $\mu$m and comprising chemically anionically modified nanofibrillar cellulose having a number-average diameter of 200 nm or less, such as 100 nm or less, and having a concentration of the chemically anionically modified nanofibrillar cellulose in the range of 0.2-2% by weight in the microbeads, such as in the range of 0.2-0.8% by weight, wherein the nanofibrillar cellulose is ionically crosslinked with multivalent cations.

10. The microbeads of claim 9, wherein the multivalent cations are selected from multivalent metal and earth alkali metal cations, for example selected from cations of calcium, magnesium, barium, zinc, aluminum, gold, platinum and titanium.

11. The microbeads of claim 9 or 10 having a coefficient of variation (CV) in the range of 15-30%.

12. The microbeads of any of the claims 9-11 having an average diameter in the range of 100-230 $\mu$m, such as in the range of 100-190 $\mu$m.

13. The microbeads of any of the claims 9-12 having an average diameter in the range of 200-1000 $\mu$m, such as in the range of 200-500 $\mu$m, and preferably a concentration of nanofibrillar cellulose in the range of 0.2-1 % by weight, such as in the range of 0.3-0.5% by weight.

14. The method of any of the claims 1-8 or the microbeads of any of the claims 9-13, wherein the chemically anionically modified nanofibrillar cellulose is TEMPO oxidized nanofibrillar cellulose.

15. The method of any of the claims 1-8 or the microbeads of any of the claims 9-14, wherein the nanofibrillar cellulose, when dispersed in water, provides a zero shear viscosity in the range of 100-50000 Pa s, such as in the range of 300-8000 Pa s, and a yield stress in the range of 1-50 Pa, such as in the range of 2-15 Pa, determined by rotational rheometer at a consistency of 0.5% by weight in aqueous medium at 22±1 °C.

16. A cell culture comprising cells in the microbeads of any of the claims 9-15.

17. A method for producing cell-derived products, the method comprising

  - providing the microbeads of any of the claims 9-15,
  - providing cells,
  - combining the cells with the microbeads, and
  - allowing the cells to produce cell-derived products in the microbeads.

18. The microbeads of any of the claims 9-15 for use for providing bioactive substances to a target, such as wherein the bioactive substances are selected from vesicles and cell organelles, such as extracellular vesicles, for example exosomes, proteins, carbohydrates, lipids, nucleic acids, antibodies, hormones, viruses, parts thereof and virus-like particles.

19. Use of chemically anionically modified nanofibrillar cellulose having a number-average diameter of 200 nm or less, such as 100 nm or less, for preparing the microbeads of any of the claims 9-15.

Fig. 1

Fig. 2

Fig. 3

Fig. 4

Fig. 5

Fig. 6

Fig. 7

Fig. 8

Fig. 9

Fig. 10

Fig. 11

Fig. 11

Fig. 12

Fig. 12

Fig. 13

Fig. 13

Fig. 14

Fig. 15

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 29

0.4% ANFC, 10 cm, high speed | 0.4% ANFC, 2 cm, reduced speed | 0.5% ANFC, 2 cm, reduced speed

Day 0

Day 4

Fig. 30

0.2% ANFC, Lenti-X HEK293T

ANFC   Calcein-AM   ethD-1

Fig. 31

0.3% ANFC    0.4% ANFC    0.5% ANFC

Fig. 32

Fig. 33

Fig. 34

EP 4 148 071 A1

|  | 0.3% ANFC | 0.4% ANFC | 0.5% ANFC |
|---|---|---|---|
| 0.1M CaCl2 | | | |
| 1.0M CaCl2 | | | |

Fig. 35

Fig. 36

Fig. 37

ANFC    LentiX-mCherry

Fig. 38

**0.3% ANFC (Day 6)**
No medium change   Medium changed

**0.3% ANFC (Day 7)**
No medium change   Medium changed

[CaCl2]

0.1M

1.0M

**0.4% ANFC (Day 6)**
No medium change   Medium changed

**0.4% ANFC (Day 7)**
No medium change   Medium changed

0.1M

1.0M

**0.5% ANFC (Day 6)**
No medium change   Medium changed

**0.5% ANFC (Day 7)**
No medium change   Medium changed

0.1M

1.0M

ANFC   LentiX-mCherry   eGFP

Fig. 39

Fig. 40

Fig. 41

0.3% ANFC          0.4% ANFC          0.5% ANFC          2D

Fig. 42                eGFP (cells infected by lentivirus)

Fig. 43

6.7E5 cells 3.4E5 cells

Fig. 44

Fig. 45

2 Days 3 Days 4 Days 5 Days

Fig. 46

Fig. 47

0.3% ANFC    0.4% ANFC    0.5% ANFC

Fig. 48

EP 4 148 071 A1

Fig. 49

0.3% ANFC          0.4% ANFC          0.5% ANFC

1 Day

Overnight
shaking

2 Days

Fig. 50

Fig. 51

EP 4 148 071 A1

0.3% ANFC       0.4% ANFC       0.5% ANFC

4 Days

Overnight
shaking

5 Days

Fig. 52

Europäisches
Patentamt

European
Patent Office

Office européen
des brevets

# EUROPEAN SEARCH REPORT

Application Number

EP 22 19 4012

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 581 591 A1 (UPM KYMMENE CORP [FI]) 18 December 2019 (2019-12-18) * claims 1, 5, 7-8, 13, 17 * * paragraphs [0060], [0084] * | 1-19 | INV. C08B15/00 |
| A | CARVALHO RENATA AQUINO DE ET AL: "The potential of TEMPO-oxidized nanofibrillar cellulose beads for cell delivery applications", CELLULOSE, SPRINGER NETHERLANDS, NETHERLANDS, vol. 23, no. 6, 3 September 2016 (2016-09-03), pages 3399-3405, XP036104380, ISSN: 0969-0239, DOI: 10.1007/S10570-016-1063-2 [retrieved on 2016-09-03] * the whole document * | 1-19 | |
| A | EP 3 418 377 A1 (UPM KYMMENE CORP [FI]) 26 December 2018 (2018-12-26) * the whole document * | 1-19 | TECHNICAL FIELDS SEARCHED (IPC) |
| A | EP 3 669 651 A1 (UPM KYMMENE CORP [FI]) 24 June 2020 (2020-06-24) * the whole document * | 1-19 | A61K C08B C09J |
| A | EP 3 581 590 A1 (UPM KYMMENE CORP [FI]) 18 December 2019 (2019-12-18) * the whole document * | 1-19 | |

The present search report has been drawn up for all claims

| Place of search | Date of completion of the search | Examiner |
|---|---|---|
| The Hague | 27 January 2023 | Tarallo, Anthony |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 19 4012

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2023

| Patent document cited in search report | | Publication date | Patent family member(s) | | Publication date |
|---|---|---|---|---|---|
| EP 3581591 | A1 | 18-12-2019 | CA | 3095532 A1 | 19-12-2019 |
| | | | CN | 112334492 A | 05-02-2021 |
| | | | EP | 3581591 A1 | 18-12-2019 |
| | | | JP | 2021527736 A | 14-10-2021 |
| | | | KR | 20210021373 A | 25-02-2021 |
| | | | US | 2021130500 A1 | 06-05-2021 |
| | | | WO | 2019238327 A1 | 19-12-2019 |
| EP 3418377 | A1 | 26-12-2018 | CA | 3068127 A1 | 27-12-2018 |
| | | | CN | 110799642 A | 14-02-2020 |
| | | | EP | 3418377 A1 | 26-12-2018 |
| | | | JP | 2020524507 A | 20-08-2020 |
| | | | RU | 2019142313 A | 22-07-2021 |
| | | | US | 2020115678 A1 | 16-04-2020 |
| | | | WO | 2018234634 A1 | 27-12-2018 |
| EP 3669651 | A1 | 24-06-2020 | CA | 3062871 A1 | 21-06-2020 |
| | | | CN | 111345281 A | 30-06-2020 |
| | | | EP | 3669651 A1 | 24-06-2020 |
| | | | JP | 2020103281 A | 09-07-2020 |
| | | | SG | 10201912442S A | 29-07-2020 |
| | | | US | 2020199536 A1 | 25-06-2020 |
| EP 3581590 | A1 | 18-12-2019 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Cell Stem Cell,* 07 February 2008, vol. 2 (2), 113-7